# EUROPÄISCHE PATENTANMELDUNG

(11) **EP 2 557 075 A1**
(43) Veröffentlichungstag der Anmeldung: **13.02.2013**
(21) Anmeldenummer: 11176944.4
(22) Anmeldetag: 09.08.2011
(51) Int. Cl.: C07C 245/24

(54) **Neue Triazenverbindungen zur Behandlung von Krebs**

(71) Anmelder: Trin Therapeutics GmbH, 40210 Düsseldorf (DE)
(72) Erfinder: Ludwig, Georg, 9492 Eschen (LI)
(74) Vertreter: Gille Hrabal

(57) **Zusammenfassung**

Die vorliegende Erfindung betrifft neue Triazenverbindungen, Verfahren zu ihrer Herstellung, sie umfassende pharmazeutische Zusammensetzung sowie die Verwendung davon zur Behandlung von Krebserkrankungen beim Menschen und beim Tier. Die neuen Triazenverbindungen zeichnen sich gegenüber den bekannten Triazenverbindungen durch eine verbesserte therapeutische Breite, d.h. durch geringere Nebenwirkungen bei hoher Antitumorwirkung, insbesondere durch eine verbesserte Aufnahme in Zellen, insbesondere Krebszellen, und damit eine deutliche Erhöhung des therapeutischen Fensters für diese neuen Triazenverbindungen, aus.

## Beschreibung

Die vorliegende Erfindung betrifft neue Triazenverbindungen und Verfahren zu ihrer Herstellung, sie umfassen die pharmazeutische Zusammensetzung sowie die Verwendung davon zur Behandlung von Krebserkrankungen beim Menschen. Die neuen Triazenverbindungen zeichnen sich gegenüber den bekannten Triazenverbindungen durch eine verbesserte therapeutische Breite, d.h, durch geringere Nebenwirkungen bei hoher Antitumorwirkung, insbesondere durch eine verbesserte Aufnahme in Zellen, insbesondere Krebszellen, und damit eine deutliche Erhöhung des therapeutischen Fensters für diese neuen Triazenverbindungen, aus,

### HINTERGRUND DER ERFINDUNG:

Triazenderivate wurden in den 70er Jahren ausführlich auf ihre cytostatische Wirksamkeit und die damit vorhandene Möglichkeit zur Behandlung von Krebserkrankungen erforscht, Aufgrund ihrer starken Nebenwirkungen und Toxizität dieser zu den Alkylierungsmitteln zählenden Zytostatika sind sie aber nie zu einer breiteren Anwendung bei der Tumorbekämpfung gekommen. Eine Ausnahme stellt das Dacarbazin (DTIC) dar, das ein Prodrug von Monomethyl-triazeno-imidazol-carboxamid (MTIC) ist und hauptsächlich zur Bekämpfung von Melanomen eingesetzt wird (Montgomery JA (1976) Cancer Treat Rep 60, 205-211).

Mit Temozolomide (4-methyl-5-oxo- 2,3,4,6,8-pentazabicyclo [4.3.0] nona-2,7,9-triene- 9-carboxamide; TMZ) wurde 1999 ein weiteres Triazen zugelassen, das vorwiegend für die Therapie von Hirntumoren (Friedman HS Clinical Cancer Research Vol 6 2585-2597) eingesetzt wird, TMZ ist wie DTIC ein Prodrug von MTIC, benötigt aber im Unterschied zu diesem keine metabolische Aktivierung, um seine zytotoxische Wirkung zu entfalten, Die zytotoxische Aktivität von DTIC/TMZ wird der Bildung von 06-Methylguanin- und N7-Addukten zugeschrieben, Allerdings wird ein Teil dieser DNA-Schädigungen durch körpereigene DNA-Reparatur-mechanismen wieder rückgängig gemacht, Dabei spielt die Methylguanin-Methyltransferase (MGMT) eine zentrale Rolle (Esteller M Oncogene 2004;23: 1-8), MGMT ist ein Protein innerhalb des Zellkerns, das an der Reparatur alkylierter DNA beteiligt ist, Dieses Enzym schützt Zellen, indem es die Übertragung der Methylgruppe vom 06-Methylguanin auf sich selbst bewerkstellig, Im Unterschied zu einem Enzym agiert das MGMT als Substrat, das bei der Übertragung verbraucht wird und erst wieder erneut über das MGMT-Promotor-Gen hergestellt werden muss, So wurde für die Radiochemotherapie mit TMZ an Patienten mit Gliobastom bereits gezeigt, dass Patienten mit methyliertem Promotor-Gen eine günstigere Überlebensprognose zeigen als solche mit funktionsfähigem Gen (Hegi ME, N Eng J Med 2005; 352: 997-1003).

Um die Wirksamkeit der Triazene zu verbessern, wurde versucht, das Ausmaß der DNA-Schädigung der Zelle zu erhöhen. Dies geschah entweder mittels MGMT supprimierender Substanzen wie z.B. O6-Benzylguanine oder Lomeguatrib (Quinn JA JCO Vol,27 No.8 12b2-1267) oder durch Gabe von TMZ in kürzeren Zeitabständen oder höherer Dosis (eine Übersicht findet sich bei Wick W Feb, 2009; 69-79), Einzelne Arbeiten (z.B. JCO Vol,25 No.18 2007; 2540-2545) berichten von niedrigeren MGMT-Konzentrationen im Melanom, konnten aber keinen Überlebensvorteil für die deutlich toxischere Kombinationstherapie mit einem MGMT-Suppressor belegen, Ebenso wenig führte eine Erhöhung der Dosisintensität auf 2.100mg/m² Temozolomid pro Zyklus beim Gliobastom nicht zu einem Überlebensvortell, dafür aber zu deutlich höheren Nebenwirkungen auf das blutbildende System (Neyns B Cancer Invest. 2008; 26: 269-277).

Verbeck (British Medical Bulletin 2008, 85, 17-33) sieht ein Potenzial für zwei Arten von Wirkstoffen zur Überwindung dieser Problematik. Zum Einen solche, durch die die MGMT Konzentration spezifisch in Krebszellen reduziert werden kann und damit einhergehend die Verabreichung einer alkylierenden Substanz zu einer stärkeren Schädigung der Krebszellen führen würde, ohne den Schutzmechanismus anderer gesunder Zellen (insbesondere des blutbildenden Systems) zu reduzieren, Zum anderen wären Substanzen denkbar, deren alkylierende Wirkung entweder nur bei Krebszellen entfacht würde, oder diese Wirkung bei Krebszellen bereits in Konzentrationen einsetzt, welche bei gesunden Zellen noch keine, oder noch als verträglich einzuschätzende DNA-Schädigung hervorruft, Weisen Krebszellen eine höhere Zellpermeabilität für eine antineoplastische Substanz auf als gesunde Zellen, lässt sich auch dadurch das therapeutische Fenster verbessern,

In der Vergangenheit wurde eine große Anzahl von neuen Triazenen synthetisiert (Montgomery JA (1976) Cancer treatment reports 60: 125-134; Spassova MK and Golovinsky EV (1985) Pharmac Ther 27: 333-352; Derry E. V. Wilman and Phyllis M. Goddard; J. Med. Chem. 1980. 23, 1052-1024; DE 1768720; WO91/17753; F. Schmidt et al.; J. Med. Chem. 1994, 37, 3812-3818 (betrifft die antineoplastische Wirkung von Peptid-gebundenen 1,3-Dialkyl-3-acyltriazenen); Dan Liu Molecules 2010, 15, 9427-9436).

Ein Ansatz zur Überwindung der Verträglichkeitsproblematik von Triazenen wurde in DE 1793115 und DE 2147781 beschrieben.
Die DE 1 7931 1 5 betrifft in den Phenylkernen gegebenenfalls substituierte Mono- und bis-Dialkyl-Triazenderivate, wobei ausschließlich solche Verbindungen bevorzugt und konkret offenbart werden, worin die Phenylkerne in para-Position zur verbrückenden X-Position, entsprechend dem Substituenten R¹¹ der allgemeinen Formel (I), mit einem Triazenrest unter Bildung eines Bis-Triazens oder Wasserstoff unter Bildung eines in p-Position unsubstituierten Mono-Triazens substituiert sind, Insbesondere ergeben sich aus diesem Dokument keine Hinweise auf eine entsprechende Substituierung an R¹¹ mit einem über Sauerstoff gebundenen Rest (-O-R). Des Weiteren werden auch keine Verbindungen konkret offenbart, die an den Phenylkernen (insbesondere an R¹¹) ausschließlich Methoxy-substituiert sind oder worin die Phenylkerne mit einem gegebenenfalls substituiertem Alkoxy-Rest, ausgewählt aus einem über Sauerstoff gebundenen Zucker-Rest, substituiert sind. Hydroxy ist als möglicher Substituent der Phenylkerne der Triazenverbindungen der DE 1 7931 1 5 nicht vorgesehen, Die Substanzen aus DE 1 793 1 15 zeigten eine ausgeprägte Anti-Tumorwirkung in chemisch induzierten Brustkrebsen von Ratten. Dabei wurden Einzeldosierungen zwischen 200 - 5,000 mg/kg und kumulative Dosen von bis zu 34,000 mg/kg pro Zyklus verabreicht. Die Applikation erfolgte subkutan. Wirknachweise für andere Tumorentitäten, insbesondere auch für andere Tumorarten als solide Tumore, oder durch andere Chemikalien induzierte Mammakarzinome wurden nicht erbracht.

Die DE 2147781 offenbart 1 -Phenyl-3,3-Dialkyl-Triazene, entsprechend der allgemeinen Formel (1), mit der Substitution an R¹¹ durch eine Hydroxygruppe (-OH) oder einem entsprechenden Salz, Eine Hydroxy-Substitution an einer anderen oder weiteren Position der Phenylkerne (entsprechend R³ bis R¹⁰ der allgemeinen Formel (1)) ist hier nicht vorgesehen, Außerdem sind ausschließlich solche Verbindungen Gegenstand der Offenbarung, worin die Phenylkerne außerdem durch mindestens einen weiteren Substituenten wie insbesondere durch eine Carboxyl- oder Sulfonsäuregruppe substituiert sind. Insbesondere aus der Gruppe der Triazene, worin X eine Carbonyl-Gruppe darstellt werden ausschließlich Carboxyl- oder Sulfonyl-substituierte 1-Phenyl-3,3-Dialkyl-Triazene konkret offenbart, Die Substitution mit Carboxyl- oder Sulfonylgruppen dient der Verbesserung der Löslichkeit der eingesetzten Verbindungen. Entsprechend sind unsubstituierte 1-Phenyl-3,3-Dialkyl-Triazene (entsprechend Verbindungen der allgemeinen Formel (1) mit R¹¹ = Hydroxy oder einem entsprechenden Salz und R³ bis R¹⁰ = Wasserstoff) nicht Gegenstand der DE 2147781 und können dieser auch nicht konkret entnommen werden. Die Substanzen aus DE 2147781 zeigten ebenfalls eine Anti-Tumorwirkung ausschließlich in soliden Tumoren, nämlich chemisch induzierten Brustkrebsen von Ratten. Dabei wurden Einzeldosierungen zwischen 500 und 600 mg/kg bzw. kumulative Dosen zwischen 3.000 und 6.000 mg/kg pro Zyklus verabreicht. Die Applikation erfolgte subkutan. Auch hier wurden Wirknachweise für andere Tumorentitäten, insbesondere für andere Tumorarten als solide Tumore, nicht erbracht.

Durch die Einführung von stark polaren funktionellen Gruppen konnte nicht nur die Wasserlöslichkeit von Triazenderivaten sondern auch die rasche Ausscheidung der Substanzen stark verbessert werden. Die für Dacarbazine oder Temozolomid beschriebenen Nebenwirkungen wie Übelkeit, Erbrechen und die Suppression des blutbildenden Systems, konnten durch diese Substanzen stark reduziert werden. Allerdings wurde dies durch eine starke metabolische Belastung der Ausscheidungsorgane Leber und Niere erkauft. Brustkrebspatientinnen wurden in einer Studie mit 750 mg Substanz täglich peroral über mehrere Wochen behandelt, wobei eine Häufung von Nierenschädigungen beobachtet wurde. Aufgrund von Verträglichkeitsproblemen bei Langzeitgabe am Menschen war der verträgliche Dosisbereich limitierend, so dass das vielversprechende therapeutische Potential dieser Verbindungsklasse nicht umgesetzt werden konnte.

Die WO 2009/004060 beschreibt eine Weiterentwicklung der Triazene gemäß der DE 1793115. Dabei betrifft die WO 2009/004060 ausschließlich solche Triazenderivate, die mit einer Aminocarbonylsubstituierten Methoxy- oder Ethoxy-Gruppe substituiert sind sowie deren Verwendung zur Behandlung von Krebserkrankungen, Auch hierin ist Hydroxy als möglicher Substituent der Phenylkerne der Triazenverbindungen nicht vorgesehen. Desweiteren werden auch keine Verbindungen konkret offenbart, worin die Phenylkerne (insbesondere an R¹¹) ausschließlich Methoxy-substituiert sind oder worin die Phenylkerne mit einem gegebenenfalls substituierten Alkoxy-Rest, ausgewählt aus einem über Sauerstoff gebundenen Zucker-Rest, substituiert sind. Dabei konnte in einem direkten Vergleich der bevorzugten Verbindungen der WO 2009/004060 mit der nächstliegenden Verbindung der DE 1 7931 1 5 gezeigt werden, dass die verbesserten Triazene der WO 2009/004060 im Tierversuch keine Schädigungen der Nieren bewirkten wohingegen die Vergleichssubstanz nach der DE 1 7931 1 5 merkliche fokale tubuläre Nekrosen herbeiführte. Auch konnte für die verbesserten Triazenverbindungen der WO 2009/004060 eine überzeugende Anti-Tumorwirkung in Maus-Tumor-Xenograft Modellen für Melanome, Brustkrebs und Darmkrebs gezeigt werden. Dabei wurden Einzeldosen zwischen 300-590 mg/kg und kumulative Dosierungen von bis zu 4.900 mg/kg (Mol Cancer Ther 2009; 8 (12 Suppl); A144) intraperitoneal appliziert. Trotz der hohen Dosen kam es nur in wenigen Fällen zu Mortalität der Versuchstiere. Diese Ergebnisse deuten auf eine schlechte Zellpermeabilität der Verbindungen der WO 2009/004060 hin, was im Zusammenhang mit den entsprechenden Reparaturmechanismen in der Zelle dazu führt, dass diese Substanzen in verhältnismäßig hohen Mengen appliziert werden müssen, um entsprechende Anti-Tumoreffekte erzielen zu können. Die niedrige Zellpermeabilität der Substanzen aus WO 2009/004060 sowie DE 1 7931 1 5 konnte in Vergleichsversuchen gezeigt werden. Waldeck et al (Int J. Med. Sci 2008, 5, 273-284) beschreiben eine erhöhte Wirksamkeit sogenannter TMZ-Bioshuttles gegenüber dem heute verfügbaren TMZ in DU145 (hormonrefraktäres Prostatakarzinom) und TP366 (Gliobastom) Zelllinien. Mittels des konjugierten Peptid-Trägers könne über höhere TMZ Konzentrationen in der Tumorzelle eine Wirkungsverstärkung erreicht werden.

Die neuen Triazenverbindungen der vorliegenden Erfindung, die durch eine O-R Substitution am Phenylkern (Substituentenposition R⁷ bis R¹¹, entsprechend der Formel (1)) gekennzeichnet sind, insbesondere solche die durch eine Hydroxy- oder Methoxy-Substitution gekennzeichnet sind, sowie solche, die einen über Sauerstoff gebundenen Zucker-Rest aufweisen, zeichnen sich gegenüber den oben genannten Substanzen aus WO2009/004060 und DE 1793115 sowie den bereits zugelassenen Triazenen DTIC und TMZ insbesondere durch eine deutlich verbesserte Zellpermeabilität und damit durch eine deutliche Verbesserung des therapeutischen Fensters sowie geringerer Nebenwirkungen aus. Vergleichsversuche zeigen die höhere intrazelluläre Aufnahme der neuen Triazenverbindungen gegenüber Vergleichssubstanzen. Insbesondere konnten die Erfinder überraschenderweise zeigen, dass die erfindungsgemäßen neuen Triazenverbindungen eine vergleichbare Anti-Tumorwirkung (IC₅₀) in zahlreichen Krebszelllinien wie der HepG2 (Leber), HCT 8 (Darm), DU-1 45 (Prostata), MCF-7, MDA-MB231 (Brust) und MiaPaca-2 (Bauchspeicheldrüse) bei bis zu 100fach niedrigeren Konzentrationen aufweisen als vergleichbare Triazene wie DTIC, TMZ oder Substanzen wie sie in der WO/2009/004060, der DE 1 7931 1 5 und DE 21 47781 beschrieben sind. Weitere Versuche zeigen, dass bei Verabreichung einer wirksamen Dosis der neuen Triazenverbindungen periphäre Blutzellen nur unwesentlich geschädigt werden, wohingegen die heute zugelassenen Verbindungen bei derartigen Dosierungen zu deutlichen Zellschädigungen führen, Weitere Experimente bestätigen außerdem eine ausreichende zytotoxische Wirkung der neuen Triazenverbindungen an leukämischen Zellen, womit eine Anwendung dieser Verbindungen in der Hämatologie bzw. in der Behandlung von Blutkrebs möglich wird. Schließlich konnte gezeigt werden, dass die neuen Triazenverbindungen in hämatologischen (HL-60, CEM) und soliden Tumorzelllinien (MDA-MB231) im Unterschied z.B. zu Präparat 2 der WO/2009/004060 zu einem G2 Arrest im Zellzyklus und massivem Zelltod führen.
Zusammenfassend lässt sich damit feststellen, dass die neuen Triazenverbindungen ein breiteres therapeutisches Fenster aufweisen als andere Triazenverbindungen.

### AUFGABENSTELLUNG:

Die Aufgabe der vorliegenden Erfindung bestand somit darin, zytostatische Triazenderivate mit verringerter Toxizität bei verbesserter Wirksamkeit zu finden, um sie für die Therapie beim Menschen und am Tier, insbesondere zur Therapie der Krebserkrankungen zugänglich zu machen.

### BESCHREIBUNG DER ERFINDUNG

Die Erfinder fanden neue Triazenyl-Verbindungen mit hoher zytostatischer Aktivität und deutlich verbesserter Zellpermeabilität, deren Toxizität deutlich reduziert ist. Gegenstand der Erfindung sind daher Verbindungen der Formel (1); 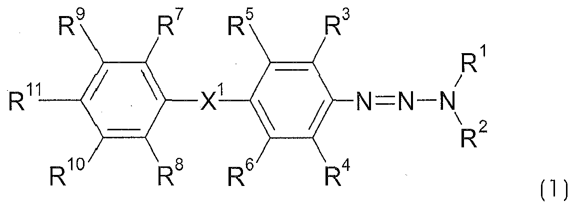 worin
R¹ und R² gleich oder verschieden sind, und jeweils ausgewählt werden aus der Gruppe, die besteht aus:
   - gegebenenfalls substituiertem Alkyl,
   - gegebenenfalls substituiertem Alkenyl, und
   - gegebenenfalls substituiertem Aryl;
X¹ aus der Gruppe ausgewählt wird, die besteht aus:
   - einer Einfachbindung
   - Carbonyl,
   - Schwefel,
   - Sauerstoff,
   - Sulfoxy,
   - Sulfonyl,
   - Azo, und
   - einem gegebenenfalls substituierten, gesättigten oder ungesättigten aliphatischen Rest mit 1 bis 6 Kohlenstoffatomen;
      und worin
      i) R¹¹ Hydroxy ist und
         R³, R⁴, R⁵, R⁶, R⁷, R⁸, R⁹ und R¹⁰ gleich oder verschieden sind, und jeweils ausgewählt werden aus Wasserstoff und Hydroxy;
      oder worin
      ii) mindestens einer der Reste R⁷, R⁸, R⁹, und R¹⁰ sowie gegebenenfalls mindestens einer der Reste R³, R⁴, R⁵ und R⁶ Hydroxy ist und
      die übrigen Reste R³, R⁴, R⁵, R⁶, R⁷, R⁸, R⁹ und R¹⁰, die nicht Hydroxy bedeuten, sowie der Rest R¹¹ gleich oder verschieden sind und jeweils ausgewählt werden aus der Gruppe, die besteht aus:
   - Wasserstoff,
   - Halogen,
   - Cyano,
   - Nitro,
   - Carboxyl,
   - Alkoxycarbonyl,
   - Aminocarbonyl,
   - gegebenenfalls substituiertem Acyl,
   - gegebenenfalls substituiertem Acyloxy
   - gegebenenfalls substituiertem Sulfonyl (-SO₂R),
   - Sulfonsäure,
   - gegebenenfalls substituiertem Sulfonyloxy (-O-SO₂R),
   - einem Sulfatrest,
   - gegebenenfalls substituiertem Alkyl,
   - gegebenenfalls substituiertem Alkoxy,
   - gegebenenfalls substituiertem Aryloxy,
   - gegebenenfalls substituiertem Alkenyl,
   - gegebenenfalls substituiertem Alkinyl, und
   - gegebenenfalls substituiertem Aryl;
      oder worin
      iii) mindestens einer der Reste R⁷, R⁸, R⁹, R¹⁰ und R¹¹ Methoxy ist und
         die übrigen Reste R⁷, R⁸, R⁹, R¹⁰ und R¹¹, die nicht Methoxy bedeuten, sowie die Reste R³, R⁴, R⁵ und R⁶ Wasserstoff sind;
      oder worin
      iv) R¹¹ ausgewählt ist aus der Gruppe die besteht aus:
   - gegebenenfalls substituiertem Acyloxy,
   - gegebenenfalls substituiertem Sulfonyloxy,
   - einem Sulfatrest,
   - gegebenenfalls substituiertem Alkoxy, wobei eine Aminocarbonylsubstituierte Alkoxy-Gruppe der allgemeinen Formel 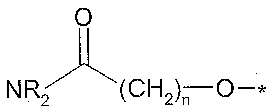 (n = 1 bis 7) ausgenommen ist, und
   - gegebenenfalls substituiertem Aryloxy, und
      R³, R⁴, R⁵, R⁶, R⁷, R⁸, R⁹ und R¹⁰ Wasserstoff sind;
      oder worin
      v) mindestens einer der Reste R⁷, R⁸, R⁹, R¹⁰ und R¹¹ sowie gegebenenfalls mindestens einer der Reste R³, R⁴, R⁵ und R⁶ gegebenenfalls substituiertes Alkoxy, ausgewählt aus einem über Sauerstoff gebundenen Zucker-Rest, ist
         und
         die übrigen Reste R³, R⁴, R⁵, R⁶, R⁷, R⁸, R⁹, R¹⁰ und R¹¹ die nicht die vorstehende Bedeutung eines über Sauerstoff gebundenen Zucker-Rests aufweisen, gleich oder verschieden sind und jeweils ausgewählt werden aus der Gruppe, die besteht aus:
   - Wasserstoff,
   - Hydroxy,
   - Halogen,
   - Cyano,
   - Nitro,
   - Carboxyl,
   - Alkoxycarbonyl,
   - Aminocarbonyl,
   - gegebenenfalls substituiertem Acyl,
   - gegebenenfalls substituiertem Acyloxy
   - gegebenenfalls substituiertem Sulfonyl (-SO₂R),
   - Sulfonsäure,
   - gegebenenfalls substituiertem Sulfonyloxy (-O-SO₂R),
   - einem Sulfatrest,
   - gegebenenfalls substituiertem Alkyl,
   - gegebenenfalls substituiertem Alkoxy,
   - gegebenenfalls substituiertem Aryloxy,
   - gegebenenfalls substituiertem Alkenyl,
   - gegebenenfalls substituiertem Alkinyl, und
   - gegebenenfalls substituiertem Aryl;
   oder pharmazeutisch verträgliche Salze davon.

Im Rahmen der gesamten Erfindung werden die vorstehend genannten Substituentengruppen wie folgt definiert:

Gegebenenfalls substituiertes Alkyl schließt im Rahmen der gesamten Erfindung bevorzugt ein:
Geradkettiges oder verzweigtes Alkyl mit 1 bis 8, bevorzugt 1 bis 6, Kohlenstoffatomen, Cycloalkyl mit 3 bis 8, bevorzugt 5 oder 6 Kohlenstoffatomen, oder Alkyl mit 1 bis 4 Kohlenstoffatomen, welches mit Cycloalkyl substituiert ist, die gegebenenfalls jeweils bevorzugt 1 bis 3 Substituenten tragen können, die bevorzugt aus der Gruppe ausgewählt werden, die besteht aus: Hydroxy, Halogen und Cyano.

Dabei schließt Halogen hier und im Rahmen der vorliegenden Erfindung Fluor, Chlor, Brom und lod, bevorzugt Fluor oder Chlor, ein.

Des Weiteren können ein oder mehrere, bevorzugter 1 bis 3 Kohlenstoffatome durch heteroanaloge Gruppen, die Stickstoff, Sauerstoff oder Schwefel enthalten, ersetzt sein. Dies bedeutet insbesondere, dass beispielsweise eine oder mehrere Methylengruppen in den Alkylresten durch NH, O oder S ersetzt sein können.

Beispiele von Alkylresten mit 1 bis 8 Kohlenstoffatomen schließen ein: eine Methylgruppe, eine Ethylgruppe, eine n-Propylgruppe, eine i-Propylgruppe, eine n-Butylgruppe, eine i-Butylgruppe, eine sec-Butylgruppe, eine t-Butylgruppe, eine n-Pentylgruppe, eine i-Pentylgruppe, eine sec-Pentylgruppe, eine t-Pentylgruppe, eine 2-Methylbutylgruppe, eine n-Hexylgruppe, eine 1-Methylpentylgruppe, eine 2-Methylpentylgruppe, eine 3-Methylpentylgruppe, eine 4-Methylpentylgruppe, eine 1-Ethylbutylgruppe, eine 2-Ethylbutylgruppe, eine 3-Ethylbutylgruppe, eine 1,1-Dimethylbutylgruppe, eine 2,2-Dimethylbutylgruppe, eine 3,3-Dimethylbutylgruppe, eine 1-Ethyl-1-methylpropylgruppe, eine n-Heptylgruppe, eine 1-Methylhexylgruppe, eine 2-Methylhexylgruppe, eine 3-Methylhexylgruppe, eine 4-Methylhexylgruppe, eine 5-Methylhexylgruppe, eine 1-Ethylpentylgruppe, eine 2-Ethylpentylgruppe, eine 3-Ethylpentylgruppe, eine 4-Ethylpentylgruppe, eine 1, 1 -Dimethylpentylgruppe, eine 2,2-Dimethylpentylgruppe, eine 3,3-Dimethylpentylgruppe, eine 4,4-Dimethylpentylgruppe, eine 1-Propylbutylgruppe, eine n-Octylgruppe, eine 1 -Methylheptylgruppe, eine 2-Methylheptylgruppe, eine 3-Methylheptylgruppe, eine 4-Methylheptylgruppe, eine 5-Methylheptylgruppe, eine 6-Methylheptylgruppe, eine 1-Ethylhexylgruppe, eine 2-Ethylhexylgruppe, eine 3-Ethylhexylgruppe, eine 4-Ethylhexylgruppe, eine 5-Ethylhexylgruppe, eine 1,1 -Dimethylhexylgruppe, eine 2,2-Dimethylhexylgruppe, eine 3,3-Dimethylhexylgruppe, eine 4,4-Dimethylhexylgruppe, eine 5,5-Dimethylhexylgruppe, eine 1-Propylpentylgruppe, eine 2-Propylpentylgruppe, usw. Bevorzugt sind solche mit 1 bis 6 Kohlenstoffatomen, insbesondere Methyl, Ethyl und n-Propyl. Am meisten bevorzugt ist Methyl.

Beispiele von Alkylgruppen, die durch Austausch mit einer oder mehreren heteroanalogen Gruppe, wie -O-, -S- oder -NH- hervorgehen sind bevorzugt solche, in denen eine oder mehrere Methylengruppe durch -O-unter Bildung einer Ethergruppe ersetzt sind, wie Methoxymethyl, Ethoxymethyl, 2-Methoxyethylen usw. Erfindungsgemäß sind auch Polyethergruppen von der Definition von Alkyl umfasst.

Cycloalkylreste mit 3 bis 8 Kohlenstoffatomen schließen bevorzugt ein: eine Cyclopropylgruppe, eine Cyclobutylgruppe, eine Cyclopentylgruppe, eine Cyclohexylgruppe, eine Cycloheptylgruppe, eine Cyclooctylgruppe usw, ein. Bevorzugt sind eine Cyclopropylgruppe, eine Cyclobutylgruppe, eine Cyclopentylgruppe und eine Cyclohexylgruppe. Heterocyclische Alkylreste, die durch Austausch von Methylen durch Heteroanaloge Gruppen aus Cycloalkyl gebildet werden, sind beispielsweise 5- oder 6-gliedrige heterocyclische Reste, wie Tetrahydrofuryl, Pyrrolidinyl, Piperidinyl oder Tetrahydropyranyl, welche gegebenenfalls mit aromatischen Ringen kondensiert sein können, etc.

Insbesondere schließen Beispiele eines mit Halogen substituierten linearen oder verzweigten Alkylrestes mit 1 bis 8 Kohlenstoffatomen ein:
eine Fluormethylgruppe, eine Difluormethylgruppe, eine
Trifluormethylgruppe, eine Chlormethylgruppe, eine Dichlormethylgruppe,
eine Trichlormethylgruppe, eine Brommethylgruppe, eine Dibrommethylgruppe, eine Tribrommethylgruppe, eine 1-Fluorethylgruppe, eine 1-Chlorethylgruppe, eine 1-Bromethylgruppe, eine 2-Fluorethylgruppe,
eine 2-Chlorethylgruppe, eine 2-Bromethylgruppe, eine 1,2-Difluorethylgruppe, eine 1 ,2-Dichlorethylgruppe, eine 1,2-Dibromethylgruppe, eine 2,2,2-Trifluorethylgruppe, eine
Heptafluorethylgruppe, eine 1 -Fluorpropylgruppe, eine 1 -Chlorpropylgruppe,
eine 1-Brompropylgruppe, eine 2-Fluorpropylgruppe, eine 2-Chlorpropylgruppe, eine 2-Brompropylgruppe, eine 3-Fluorpropylgruppe,
eine 3-Chlorpropylgruppe, eine 3-Brompropylgruppe, eine 1,2-Difluorpropylgruppe, eine 1 ,2-Dichlorpropylgruppe, eine 1 ,2-Dibrompropylgruppe, eine 2,3-Difluorpropylgruppe, eine 2,3-Dichlorpropylgruppe, eine 2,3-Dibrompropylgruppe, eine 3,3,3-Trifluorpropylgruppe, eine 2,2,3,3,3-Pentafluorpropylgruppe, eine 2-Fluorbutylgruppe, eine 2-Chlorbutylgruppe, eine 2-Brombutylgruppe, eine 4-Fluorbutylgruppe, eine 4-Chlorbutylgruppe, eine 4-Brombutylgruppe, eine 4,4,4-Trifluorbutylgruppe, eine 2,2,3,3,4,4,4-Heptafluorbutylgruppe, eine Perfluorbutylgruppe, eine 2-Fluorpentylgruppe, eine 2-Chlorpentylgruppe,
eine 2-Brompentylgruppe, eine 5-Fluorpentylgruppe, eine 5-Chlorpentylgruppe, eine 5-Brompentylgruppe, eine Perfluorpentylgruppe,
eine 2-Fluorhexylgruppe, eine 2-Chlorhexylgruppe, eine 2-Bromhexylgruppe,
eine 6-Fluorhexylgruppe, eine 6-Chlorhexylgruppe, eine 6-Bromhexylgruppe,
eine Perfluorhexylgruppe, eine 2-Fluorheptylgruppe, eine 2-Chlorheptylgruppe, eine 2-Bromheptylgruppe, eine 7-Fluorheptylgruppe,
eine 7-Chlorheptylgruppe, eine 7-Bromheptylgruppe, eine Perfluorheptylgruppe, usw.

Beispiele eines mit Hydroxy substituierten Alkylrestes schließen die oben genanten Alkylreste ein, die 1 bis 3 Hydroxylreste aufweisen, wie zum Beispiel Hydroxymethyl, 2-Hydroxyethyl, 3-Hydroxypropyl etc.

Beispiele eines mit Aryl substituierten Alkylrestes schließen die oben genannten Alkylreste ein, die 1 bis 3 Arylreste, wie nachstehend definiert, aufweisen, wie zum Beispiel wie zum Beispiel Phenylmethyl (Benzyl), 2-Phenylethyl, 2- oder 3-Phenylpropyl etc.. Beispiele von Heteroarylsubstituiertem Alkyl, schließen beispielsweise ein: 2-Pyridin-2-yl-ethyl, 2-Pyridin-3-yl-ethyl, Pyridin-2-yl-methyl, Pyridin-3-yl-methyl, 2-Furan-2-yl-ethyl, 2-Furan-3-yl-ethyl, Furan-2-yl-methyl, Furan-3-yl-methyl, 2-Thiophen-2-ylethyl, 2-Thiophen-3-yl-ethyl, Thiophen-2-yl-methyl, Thiophen-3-yl-methyl etc.. Bevorzugtes Arylalkyl ist Benzyl,

Besonders bevorzugt sind R¹ und R² jeweils Alkyl, und sind bevorzugt gleich (was wiederum bevorzugt ist) oder verschieden und jeweils geradkettiges oder verzweigtes Alkyl mit 1 bis 6, bevorzugt 1 bis 4, bevorzugter 1 bis 3 Kohlenstoffatomen, wobei lineares Alkyl bevorzugt ist. Bevorzugt sind R¹ und R² Methyl oder Ethyl. Am meisten bevorzugt sind R¹ und R² Methyl,

Gegebenenfalls substituiertes Alkenyl schließt im gesamten Rahmen der Erfindung bevorzugt ein:
Geradkettiges oder verzweigtkettiges Alkenyl mit 2 bis 8 Kohlenstoffatomen und Cycloalkenyl mit 3 bis 8 Kohlenstoffatomen, die gegebenenfalls durch bevorzugt 1 bis 3 Substituenten substituiert sein können, wie Hydroxy, Halogen oder Alkoxy. Beispiele schließen ein: Vinyl, 1-Methylvinyl, Allyl, 1-Butenyl, Isopropenyl, Cyclopropenyl, Cyclobutenyl, Cyclopentenyl, Cyclohexenyl. Bevorzugt sind Vinyl oder Allyl.

Gegebenenfalls substituiertes Alkinyl schließt im gesamten Rahmen der Erfindung bevorzugt ein:
Geradkettiges oder verzweigtkettiges Alkinyl mit 2 bis 8 Kohlenstoffatomen und Cycloalkinyl mit 5 bis 8 Kohlenstoffatomen, die gegebenenfalls durch bevorzugt 1 bis 3 gleichen oder verschiedenen Substituenten substituiert sein können. Bezüglich der Definition des gegebenenfalls substituierten Alkinyls wird auf die vorstehende Definition des gegebenenfalls substituierten Alkyls mit mehr als einem Kohlenstoffatom verwiesen, wobei die gegebenenfalls substituierten Alkine mindestens eine C≡C-Dreifachbindung umfassen. Beispiele schließen ein: Ethinyl, Propinyl, Butinyl, Pentinyl sowie gegebenenfalls wie vorstehend definierte substituierte Varianten davon. Bevorzugt ist Ethinyl sowie gegebenenfalls substituiertes Ethinyl.

Gegebenenfalls substituiertes Aryl schließt im gesamten Rahmen der Erfindung bevorzugt ein:
Aromatische Kohlenwasserstoffreste mit 6 bis 14 Kohlenstoffatomen (wobei die Kohlenstoffatome der Substituenten nicht mitgezählt sind) und 5- bis 10-gliedrige aromatische heterocyclische Reste (Heteroaryl) mit bis zu 3 Heteroatomen aus der Reihe S, O, N, welche mono- oder bicyclisch sein können und die durch bevorzugt 1 bis 3 Substituenten ausgewählt aus Hydroxy, Halogen, Cyano, Nitro, Alkyl, Acyl und Alkoxy substituiert sein können. Dabei kann bezüglich der Definition von Alkyl und Halogen auf die vorstehenden und bezüglich der Definition von Acyl und Alkoxy auf die nachfolgenden Definitionen bzw. Beispiele verwiesen werden.

Aromatische Kohlenwasserstoffreste mit 6 bis 14 Kohlenstoffatomen schließen beispielweise ein: Phenyl, Naphthyl, Phenanthrenyl und Anthracenyl, die gegebenenfalls substituiert sein können. Bevorzugt ist Phenyl.

Heteroaromatische Reste schließen beispielsweise ein: Pyridyl, Pyridyl-N-oxid, Pyrimidyl, Pyridazinyl, Pyrazinyl, Thienyl, Furyl, Pyrrolyl, Pyrazolyl, Imidazolyl, Thiazolyl, Oxazolyl oder Isoxazolyl, Indolizinyl, Indolyl, Benzo[b]thienyl, Benzo[b]furyl, indazolyl, Chinolyl, Isochinolyl, Naphthyridinyl, Chinazollnyl. 5-oder 6-gliedrige aromatische Heterocyclen wie z.B. Pyridyl, Pyridyl-N-oxid, Pyrimidyl, Pyridazinyl, Furyl und Thienyl sind bevorzugt.

Gegebenenfalls substituiertes Acyl schließt hier und im Folgenden ein: Gegebenenfalls substituiertes aliphatisches Acyl (Alkanoyl = Alkyl-CO-, worin bezüglich der Alkylgruppe auf vorstehende Definition von gegebenenfalls substituiertem Alkyl verwiesen werden kann). Sowie gegebenenfalls substituiertes aromatisches oder heteroaromatisches Acyl (Aroyl = Aryl-CO-), worin bezüglich Aryl auf vorstehende Definition von gegebenenfalls substituiertem Aryl verwiesen werden kann.

Alkanoyl ist bevorzugt: Cl bis C6 Alkanoyl, wie Formyl, Acetyl, Propionyl, Butyryl, Isobutyryl, Valeryl, Isovaleryl, Pivaloyl, Hexanoyl, usw. Bevorzugt ist Cl bis C5 Alkanoyl, besonders bevorzugt ist Formyl und Acetyl.

Aroyl ist bevorzugt: C5 bis C10 Aroyl, wie Benzoyl, wie gegebenenfalls substituiertes Benzoyl, Toluoyl, Xyloyl, usw. Definitionsgemäß schließt Aroyl auch Hetaroyl (= Heteroaryl-CO) ein. Hetaroyl ist bevorzugt C5 bis C10 Hetaroyl, wie Furanoyl (wie Furan-2-carbonyl, Furan-3-carbonyl, Thienoyl (wie Thiophen-2-carbonyl, Thiophen-3-carbonyl), Pyridoyl (wie Pyridin-2-carbonyl, Pyridin-3-carbonyl) etc..

Alkoxy schließt eine gegebenenfalls substituierte Alkyl-O-Gruppe ein, worin hinsichtlich der Definition der Alkylgruppe auf vorstehende Definition verwiesen werden kann. Bevorzugte Alkoxygruppen sind lineare oder verzweigte Alkoxygruppen mit bis zu 6 Kohlenstoffatomen, wie eine Methoxygruppe, eine Ethoxygruppe, eine n-Propyloxygruppe, eine i-Propyloxygruppe, eine n-Butyloxygruppe, eine i-Butyloxygruppe, eine sec-Butyloxygruppe, eine t-Butyloxygruppe, eine n-Pentyloxygruppe, eine i-Pentyloxygruppe, eine sec-Pentyloxygruppe, eine t-Pentyloxygruppe, eine 2-Methylbutoxygrupe, eine n-Hexyloxygruppe, eine i-Hexyloxygruppe, eine t-Hexyloxygruppe, eine sec-Hexyloxygruppe, eine 2-Methylpentyloxygruppe, eine 3-Methylpentyloxygruppe, eine 1 -Ethylbutyloxygruppe, eine 2-Ethylbutyloxygruppe, eine 1,1-Dimethylbutyloxygruppe, eine 2,2-Dimethylbutyloxygruppe, eine 3,3-Dimethylbutyloxygruppe, eine 1-Ethyl-1-methylpropyloxygruppe, usw., sowie Cycloalkyloxy-Gruppen wie eine Cyclopentyloxygruppe oder eine Cyclohexyloxygruppe etc..

Gegebenenfalls substituiertes Alkoxy schließt im Rahmen der vorstehenden Erfindung außerdem einen über Sauerstoff gebundenen Zucker-Rest (-O-Z) ein. Darin umfasst der Begriff Zucker-Rest (-Z) im Sinne der vorliegenden Erfindung Aldosen und Ketosen mit einer Kettenlänge von 3 bis 7, bevorzugt 4 bis 6 Kohlenstoffatomen, wobei Pentosen (5 Kohlenstoffatome) und Hexosen (6 Kohlenstoffatome) besonders bevorzugt sind. Der Begriff Zucker-Rest im Sinne der vorliegenden Erfindung umfasst insbesondere Mono-, Di- und Tri-Saccharide.
Die Gruppe der Monosaccharide umfasst beispielsweise Triosen (3 Kohlenstoffatome) wie D- und L-Glycerinaldehyd (Aldotriosen) sowie Dihydroxyaceton (Ketotriose), Tetrosen (4 Kohlenstoffatome) wie Erythrose und Threose (Aldotetrosen) sowie Erythrulose (Ketotetrose), Pentosen (5 Kohlenstoffatome) wie Ribose, Arabinose, Xylose, Lyxose und Desoxyribose (Aldopentosen) sowie Ribulose und Xylulose (Ketopentosen), Hexosen (6 Kohlenstoffatome) wie Allose, Altrose, Glucose, Mannose, Gulose, Idose, Galaktose, Talose und Rhamnose (Aldohexosen) sowie Fructose und Sorbose (Ketohexosen) etc..
Die Gruppe der Di-Saccharide umfasst beispielsweise Cellobiose, Gentiobiose, Isomaltose, Isomaltulose, Lactose, Lactulose, Laminaribiose, Maltose, Maltulose, Melibiose, Neohesperidose, Neothrehalose, Nigerose, Palatinose, Rutinose, Sophorose, Saccharaso, Trehalose etc..
Die Zucker-Reste können offenkettig oder in Form der cyclischen Halbacetale vorliegen. Definitionsgemäß sind die D- und L-Enantiomere sowie Mischungen davon und Racemate umfasst, Im Sinne der vorliegenden Erfindung schließt der Begriff Zucker-Rest außerdem Zucker-Derivate, wie beispielsweise Aminozucker (z.B. Glucosamin, N-Acetyl-D-glucosamin, N-Acetyl-D-galactosamin), Desoxyzucker (z.B, Fucose, Desoxyribose und Dicarbonsäuren, Polyhydroxycarbonsäuren oder Zuckersäuren der vorstehend genannten Zucker, wie insbesondere Aldonsäuren, Aldarsäuren und Uronsäuren und ihre Salze wie z.B. Gluconsäure, Glucarsäure, Glucuronsäure bzw. Glucuronat etc., Galactonsäure, Galactarsäure, Galacturonsäure etc.. Ebenfalls umfasst sind Lactone der Zuckersäuren. Besonders bevorzugte Zuckerreste sind ausgewählt aus der Gruppe der Zuckersäuren, insbesondere aus der Gruppe der Uronsäuren wie insbesondere Glucuronsäure. Erfindungsgemäß liegt der über einen Sauerstoff gebundene Zuckerrest bevorzugt in Form einer sogenannten glykosidischen Bindung (O-glykosidische Bindung) vor. Dabei bezeichnet eine glykosidische Bindung die chemische Bindung zwischen dem anomeren Kohlenstoffatom des Zuckerrests (Glycon) und dem Heteroatom eines Aglycons, entsprechend dem Sauerstoffatom über den der Zuckerrest an den Grundkörper gebunden wird, wobei das Brücken-Sauerstoffatom von der Alkohol- bzw. Phenolfunktion des Grundkörpers (Aglycon) stammt. Verbindungen, die eine glycosidische Bindung enthalten, werden im Folgenden auch als Glycoside bezeichnet. Bei einem Glycosid liegt die Aldehydfunktion der Aldosen (z. B. Glucose) oder die Ketofunktion der Ketosen (z. B. Fructose) als zyklisches Vollacetal vor. Dabei bezeichnet ein Acetal das Kondensationsprodukt aus einem Aldehyd oder Keton (z.B. in Form des cyclischen Halbacetals) und einem oder zwei Alkoholen unter Bildung des Vollacetals.
Bevorzugte glykosidisch gebundene Zuckerreste sind ausgewählt aus über einen Sauerstoff (O-glykosidisch) an den Phenylkern gebundener Glucuronsäure, entsprechend der Formel:

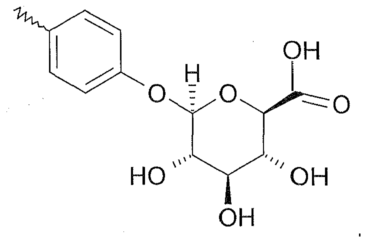

Aus der Gruppe der gegebenenfalls substituierten Alkoxy-Reste sind bevorzugt eine Methoxygruppe, eine Ethoxygruppe, eine n-Propyloxygruppe, eine i-Propyloxygruppe, eine n-Butyloxygruppe, eine 1-Butyloxygruppe, eine sec-Butyloxygruppe, eine t-Butyloxygruppe sowie über einen Sauerstoff gebundene Zuckerreste. Besonders bevorzugt ist die Methoxygruppe sowie ein über einen Sauerstoff gebundener Zuckerrest, insbesondere ein Zuckersäurerest, der besonders bevorzugt ein Glucuronsäurerest ist.

Im Rahmen der vorliegenden Erfindung ist im Fall iv), worin
R¹¹ ausgewählt ist aus der Gruppe die besteht aus:
- gegebenenfalls substituiertem Acyloxy,
- gegebenenfalls substituiertem Sulfonyloxy,
- einem Sulfatrest,
- gegebenenfalls substituiertem Alkoxy, und
- gegebenenfalls substituiertem Aryloxy; und worin
R³, R⁴, R⁵, R⁶, R⁷, R⁸, R⁹ und R¹⁰ Wasserstoff sind,
eine Aminocarbonyl-substituierte Alkoxy-Gruppe, insbesondere eine Aminocarbonyl-substituierte Methoxy- und/oder Ethoxy-Gruppe von der Definition von gegebenenfalls substituiertem Alkoxy ausgenommen. Eine entsprechende Aminocarbonylsubstituierte Alkoxy-Gruppe entspricht beispielsweise der allgemeinen Formel 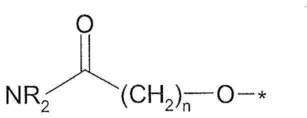 (n = 1 bis 7; * markiert die Bindungsposition zum Grundgerüst).

Aryloxy schließt eine gegebenenfalls substituierte Aryl-O-Gruppe ein, worin hinsichtlich der Definition der Arylgruppe auf vorstehende Definition von Aryl verwiesen werden kann. Bevorzugte Aryloxy-Gruppen umfassen 5- und 6-gliedrige Aryl-Gruppen, worunter Phenoxy, welches gegebenenfalls substituiert sein kann, bevorzugt ist.

Entsprechend der vorstehenden Definition von Aryl schließt Aryloxy auch eine gegebenenfalls substituierte Heteroaryl-O-Gruppe (Heteroaryloxy-Gruppe) ein. Bevorzugte Heteroaryloxy-Gruppen umfassen 5- und 6-gliedrige Heteroaryloxy-Gruppen, worunter Pyridin-2-yloxy, Pyridin-3-yloxy, Thiophen-2-yloxy, Thiophen-3-yloxy, Furan-2-yloxy, Furan-3-yloxy bevorzugt sind.

Acyloxy schließt eine gegebenenfalls substituierte Acyl-O-Gruppe (-O-CO-R mit Alkanoyloxy = -O-CO-Alkyl, Aroyloxy = -O-CO-Aryl und Hetaroyloxy = -O-CO-Heteroaryl) ein, worin hinsichtlich der Definition der Acylgruppe auf vorstehende Definition von gegebenenfalls substituiertem Acyl verwiesen werden kann, Bevorzugte Acyloxygruppen sind Formyloxy, Acetyloxy und Benzoyloxy,

Die Substituenten R³ bis R¹¹ können außerdem die Bedeutung von Carboxyl- bzw. eines Carboxylrests (-CO-OH) sowie von Sulfonsäure bzw. eines Sulfonsäurerests (-SO₂-OH /-SO₃H) oder eines Sulfatrests (-O-SO₂-OH), bzw. jeweils eines pharmazeutisch verträglichen Salzes davon, aufweisen.

Gegebenenfalls substituiertes Alkoxycarbonyl (-CO-OR / -(C=O)-OR) schließt hinsichtlich der Definition von Alkoxy oben erwähntes gegebenenfalls substituiertes Alkoxy ein, bevorzugt ist Methoxycarbonyl und Ethoxycarbonyl,

Gegebenenfalls substituiertes Aminocarbonyl stellt im Rahmen der gesamten Erfindung bevorzugt Carbamoyl (H₂NCO-) oder Mono- oder Dialkylaminocarbonyl (H(Alkyl)N-CO- oder (Alkyl)₂N-CO-) dar, worin hinsichtlich der Definition von Alkyl auf die oben stehenden Erläuterungen für gegebenenfalls substituiertes Alkyl verwiesen werden kann.

Des Weiteren stellt gegebenenfalls substituiertes Sulfonyl (-SO₂-R) im Rahmen der gesamten Erfindung gegebenenfalls substituiertes Alkylsulfonyl (Alkyl-SO₂-) sowie gegebenenfalls substituiertes Arylsulfonyl (Aryl-SO₂-) dar, worin hinsichtlich der Definition von Alkyl und Aryl auf die oben stehenden Erläuterungen für gegebenenfalls substituiertes Alkyl und gegebenenfalls substituiertes Aryl verwiesen werden kann. Damit schließt Arylsulfonyl definitionsgemäß auch Heteroarylsulfonyl (Heteroaryl-SO₂-) ein. Besonders bevorzugt sind Methylsulfonyl, Ethylsulfonyl, Phenylsulfonyl, Tolylsulfonyl oder Benzylsulfonyl.

Gegebenenfalls substituiertes Sulfonyl kann des Weiteren die Bedeutung einer gegebenenfalls substituierten Aminosulfonyl-Gruppe (-SO₂-NR₂), insbesondere Sulfamoyl (H₂N-SO₂-) oder Mono- oder Dialkylaminosulfonyl (Alkyl)₂N-SO₂-) aufweisen, wobei hierin hinsichtlich der Definition von Alkyl auf die oben stehenden Erläuterungen für gegebenenfalls substituiertes Alkyl verwiesen werden kann.

Sulfonyloxy schließt eine gegebenenfalls substituierte Sulfonyl-O-Gruppe (-O-SO₂-R mit Alkylsulfonyloxy = -O-SO₂-Alkyl, Arylsulfonyloxy = -O-SO₂-Aryl und Heteroarylsulfonyloxy = -O-SO₂-Heteroaryl, sowie Aminosufonyloxy = -O-SO₂-NR₂) ein, worin hinsichtlich der Definition der Sulfonylgruppe auf vorstehende Definition von gegebenenfalls substituiertem Sulfonyl verwiesen werden kann. Bevorzugte Sulfonyloxygruppen sind Methylsulfonyloxy, Ethylsulfonyloxy, Phenylsulfonyloxy, Tolylsulfonyloxy oder Benzylsulfonyloxy,

In der allgemeinen Formel (1) wird X¹ aus der Gruppe ausgewählt, die besteht aus:
- einer Einfachbindung
- Carbonyl (-CO-),
- Schwefel (-S-),
- Sauerstoff (-O-),
- Sulfoxy (-SO-),
- Sulfonyl (-SO₂-),
- Azo (-N=N-), und
- einem gegebenenfalls substituierten, gesättigten oder ungesättigten aliphatischen Rest mit 1 bis 6 Kohlenstoffatomen.

Ein gegebenenfalls substituierter, gesättigter oder ungesättigter aliphatischer Rest mit 1 bis 6 Kohlenstoffatomen für X¹ schließt im Rahmen der vorliegenden Erfindung ein: gegebenenfalls substituiertes Alkandiyl, wie oben definiert, gegebenenfalls substituiertes Alkendiyl, wie oben definiert, und gegebenenfalls substituiertes Alkindiyl, wie oben definiert, ein. Bevorzugt ist X¹ Alkandiyl, Alkendiyl oder Alkindiyl mit bis zu 4, bevorzugter mit bis zu 2 Kohlenstoffatomen, wie Methylen (-CH₂-), welcher gegebenenfalls durch Hydroxyl substituiert sein kann (wie zum Beispiel -CH(OH)-),

Am meisten bevorzugt sind Verbindungen der allgemeinen Formel (1) in denen X¹ Carbonyl (-CO-) ist.

Im Rahmen der vorliegenden Erfindung sind insbesondere die folgenden Ausführungsformen bevorzugt:
1. Bevorzugte Ausführungsform:
   R¹ und R² sind jeweils Alkyl und
   X¹ ist Carbonyl (-CO-); und
   die Substituenten R³ bis R¹¹ in der allgemeinen Formel (1) werden unter den vorstehend definierten Alternativen i) bis v) mit der darin definierten Bedeutung der Substituenten ausgewählt.
   Dabei sind R¹ und R² gleich oder verschieden und bevorzugt ausgewählt aus Methyl und Ethyl, In einer ganz besonders bevorzugten Ausführungsform sind R¹ und R² gleich und weisen die Bedeutung von Methyl oder Ethyl, bevorzugt von Methyl auf,
2. Bevorzugte Ausführungsform:
   R¹, R² und X¹ weisen die vorstehend definierte allgemeine Bedeutung oder die Bedeutung der vorstehenden bevorzugten Ausführungsform 1 auf und die Gruppen R³ bis R¹⁰ und R¹¹ in der allgemeinen Formel (1) werden im Rahmen der vorliegenden Erfindung unter den folgenden Alternativen ausgewählt:
      i) R¹¹ ist Hydroxy und
         R³, R⁴, R⁵, R⁶, R⁷, R⁸, R⁹ und R¹⁰ bedeuten Wasserstoff;
         oder
      ii) mindestens einer der Reste R⁷, R⁸, R⁹ und R¹⁰ sowie gegebenenfalls mindestens einer der Reste R³, R⁴, R⁵ und R⁶ ist Hydroxy und
         die übrigen Reste R³, R⁴, R⁵, R⁶, R⁷, R⁸, R⁹ und R¹⁰, die von Hydroxy verschieden sind, sowie der Rest R¹¹ sind gleich oder verschieden und werden jeweils ausgewählt aus der Gruppe, die besteht aus:
         - Wasserstoff,
         - Halogen,
         - Cyano,
         - Carboxyl,
         - Alkoxycarbonyl,
         - gegebenenfalls substituiertem Acyl,
         - gegebenenfalls substituiertem Acyloxy
         - gegebenenfalls substituiertem Sulfonyl (-SO₂R),
         - gegebenenfalls substituiertem Sulfonyloxy (-O-SO₂R),
         - gegebenenfalls substituiertem Alkyl,
         - gegebenenfalls substituiertem Alkoxy,
         - gegebenenfalls substituiertem Aryloxy, und
         - gegebenenfalls substituiertem Aryl;
         oder
      iii) der Rest R¹¹ sowie gegebenenfalls mindestens einer der Reste R⁷, R⁸, R⁹ und R¹⁰ ist Methoxy und
         die übrigen Reste R⁷, R⁸, R⁹ und R¹⁰, die von Methoxy verschieden sind, sowie die Reste R³, R⁴, R⁵ und R⁶ bedeuten Wasserstoff;
         oder
      iv) R¹¹ ist ausgewählt aus der Gruppe die besteht aus:
         - gegebenenfalls substituiertem Alkoxy, wobei eine Aminocarbonylsubstituierte Alkoxy-Gruppe, insbesondere eine Aminocarbonylsubstituierte Alkoxy-Gruppe der allgemeinen Formel 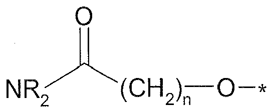 (n = 1 bis 7) ausgenommen ist,
         - gegebenenfalls substituiertem Sulfonyloxy, und
         - einem Sulfatrest; und
         R³, R⁴, R⁵, R⁶, R⁷, R⁸, R⁹ und R¹⁰ sind Wasserstoff;
         oder
      v) mindestens einer der Reste R⁷, R⁸, R⁹, R¹⁰ und R¹¹ sowie gegebenenfalls mindestens einer der Reste R³, R⁴, R⁵ und R⁶ ist gegebenenfalls substituiertes Alkoxy, welches ausgewählt ist aus einem über Sauerstoff gebundenen Zucker-Rest,
         und
         die übrigen Reste R³, R⁴, R⁵, R⁶, R⁷, R⁸, R⁹, R¹⁰ und R¹¹ die nicht die vorstehende Bedeutung eines über Sauerstoff gebundenen Zucker-Rests aufweisen, sind gleich oder verschieden und werden jeweils ausgewählt aus der Gruppe, die besteht aus:
         - Wasserstoff,
         - Hydroxy,
         - Halogen,
         - Cyano,
         - Carboxyl,
         - Alkoxycarbonyl,
         - gegebenenfalls substituiertem Acyl,
         - gegebenenfalls substituiertem Acyloxy
         - gegebenenfalls substituiertem Sulfonyl (-SO₂R),
         - gegebenenfalls substituiertem Sulfonyloxy (-O-SO₂R),
         - gegebenenfalls substituiertem Alkyl,
         - gegebenenfalls substituiertem Alkoxy,
         - gegebenenfalls substituiertem Aryloxy, und
         - gegebenenfalls substituiertem Aryl.
3. Weitere bevorzugte Ausführungsformen
   In weiteren bevorzugten Ausführungsformen können die Gruppen R³ bis R¹⁰ und R¹¹ in der allgemeinen Formel (1) außerdem unter den folgenden Alternativen ausgewählt werden:
   ii) einer der Reste R⁷, R⁸, R⁹ und R¹⁰ ist Hydroxy ist und
      die übrigen Reste R⁷, R⁸, R⁹ und R¹⁰, die nicht Hydroxy bedeuten, sowie die Reste R³, R⁴, R⁵ und R⁶ sind wie in einer der vorstehenden Ausführungsformen definiert. Bevorzugt sind die übrigen Reste R⁷, R⁸, R⁹ und R¹⁰, die nicht Hydroxy bedeuten, sowie die Reste R³, R⁴, R⁵ und R⁶ Wasserstoff;
      oder
   iii) der Rest R¹¹ ist Methoxy und
      die Reste R³, R⁴, R⁵, R⁶ R⁷, R⁸, R⁹ und R¹⁰ bedeuten Wasserstoff;
      oder
   iv) R¹¹ wird ausgewählt aus der Grupppe die besteht aus:
      - gegebenenfalls substituiertem Alkoxy, wobei eine Aminocarbonylsubstituierte Alkoxy-Gruppe der allgemeinen Formel 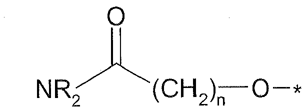 (n = 1 bis 7) ausgenommen ist, insbesondere wird die gegebenenfalls substituierte Alkoxy-Gruppe ausgewählt aus Methoxy, entsprechend auch der bevorzugten Variante (iii), und
      - einem Sulfatrest; und
         R³, R⁴, R⁵, R⁶, R⁷, R⁸, R⁹ und R¹⁰ sind Wasserstoff;
         oder
   v) einer der Reste R⁷, R⁸, R⁹, R¹⁰ und R¹¹ ist gegebenenfalls substituiertes Alkoxy, ausgewählt aus einem über Sauerstoff gebundenen Zucker-Rest, und
      die übrigen Reste R⁷, R⁸, R⁹ und R¹⁰, die nicht die vorstehende Bedeutung eines über Sauerstoff gebundenen Zucker-Rests aufweisen, sowie die Reste R³, R⁴, R⁵ und R⁶ sind wie in einer der vorstehenden Ausführungsformen definiert. Bevorzugt sind die übrigen Reste R⁷, R⁸, R⁹ und R¹⁰, die nicht die vorstehende Bedeutung eines über Sauerstoff gebundenen Zucker-Rests aufweisen, sowie die Reste R³, R⁴, R⁵ und R⁶ Wasserstoff;
      oder die Alternative v) wird wie folgt ausgewählt:
   v) einer der Reste R⁷, R⁸, R⁹, R¹⁰ und R¹¹, besonders bevorzugt R¹¹, ist ein über Sauerstoff gebundener Zucker-Rest, welcher ausgewählt ist
   aus einem über Sauerstoff gebundenen Zuckersäure-Rest, insbesondere einem Glucuronsäure-Rest, und
   die übrigen Reste R⁷, R⁸, R⁹ und R¹⁰, die nicht die vorstehende Bedeutung eines über Sauerstoff gebundenen Zuckersäure-Rests bzw, eines Glucuronsäure-Rests aufweisen, sowie die Reste R³, R⁴, R⁵ und R⁶ sind wie in einer der vorstehenden Ausführungsformen definiert und weisen bevorzugt die Bedeutung von Wasserstoff auf.

In diesen weiteren bevorzugten Ausführungsformen weisen R¹, R² und X¹ jeweils die vorstehend definierte allgemeine Bedeutung oder die Bedeutung der vorstehenden bevorzugten Ausführungsform 1 auf.

Besonders bevorzugt sind die Verbindungen, die aus der Gruppe ausgewählt werden, die besteht aus: 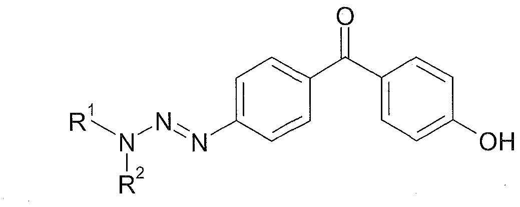 (entsprechend Alternative i)), 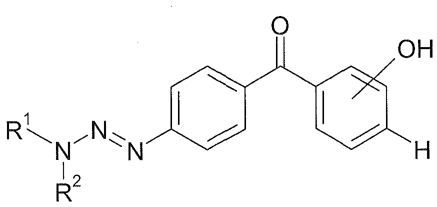 (entsprechend Alternative ii)), 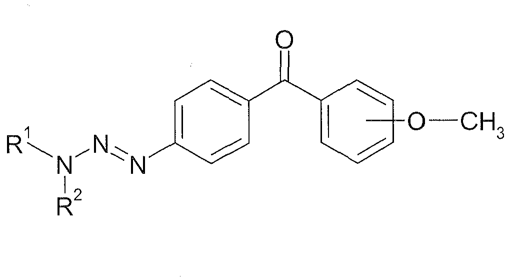 (entsprechend Alternative iii)),
insbesondere 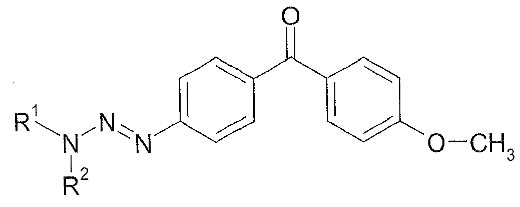 (entsprechend Alternative iii) und iv)),
und 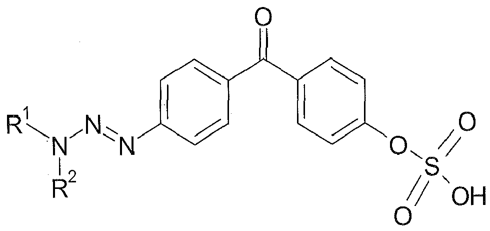 (entsprechend Alternative iv)), sowie 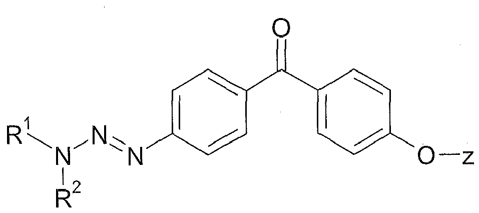 (entsprechend Alternative v));
worin jeweils R¹ und R² die Bedeutung wie im Rahmen der vorliegenden Erfindung definiert aufweisen und worin Z für einen Zucker-Rest, wie vorstehend definiert, steht;
oder pharmazeutisch verträgliche Salze davon.

Am meisten bevorzugt sind die Verbindungen, die aus der Gruppe ausgewählt werden, die besteht aus: 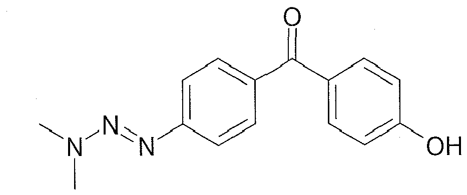 (entsprechend Alternative i)), 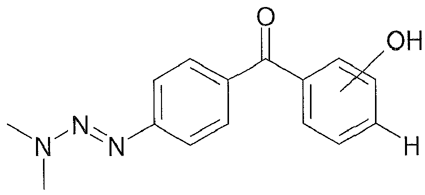 (entsprechend Alternative ii)), 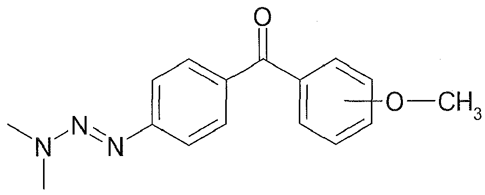 (entsprechend Alternative iii)), insbesondere 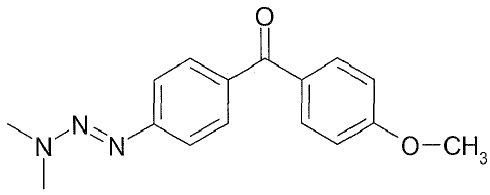 (entsprechend Alternative iii) und iv),
und 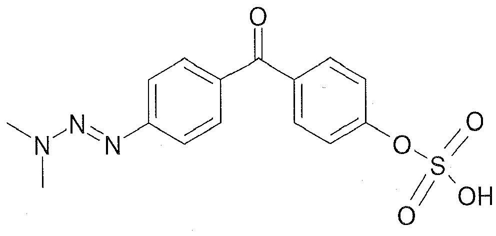 (entsprechend Alternative iv)), sowie 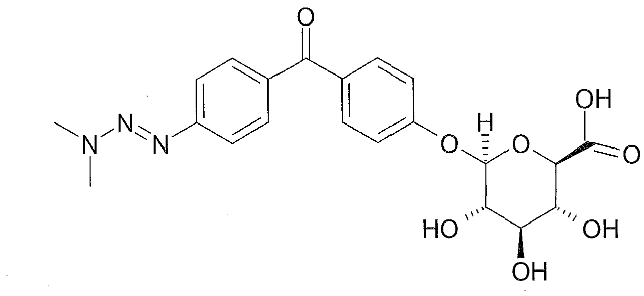 entsprechend Alternative v));
oder pharmazeutisch verträgliche Salze davon,

(In diesen Strukturformeln bedeutet dabei beispielsweise ein Strukturelement der Formel 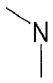 eine Dimethylaminogruppe, d.h. die Methylgruppen sind durch einfache Striche dargestellt, eine Schreibweise, die dem Fachmann gut bekannt ist.

Die Schreibweise des (der) Substituenten: 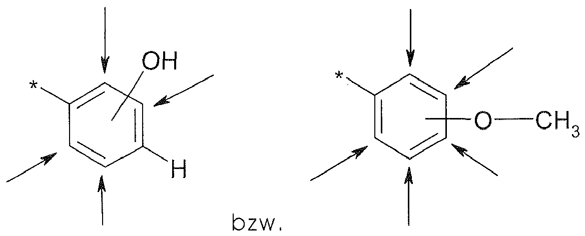 bedeutet, dass der Substituent -OH bzw, -OCH₃ an den mit den Pfeilen gekennzeichneten Substituentenpositionen gebunden sein kann. *markiert die Bindungsstelle zum Grundgerüst.

Erfindungsgemäße Triazen-Verbindungen, die basische Gruppen enthalten, können in Form ihrer pharmazeutisch annehmbaren Salze mit pharmazeutisch annehmbaren Säuren angewendet werden, wie z.B. Salze mit Mineralsäuren, Carbonsäuren und Sulfonsäuren, wie zum Beispiel mit Salzsäure, Bromwasserstoffsäure, Iodwasserstoffsäure, Schwefelsäure, Phosphorsäure, Weinsäure, Methansulfonsäure, Hydroxyethansulfonsäure, Acetursäure (Acetylglycin), Maleinsäure, Propionsäure, Fumarsäure, Toluolsulfonsäure, Benzolsulfonsäure, Trifluoressigsäure, Naphthalindisulfonsäure-1,5, Salicylsäure, Benzoesäure, Milchsäure, Äpfelsäure, 3-Hydroxy-2-naphthoesäure-2, Zitronensäure oder Essigsäure.

Erfindungsgemäße Triazen-Verbindungen, die saure Gruppen enthalten, können in Form ihrer pharmazeutisch annehmbaren Salze mit pharmazeutisch annehmbaren Basen angewendet werden, wie z.B. Salze mit Alkali- oder Erdalkalihydroxiden, wie NaOH, KOH, Ca(OH)₂, Mg(OH)₂ etc., Aminverbindungen, wie Ethylamin, Diethylamin, Triethylamin, Ethyldiisopropylamin, Ethanolamin, Diethanolamin, Triethanolamin, Methylglucamin, Dicyclohexylamin, Dimethylaminoethanol, Prokain, Dibenzylamin, N-Methylmorpholin, Arginin, Lysin, Ethylendiamin, N-Methylpiperidin 2-Amino-2-methyl-propanol-(1), 2-Amino-2-methyl-propandiol-(1,3), 2-Amino-2-hydroxyl-methyl-propandiol-(1,3) (TRIS) etc..

Erfindungsgemäß sind sowohl die freien Säuren als auch deren pharmazeutisch annehmbaren Salze vom Umfang der Erfindung umfasst.

Die Wasserlöslichkeit bzw. die Löslichkeit in physiologischer Kochsalzlösung und damit gegebenenfalls auch die Wirksamkeit der erfindungsgemäßen Verbindungen kann durch Salzbildung generell, speziell auch durch die Wahl des Gegenions signifikant beeinflusst werden.

Die Wasserlöslichkeit bzw, die Löslichkeit in physiologischer Kochsalzlösung und damit gegebenenfalls auch die Wirksamkeit der erfindungsgemäßen Verbindungen hängt aber unter Umständen auch von der Grundstruktur der Verbindungen selbst signifikant ab. Eine hohe Wasserlöslichkeit der erfindungsgemäßen Verbindungen ist nicht unbedingt entscheidend. Beispielsweise ist eine besonders bevorzugte erfindungsgemäße Verbindung (Beispielverbindung 1) stark lipophil.
Die verbesserte Wirksamkeit und erhöhte Zellpermeabilität solcher erfindungsgemäßen Triazenverbindungen, die mit einem über einen Sauerstoff gebundenen Zuckerrest, insbsondere mit einem glykosidisch gebundenen Uronsäurerest wie bevorzugt mit einem Glucuronsäurerest substituiert sind, kann dabei darauf zurückgeführt werden, dass derartige Triazenverbindungen als Stoffwechselmetaboliten eine hohe Bioverfügbarkeit im menschlichen und tierischen Körper bewirken. So konnte eine deutlich höhere Wirkung für ein Glucuronid einer Schering-Plough Substanz gezeigt werden, da das Glucuronid in der Darmwand akkumulierte und nicht wie die Muttersubstanz über Portalplasma in der Leber [BJ Pharmacology 2000, 129; S1748-1754].

Die Verwendung von 2-Amino-2-hydroxyl-methyl-propandiol-(1 ,3) (TRIS)- und Natrium-Salzen ist vor dem Hintergrund der Erhöhung der Wasserlöslichkeit der erfindungsgemäßen Verbindungen bevorzugt.

Die erfindungsgemäßen Verbindungen können in Abhängigkeit von ihrer Struktur bei Vorliegen asymmetrischer Kohlenstoffatome in stereoisomeren Formen (Enantiomere, Diastereomere) existieren. Die Erfindung umfasst deshalb die Verwendung der Enantiomeren oder Diastereomeren und ihrer jeweiligen Mischungen. Die enantiomerenreinen Formen können gegebenenfalls durch übliche Verfahren der optischen Auflösung, wie durch fraktionierte Kristallisation von Diastereomeren daraus durch Umsetzung mit optisch aktiven Verbindungen erhalten werden. Sofern die erfindungsgemäßen Verbindungen in tautomeren Formen vorkommen können, umfasst die vorliegende Erfindung die Verwendung sämtlicher tautomerer Formen.

Die vorliegende Erfindung betrifft weiterhin ein Verfahren zur Herstellung der Verbindungen der Formel (1), mit der Bedeutung der Substituenten gemäß Alternative i) oder ii), welches die Schritte umfasst:

Umsetzung einer Verbindung der Formel (2) 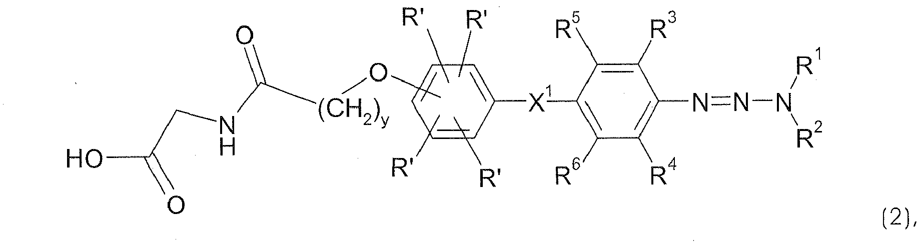 oder einem Salz, wie insbesondere einem Natrium-, Kalium oder Calciumsalz davon,
durch Hydrolyse der Peptidbindung zu Verbindungen der Formel (2') 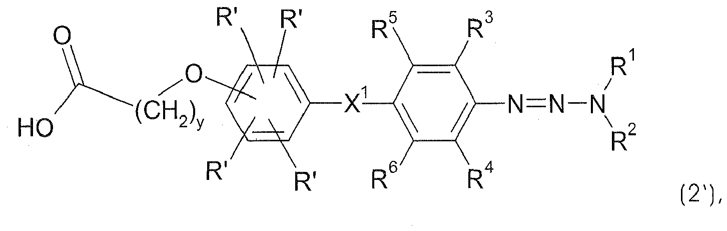 oder einem Salz, wie insbesondere einem Natrium-, Kalium oder Calciumsalz davon,
worin jeweils R' die entsprechende Bedeutung der Substituenten R⁷ bis R¹¹ und die Substituenten R¹ bis R⁶ die Bedeutung gemäß einer oder mehrerer der vorstehenden Definitionen, insbesondere die der Alternativen i) oder ii) aufweist und worin jeweils y = 1 oder 2 bedeutet,
und/oder

Umsetzung einer Verbindung der Formel (2') oder einem Salz, wie insbesondere einem Natrium-, Kalium oder Calciumsalz davon, mittels beta-Eliminierung zu Verbindungen der Formel (1), mit der Bedeutung der Substituenten gemäß einer oder mehrerer der vorstehenden Definitionen der Alternativen 1) oder ii)

Gegebenenfalls kann der Wasserstoff der Hydroxy-Gruppe anschließend mit geeigneten Substituenten weiter zu Verbindungen der Formel (1) mit der Bedeutung der Substituenten gemäß einer oder mehrerer der vorstehenden Definitionen der Alternativen iii) oder iv) umgesetzt werden.

Insbesondere ist im Rahmen der vorliegenden Erfindung bevorzugt ein Verfahren zur Herstellung der Verbindungen der Formel (1) mit der Bedeutung der Substituenten gemäß Alternative i), welches die Schritte umfasst:

Umsetzung einer Verbindung der Formel (3) 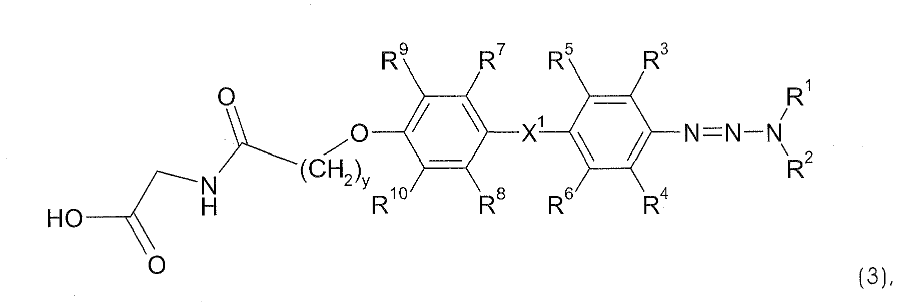 oder einem Salz, wie insbesondere einem Natrium-, Kalium oder Calciumsalz davon,
durch Hydrolyse der Peptidbindung zu Verbindungen der Formel (3') 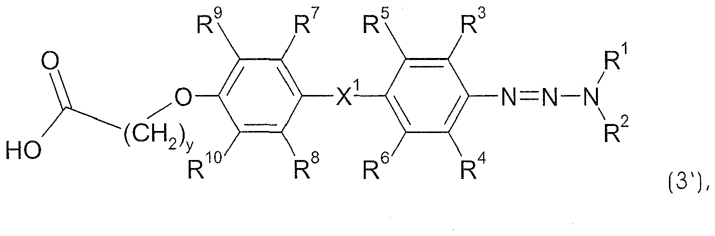 oder einem Salz, wie insbesondere einem Natrium-, Kalium oder Calciumsalz davon,
worin die Substituenten R¹ bis R¹⁰ die Bedeutung gemäß einer oder mehrerer der vorstehenden Definitionen, insbesondere die der Alternative i) aufweist und worin jeweils y = 1 oder 2 bedeutet,
und/oder

Umsetzung einer Verbindung der Formel (3') oder einem Salz davon mittels beta-Eliminierung zu Verbindungen der Formel (1') 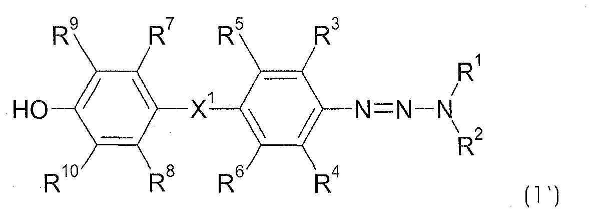 worin jeweils die Substituenten R¹ bis R¹⁰ die Bedeutung einer oder mehrerer der vorstehenden Definitionen aufweisen.

Gegebenenfalls kann der Wasserstoff der Hydroxy-Gruppe (an Position R¹¹) anschließend mit geeigneten Substituenten weiter zu Verbindungen der Formel (1) mit der Bedeutung der Substituenten gemäß einer oder mehrerer der vorstehenden Definitionen der Alternativen iii) oder iv) umgesetzt werden.

Die vorliegende Erfindung betrifft weiterhin ein Verfahren zur Herstellung der Verbindungen der Formel (1), mit der Bedeutung der Substituenten gemäß Alternative v), welches die Schritte umfasst:

Umsetzung einer Verbindung der Formel (1) 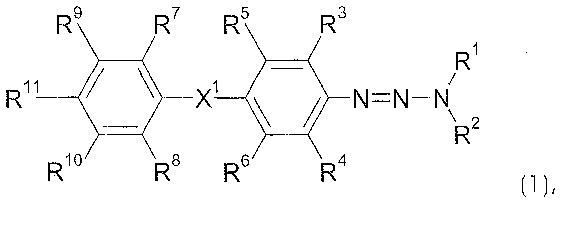 oder einem Salz, wie insbesondere einem Natrium-, Kalium oder Calciumsalz davon,
unter Glucuronidierung zu Verbindungen der Formel (1 ") 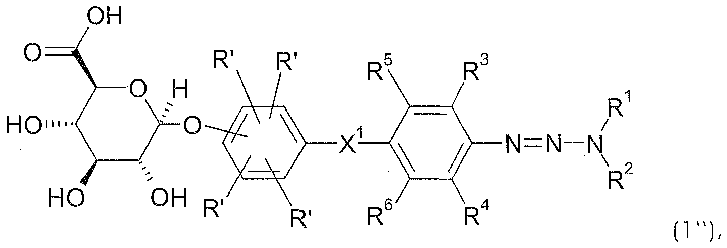 worin jeweils R' die entsprechende Bedeutung der Substituenten R⁷ bis R¹¹ und die Substituenten R¹ bis R⁶ die Bedeutung gemäß einer oder mehrerer der vorstehenden Definitionen der Alternative v) aufweist,
oder einem Salz wie insbesondere einem Natrium-, Kalium oder Calciumsalz davon, umgesetzt werden.

Insbesondere ist im Rahmen der vorliegenden Erfindung bevorzugt ein Verfahren zur Herstellung der Verbindungen der Formel (1) mit der Bedeutung der Substituenten gemäß Alternative v), welches die Schritte umfasst:

Umsetzung einer Verbindung der Formel (1') 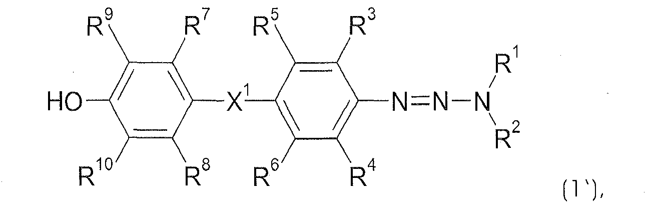 oder ein Salz, wie insbesondere einem Natrium-, Kalium oder Calciumsalz davon,
unter Glucuronidierung zu Verbindungen der Formel (1'") 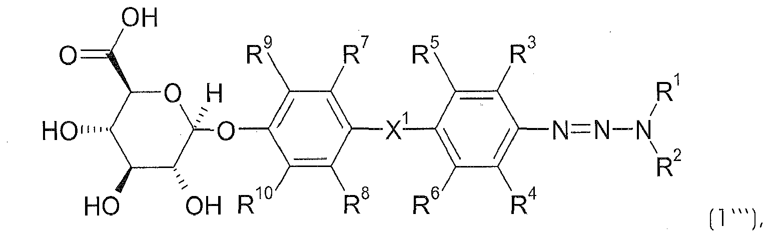 worin jeweils die Substituenten R¹ bis R¹⁰ die Bedeutung einer oder mehrerer der vorstehenden Definitionen der Alternative v) aufweisen.

Die Herstellung der Triazenverbindungen (2) kann dabei analog der in WO 2009/004060 beschriebenen Herstellweise erhalten werden, welche zum Offenbarungsgehalt der vorliegenden Anmeldung zu rechnen ist.

Folgendes Schema erläutert das Herstellverfahren am Beispiel von Beispielverbindung 1 :

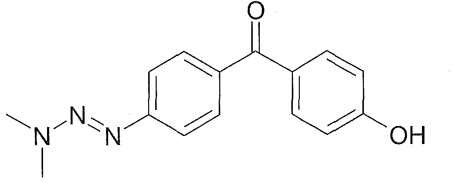

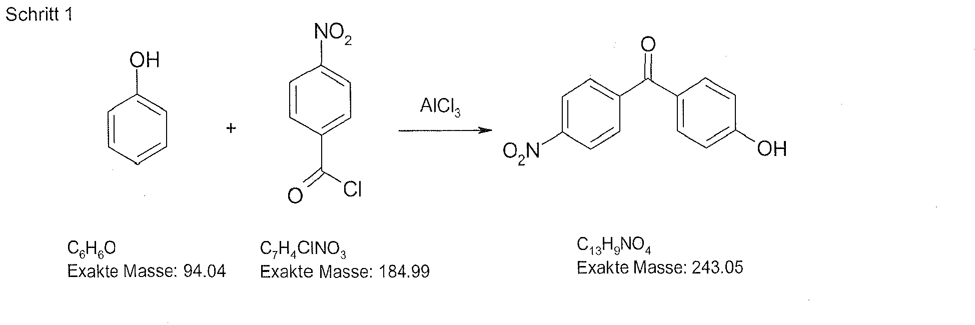

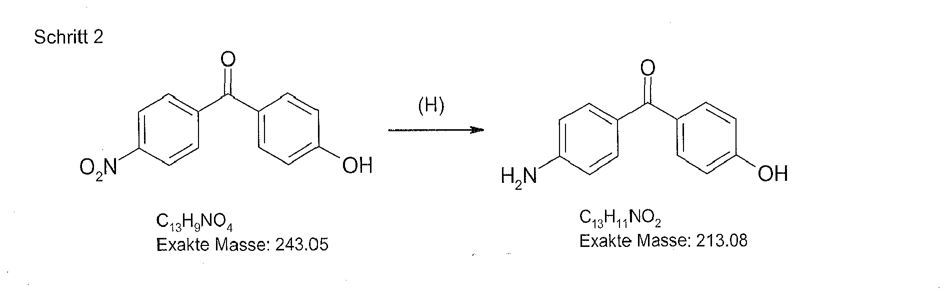

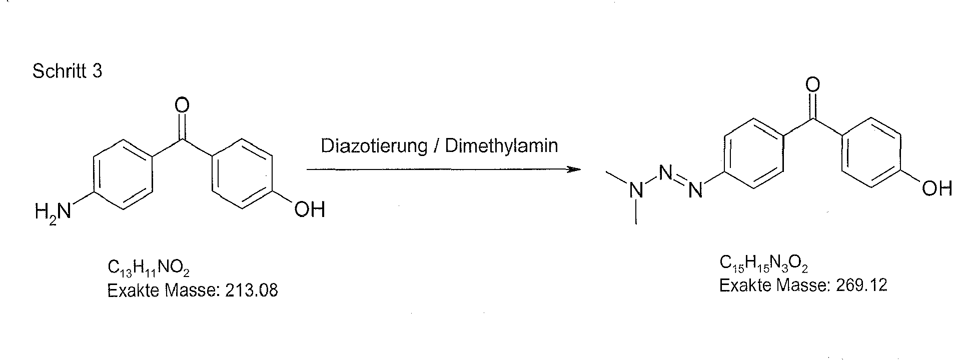

Alternativ ist auch die Umsetzung nach folgendem Schema möglich und im Rahmen der vorliegenden Erfindung bevorzugt:

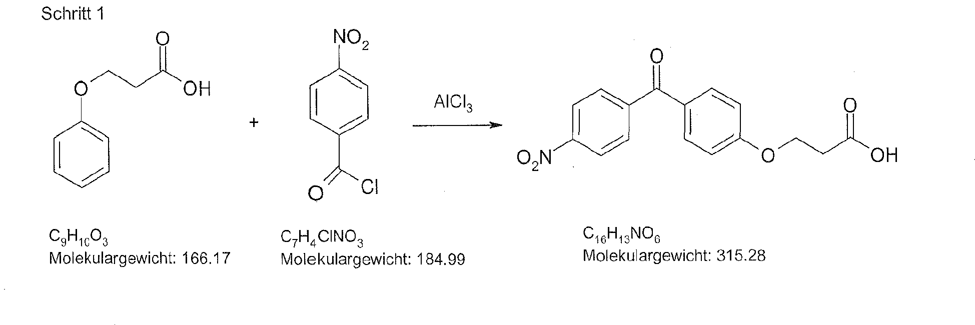

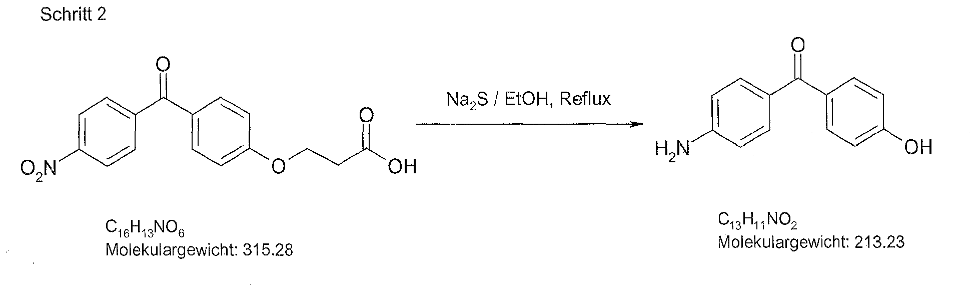

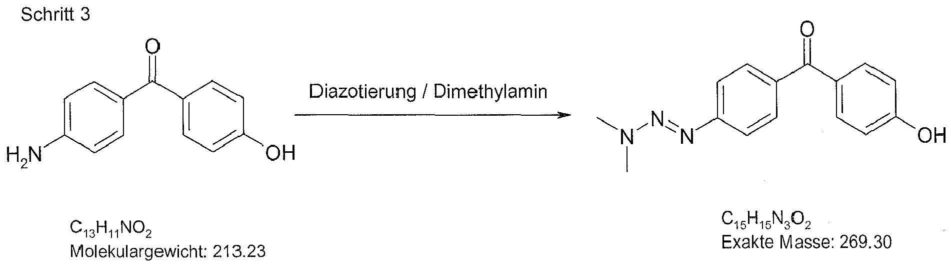

Die gezeigten Schritte stellen an sich bekannte Reaktionstypen (Friedels Crafts Acylierung, Hydrierung bzw. Reduktion der Nitrogruppe, Diazotierung, Hydrolyse bzw. Verseifung) dar, die in an sich bekannter Weise durchgeführt werden können. Durch Umsetzen mit einer pharmazeutische verträglichen Base erhält man das entsprechende Salz.
Hinsichtlich der Stilbenderivate, in denen X¹ = -CH=CH- ist, kann beispielsweise auf die WO2004/1 06358 verwiesen werden. Die Herstellung weiterer Ausgangsverbindung ist in der bereits erwähnten DE 1 7931 1 5 A1, der DE 2147781 A1 sowie in der DE 1768720 A1 beschrieben.

Hinsichtlich bevorzugter Reaktionsbedingungen sei auf die Beispiele verwiesen.

Einige der erfindungsgemäß verwendeten Zwischenprodukte sind neu und tragen über die durch sie beigesteuerten Strukturelemente zu den Eigenschaften der Endprodukte bei. Die Erfindung betrifft demnach auch neue Zwischenprodukte, wie insbesondere die in den Beispielen dargestellten.

Die vorliegende Erfindung betrifft weiterhin Verbindungen der Formel (1) zur Verwendung als Arzneimittel sowie die Verwendung der Verbindungen der Formel (1) zur Herstellung eines Arzneimittels, insbesondere zur Verwendung in der Behandlung von Krebserkrankungen.

Die erfindungsgemäßen Verbindungen können beispielsweise zur Behandlung folgender Tumorarten verwendet werden: Adenokarzinom, Aderhautmelanom, Akuter Leukämie, Akustikusneurinom, Ampullenkarzinom, Analkarzinom, Astrozytome, Basaliom, Bauchspeicheldrüsenkrebs, Bindegewebstumor, Blasenkrebs, Bronchialkarzinom, Nicht-kleinzelliges Bronchialkarzinom, Brustkrebs, Burkitt-Lymphom, Corpuskarzinom, CUP-Syndrom, Dickdarmkrebs, Dünndarmkrebs, Dünndarmtumore, Eierstockkrebs, Endometriumkarzinom, Ependymom, Epithel-Krebsarten, Ewing-Tumoren, Gastrointestinale Tumoren, Gallenblasenkrebs, Gallenkarzinome, Gebärmutterkrebs, Gebärmutterhalskrebs, Glioblastome, Gynäkologische Tumoren, Hals-, Nasen- und Ohrentumoren, Hämatologische Tumoren und Hämatologische Neoplasien (Blutkrebs), Haarzell-Leukämie, Harnröhrenkrebs, Hautkrebs, Hirntumoren (Gliome), Hirnmetastasen, Hodenkrebs, Hypophysentumor, Karzinoide, Kaposi-Sarkom, Kehlkopfkrebs, Keimzellentumor, Knochenkrebs, kolorektales Karzinom, Kopf-Hals-Tumore (Tumore des Hals- Nasen- und Ohrenbereichs), Kolonkarzinom, Kraniopharyngeome, Krebs im Mundbereich und auf der Lippe, Leberkrebs, Lebermetastasen, Leukämie, Lidtumor, Lungenkrebs, Lymphdrüsenkrebs (Hodgkin/Non-Hodgkin), Lymphome, Magenkrebs, Malignes Melanom, malignes Neoplasma, Malignome des Magen-DarmTraktes, Mammakarzinom, Mastdarmkrebs, Medulloblastome, Melanom, Meningeome, Merkelzell Karzinom, Morbus Hodgkin, Mycosis fungoides, Nasenkrebs, Neurinom, Neuroblastom, Nierenkrebs, Nierenzellkarzinome, Non-Hodgkin-Lymphome, Oligodendrogliom, Ösophaguskarzinom, osteolytische Karzinome und osteoplastische Karzinome, Osteosarkom, Ovarial-Karzinom, Pankreaskarzinom, Peniskrebs, Plasmozytom, Plattenepithelkarzinome des Kopfes und Halses, Prostatakrebs, Rachenkrebs, Rektumkarzinom, Retinoblastom, Scheidenkrebs, Schilddrüsenkarzinom, Schneeberger Krankheit, Speiseröhrenkrebs, Spinaliom, T-Zell-Lymphom (Mycosis fungoides), Thymom, Tubenkarzinom, Tumoren des Auges, Urethrakrebs, Urologische Tumoren, Urothelkarzinom, Vulvakrebs, Warzenbeteiligung, Weichteiltumoren, Weichteilsarkom, Wilms Tumor, Zervixkarzinom und Zungenkrebs, Ergänzend kann auch auf die Aufzählung der Krebsarten beispielsweise in WO2007061978 (Seite 16, Zeile 22 bis Seite 18, Zeile 2) oder in der US2007135424A1 (Seite 9, linke Spalte, Abschnitt 122) verwiesen werden, welche zum Offenbarungsgehalt der vorliegenden Anmeldung zu rechnen sind, Die Verbindungen der vorliegenden Erfindung können auch in weiteren Indikationen verwendet werden, wie den in der US2007135424A1 in den Abschnitten 123 bis 142 genannten.

Besonders bevorzugt werden die erfindungsgemäßen Verbindungen zur Behandlung von Bauchspeicheldrüsenkrebs, Leberkrebs, Prostatakrebs, Brustkrebs, Darmkrebs, Melanomen und/oder Blutkrebs (hämatologischen Neoplasien) eingesetzt.

Die Erfindung betrifft weiterhin die Verwendung der Verbindungen der Formel (1) in Kombination mit mindestens einem weiteren Chemotherapeutika zur Behandlung von Krebs sowie der jeweils damit einhergehenden Symptome.

Die Verbindungen der vorliegenden Erfindung können demnach auch in Kombination mit weiteren in der Behandlung von Krebs bzw, Tumoren bekannten Chemotherapeutika und/oder mit Arzneimitteln, die begleitend mit den Chemotherapeutika während einer Chemotherapie verabreicht werden, verwendet werden. Beispiele solcher in Kombination anwendbarer Chemotherapeutika und weiterer in der Chemotherapie eingesetzter Arzneimittel finden sich beispielsweise in der WO2007061978 unter dem Stichwort "Combination Therapy" (Seite 23, Zeile 1 bis Seite 30, Zeile 18) oder in der US2007135424A (Abschnitte 153 bis 171), auf welche auch insoweit vollinhaltlich Bezug genommen wird.

Die vorliegende Erfindung betrifft weiterhin pharmazeutische Zusammensetzungen, enthaltend mindestens eine der Verbindungen der Formel (1) zusammen mit mindestens einem pharmakologisch verträglichen Träger, Hilfsstoff oder Lösungsmittel. Dabei handelt es sich um übliche pharmazeutische Träger, Hilfsstoff oder Lösungsmittel. Genannte pharmazeutische Zusammensetzungen sind beispielsweise geeignet zur Inhalation oder zur intravenösen, intraperitonealen, intramuskulären, intravaginalen, intrabuccalen, perkutanen, subkutanen, mucokutanen, oralen, rektalen, transdermalen, topikalen, intradermalen, intragastralen oder intrakutanen Applikation und liegen beispielsweise in der Form von Pillen, Tabletten, magensaftresistente Tabletten, Filmtabletten, Schichttabletten, Retardformulierungen zur oralen, subkutanen oder kutanen Verabreichung (insbesondere als Pflaster), Depotformulierung, Dragees, Zäpfchen, Gelen, Salben, Sirup, Inhalationspulvern, Granulaten, Suppositorien, Emulsionen, Dispersionen, Mikrokapseln, Mikroformulierungen, Nanoformulierungen, liposomalen Formulierungen, Kapseln, magensaftresistente Kapseln, Pulver, Inhalationspulvern, mikrokristallinen Formulierungen, Inhalationssprays, Puder, Tropfen, Nasentropfen, Nasensprays, Aerosolen, Ampullen, Lösungen, Säfte, Suspensionen, Emulsionen, Infusionslösungen oder Injektionslösungen etc. vor.

Die erfindungsgemäßen Verbindungen können in pharmazeutischen Zusammensetzung verabreicht werden, die verschiedene organische oder anorganische Träger- und/oder Hilfsmaterialien enthalten können, wie sie üblicherweise für pharmazeutische Zwecke insbesondere für feste Arzneimittelformulierungen verwendet werden, wie beispielsweise Exzipienten (wie Saccharose, Stärke, Mannit, Sorbit, Lactose, Glucose, Cellulose, Talk, Calciumphosphat, Calciumcarbonat), Bindemittel (wie Cellulose, Methylcellulose, Hydroxypropylcellulose, Polypropylpyrrolidon, Gelatine, Gummiarabicum, Polyethylenglykol, Saccharose, Stärke),

Desintegrationsmittel (wie Stärke, hydrolysierte Stärke, Carboxymethylcellulose, Calciumsalz von Carboxymethylcellulose, Hydroxypropylstärke, Natriumglycolstärke, Natriumbicarbonat, Calciumphosphat, Calciumcitrat), Gleit- bzw. Schmiermittel (wie Magnesiumstearat, Talk, Natriumlaurylsulfat), ein Geschmacksbildner (wie Citronensäure, Menthol, Glycin, Orangenpulver), Konservierungsmtitel (wie Natriumbenzoat, Natriumbisulfit, Methylparaben, Propylparaben), Stabilisatoren (wie Citronensäure, Natriumcitrat, Essigsäure, und Multicarbonsäuren aus der Titriplex Reihe wie z.B. Diethylentriaminpentaessigsäure (DTPA), Suspendiermittel (wie Methylcellulose, Polyvinylpyrrolidon, Aluminiumstearat), Dispergiermittel, Verdünnungsmittel (wie Wasser, organische Lösungsmittel), Bienenwachs, Kakaobutter, Polyethylenglykol, weißes Petrolatum etc..

Flüssige Arzneimittelformulierungen, wie Lösungen, Suspensionen und Gele enthalten üblicherweise einen flüssigen Träger, wie Wasser und/oder pharmazeutisch verträgliche organische Lösungsmittel. Weiterhin können derartige flüssigen Formulierungen auch pH-einstellende Mittel, Emulgatoren oder dispergierende Agenzien, puffernde Agenzien, Konservierungsmittel, Netzmittel, Geliermittel (beispielsweise Methylcellulose), Färbemittel und/oder Aromastoffe enthalten. Die Zusammensetzungen können isotonisch sein, das heißt diese können den gleichen osmotischen Druck wie Blut haben. Die Isotonie der Zusammensetzung kann durch die Verwendung von Natriumchlorid oder anderer pharmazeutisch annehmbare Agenzien wie beispielsweise Dextrose, Maltose, Borsäure, Natriumtartrat, Propylenglykol oder andere anorganische oder organisch lösliche Substanzen eingestellt werden. Die Viskosität der flüssigen Zusammensetzungen kann unter Verwendung eines pharmazeutisch annehmbaren Verdickungsmittels, wie Methylcellulose eingestellt werden. Andere geeignete Verdickungsmittel umfassen beispielsweise Xanthan, Carboxymethylcellulose, Hydroxypropylcellulose, Carbomer und dergleichen. Die bevorzugte Konzentration des Verdickungsmittels wird von dem ausgewählten Agens abhängen, Pharmazeutisch annehmbare Konservierungsmittel können verwendet werden, um die Haltbarkeit der flüssigen Zusammensetzung zu erhöhen. Benzylalkohol kann geeignet sein, obwohl eine Vielzahl von Konservierungsmitteln einschließlich beispielsweise Paraben, Thimerosal, Chlorbutanol oder Benzalkoniumchlorid ebenfalls verwendet werden können.

Als Stabilisierungsmittel für die pharmazeutischen festen oder flüssigen Formulierungen der erfindungsgemäßen Verbindungen, wie insbesondere der Verbindung von Beispiel 2a), erweist sich insbesondere die Diethylentriaminpentaessigsäure (DTPA).

Der Wirkstoff kann beispielsweise mit einer Einheitsdosis von 0,01 mg/kg bis 500 mg/kg Körpergewicht beispielweise bis zu 1 bis 4 mal am Tag verabreicht werden. Die Dosierung kann jedoch je nach Alter, Gewicht, Zustand des Patienten, Schwere der Erkrankung oder Art der Verabreichung erhöht oder herabgesetzt werden.

Die Erfindung wird durch die folgenden Beispiele näher veranschaulicht. Die Beispiele stellen lediglich Exemplifizierungen dar, und der Fachmann ist in der Lage die spezifischen Beispiele auf weitere beanspruchte Verbindungen auszudehnen.

### BEISPIELE:

### AUSGANGSPRÄPARATE:

Die Herstellung der Ausgangspräparate 1 bis 5 kann analog der in WO 2009/004060 beschriebenen Herstellweise erfolgen.

### Präparat 1:

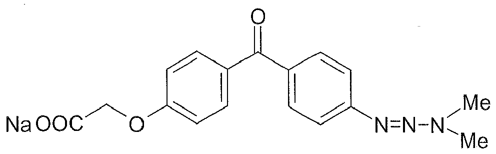

(entsprechend Präparat 1 der Beispiele der WO 2009/004060)

### Präparat 2:

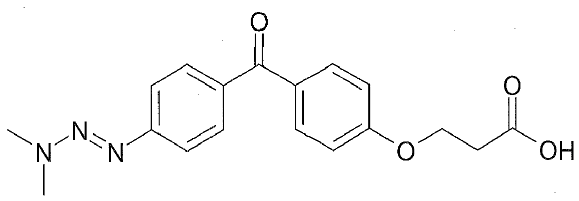

(entsprechend Präparat 2 der Beispiele der WO 2009/004060)

### Präparat 3:

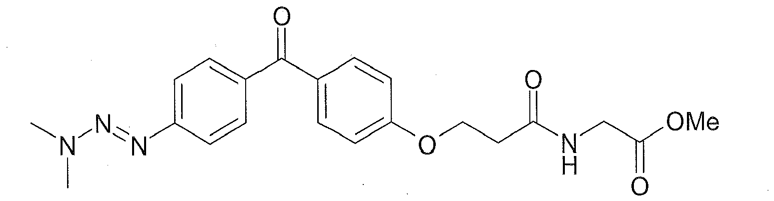

(entsprechend Beispiel 1 der Beispiele der WO 2009/004060)

### Präparat 4:

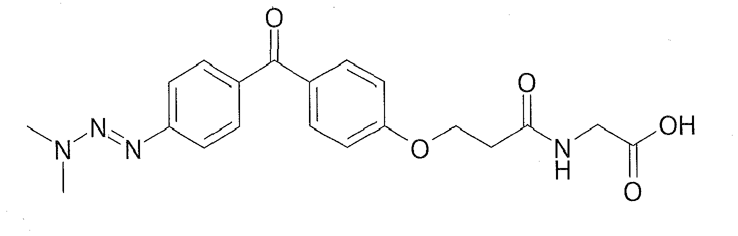

(entsprechend Beispiel 2 der Beispiele der WO 2009/004060)

### Präparat 5:

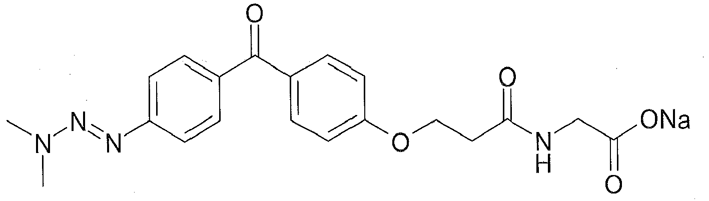

(entsprechend Beispiel 2a) der Beispiele der WO 2009/004060)

### Herstellung der Beispielverbindungen:

### Beispiel 1 :

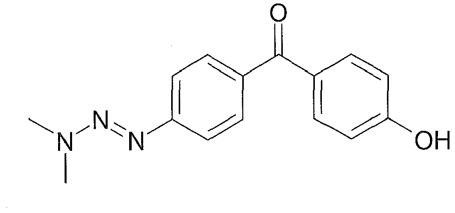

Zu 100g (0,237 mol) der als Präparat 5 bezeichneten Ausgangsverbindung wurde Methanol (1,0 L, 10 Vol.) gegeben. Über einen Zeitraum von 30 Minuten wurde bei 25°C tropfenweise eine Lösung von Natriumhydroxid (28,0 g, 0,7 mol) in Wasser (40 mL) zugegeben. Die Reaktionsmasse wurde zum Rückfluss für eine Stunde bei 65 ° C erhitzt, Der Fortschritt der Reaktion wurde durch TLC (Thin-Layer-Cromatography; Methanol und Dichloromethane im Verhältnis 1 : 9) kontrolliert, Die Reaktionsmasse wurde unter Vakuum bei 50°C vollständig konzentriert um das Lösungsmittel zu entfernen. Die konzentrierte Masse wurde sodann in Wasser (1,0 l, 10 vol) aufgelöst, Der pH-Wert der Lösung wurde auf 3-3,5 eingestellt mit 1,5 N HCl, Der ausgefallene Feststoff wurde in Ethylacetat (2X500mL) extrahiert. Das Ethylacetat-Extrakt wurde mit Wasser (3x500mL), gefolgt von 20% Kochsalzlösung (500ml) gewaschen. Die organische Schicht wurde über Natriumsulfat (50g) getrocknet und konzentriert. Im Anschluss wurde die Verbindung im Rotavapour unter 50°C getrocknet.

Ausbeute: 55,0 g (85,85 % der Theorie)
Reinheit HPLC: 99,7 %

Das Produkt ist ein gelb-hell-bräunliches kristallines Pulver. Es ist in Methanol löslich. Der Schmelzpunkt liegt bei 157-160°C.

Abbildung 1 zeigt das ¹³C-NMR-Spektrum (100 MHz - DMSO) für die nach diesem Syntheseschema erhaltene Beispielverbindung 1 ,

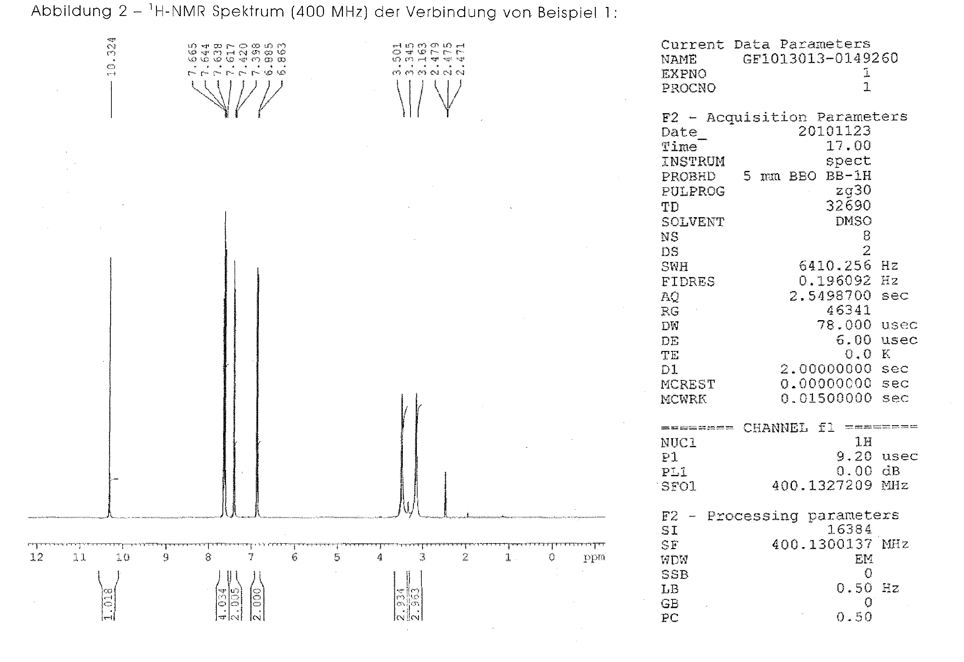

Abbildung 2 zeigt das ¹H-NMR-Spektrum (400 MHz - DMSO) für die nach diesem Syntheseschema erhaltene Beispielverbindung 1.

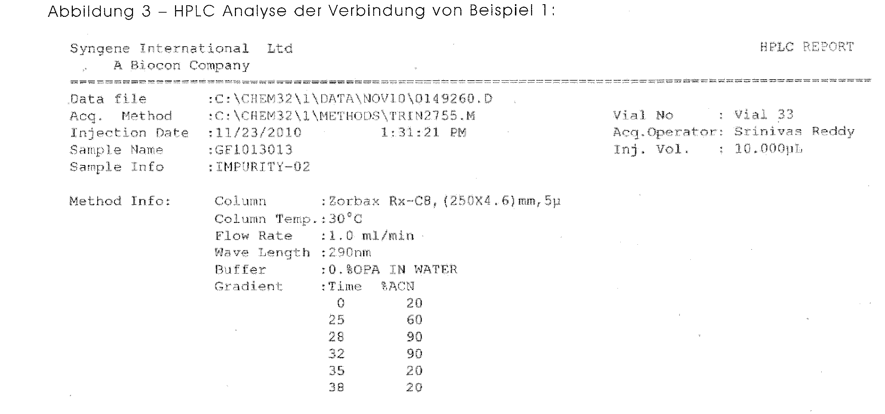

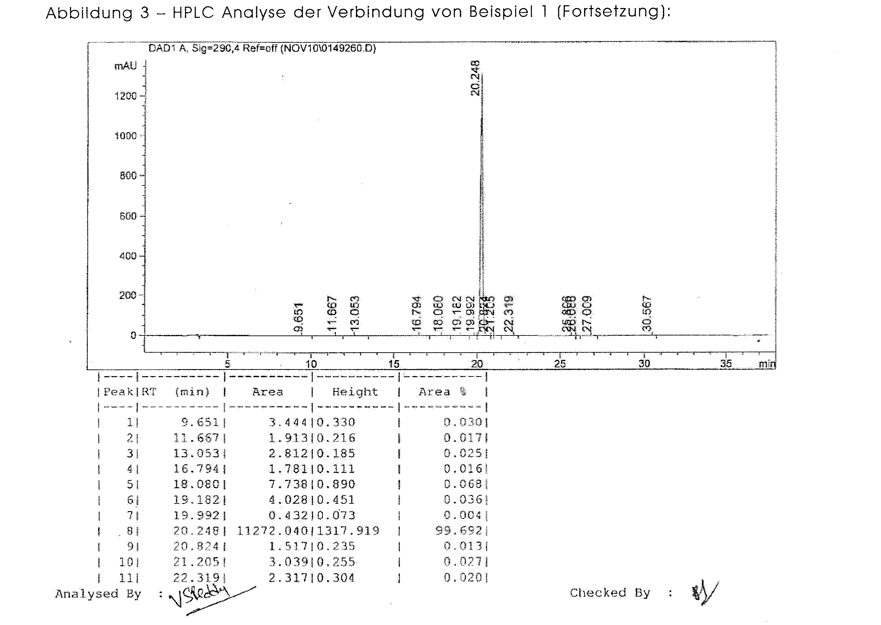

Abbildung 3 zeigt die Ergebnisse des HPLC Spektrum für die nach diesem Syntheseschema erhaltene Beispielverbindung 1.

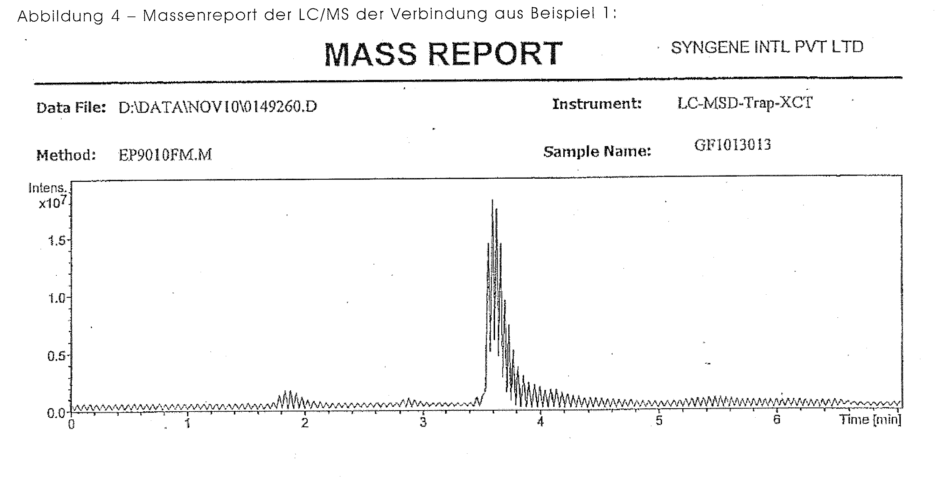

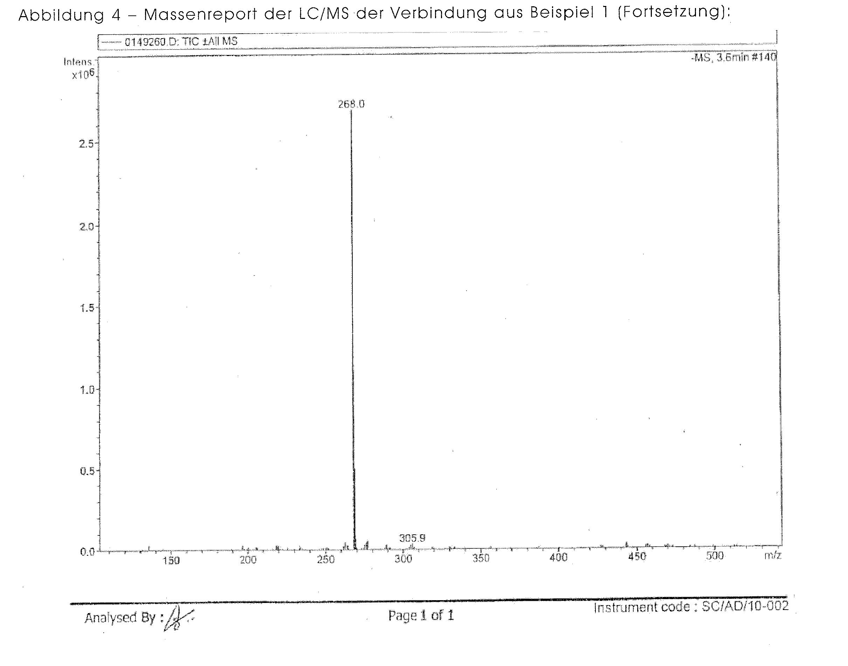

Abbildung 4 zeigt die Ergebnisse aus der LC/MS Analyse für die nach diesem Syntheseschema erhaltene Beispielverbindung 1.

Weiterhin ist es auch möglich, und bevorzugt, Beispielverbindung 1 wie folgt herzustellen:

### Herstellung von Zwischenprodukt 1 :

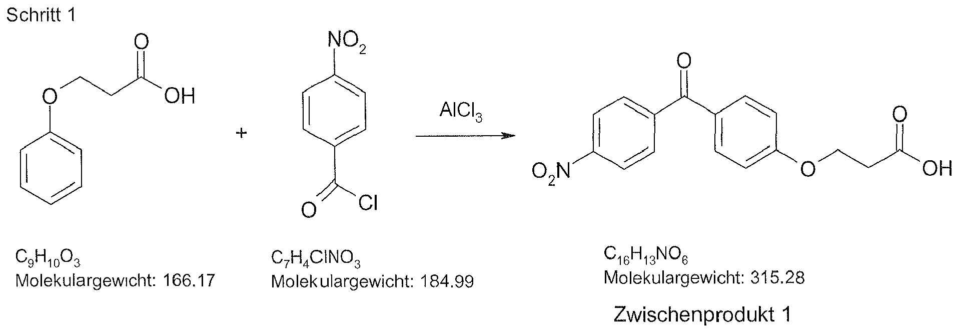

### Schritt 1 Experimentelle Durchführung:

In einen trockenen 1,0l Reaktionskolben, der mit Ruhrerkondensator und Thermotasche ausgestattet ist, wurde 1, 2-Dichlorethan (780,0 ml, 44,0V) eingefüllt und auf 0 - 5°C gekühlt, Wasserfreies Aluminiumchlorid (39,34 g, 0,295 mol) wurde eingefüllt und die Suspension 30 Minuten bei 0-5 ° C gerührt. P-Nitrobenzoylchlorid (19,765 g, 0,106 Mol), gelöst in 1, 2-Dichlorethan (100 mL, 5,0 V), wurde bei 0 - 5°C über 1 Stunde zugegeben Die Reaktionsmischung wurde 1 Stunde bei 0 - 5°C gerührt.
Phenoxypropionsdure (1 7,7 g, 0,106 mol) wurde portionsweise über 1 Stunde zugegeben. Nach vollstandiger Zugabe wurde das Reaktionsgemisch bei 0 - 5°C für 4 h gehalten und für weitere 1 2 Stunden unter allmählicher Erhöhung der Reaktionstemperatur auf 22-26 ° C gerührt. Der Reaktionsfortschritt wurde durch TLC (Thin-Layer-Chromatogryphy; TLC-System: Essigester : Hexan 7:3) überwacht.
Die Reaktionsmischung wurde in Eiswasser (1000ml, 57.0V), das mit konzentrierter Salzsäure (19,8 ml, 1,0 V) und Toluol (497 mL, 28.0V) versetzt war, abgeschreckt. Die resultierende Aufschlämmung wurde bei 25°C für 1 Stunde geruhrt, Das Produkt wurde in Toluol (25.0mL) gewaschen und filtriert und unter Vakuum bei 50°C für 1 2 Stunden getrocknet.

| | |
|---|---|
| Ausbeute | 20.0 g (Theorie: 59.55 %) |
| Reinheit HPLC | 95.28% |
| ¹H NMR | Übereinstimmung |
| LC-MS | Übereinstimmung (Anlage 7) |

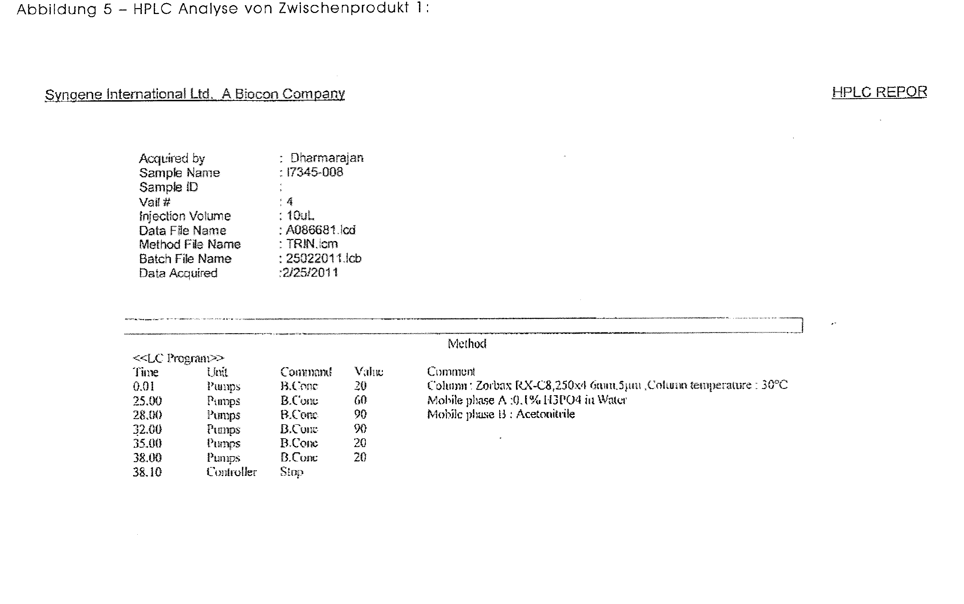

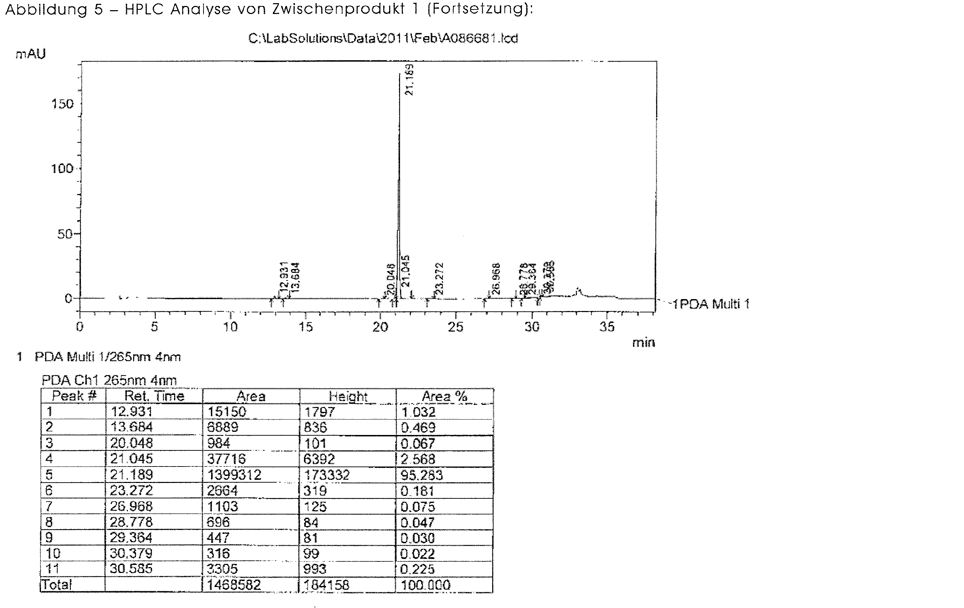

Abbildung 5 zeigt die Ergebnisse der HPLC für das nach diesem Syntheseschema erhaltene Zwischenprodukt 1.

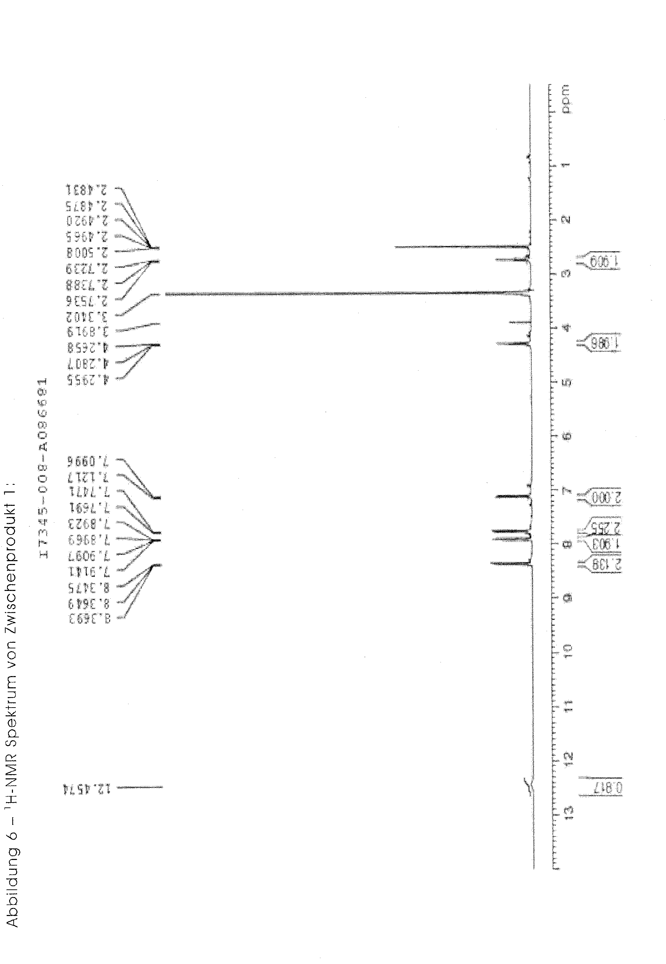

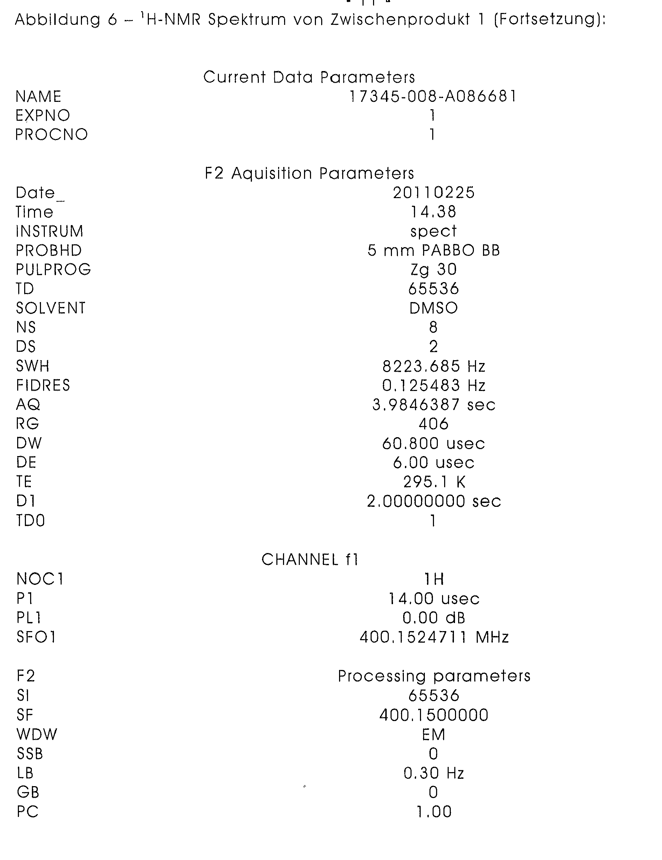

Abbildung 6 zeigt das ¹H-NMR-Spektrum für das nach diesem Syntheseschema erhaltene Zwischenprodukt 1 .

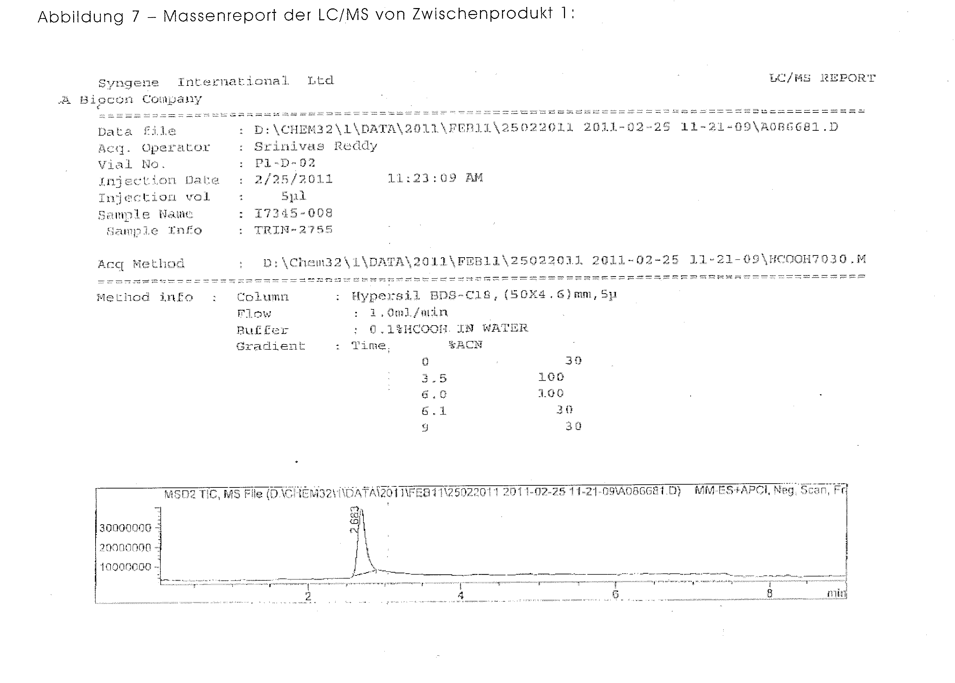

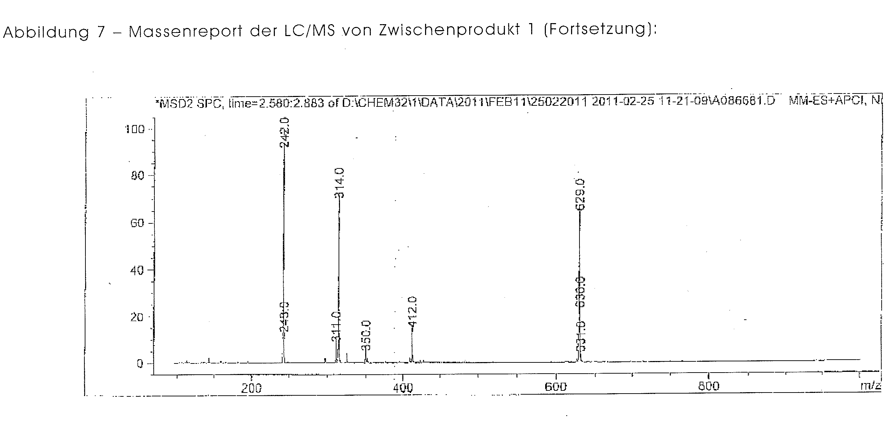

Abbildung 7 zeigt die Ergebnisse der der LC/MS Analyse Spektrum für das nach diesem Syntheseschema erhaltene Zwischenprodukt 1 .

### Herstellung von Zwischenprodukt 2:

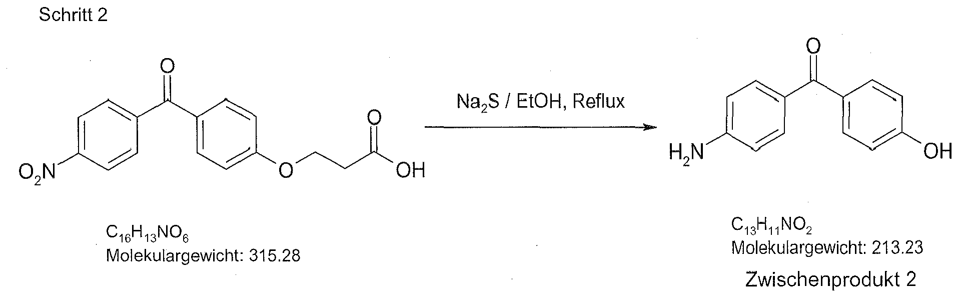

### Schritt 2 Experimentelle Durchführung:

In einen 250 ml-Reaktionskolben, der mit Rührer und Kühler ausgestattet ist, wurden 10g (0,031 mol) des Zwischenprodukts 1 und Ethanol (100 mL) zusammengegeben. Das Gemisch wurde auf 15-20°C abgekühlt; sodann wurde über einen Zeitraum von 2 Stunden eine Lösung von Natriumsulfid (11,11 g, 0,142 Mol in 27,2 ml Wasser) zugegeben. Die Reaktionsmischung wurde bei 75°C für 16 Stunden gerührt. Der Reaktionsfortschritt wurde mittels TLC kontrolliert. (TLC-System: Essigester: Hexan 7:3). Das Reaktionsgemisch wurde durch Kieselgur (HyFlo®) filtriert, Mittels Rotationsverdampfer wurde bei 50°C der Großteil der Lösungsmittel entfernt. Das Konzentrat wurde in Wasser gelöst (100 mL) und der pH-Wert mittels Essigsäure (33,0 ml) auf pH 3 eingestellt. Das Produkt wurde in Ethylacetat (2x100 ml) extrahiert. Die vereinigten Extrakte wurden zweimal mit Wasser (2 x 50 mL) und einmal mit 20%iger Salzlösung (1 x 50 ml) gewaschen. Die organische Schicht wurde über Natriumsulfat getrocknet (10 g) und unter Vakuum bei 50°C destilliert, um den Großteil der Lösungsmittel zu entfernen. Das Produkt wurde in n-Hexan aufgeschlämmt, filtriert und unter Vakuum bei 60 ° C für ungefähr 1 2 Stunden getrocknet.

| | |
|---|---|
| Ausbeute | 5.0 g (74% Theorie) |
| Reinheit HPLC | 85.64 % |
| ¹H NMR | Entspricht der Molekularstruktur |
| LC-MS | Entspricht molekularem Gewicht |

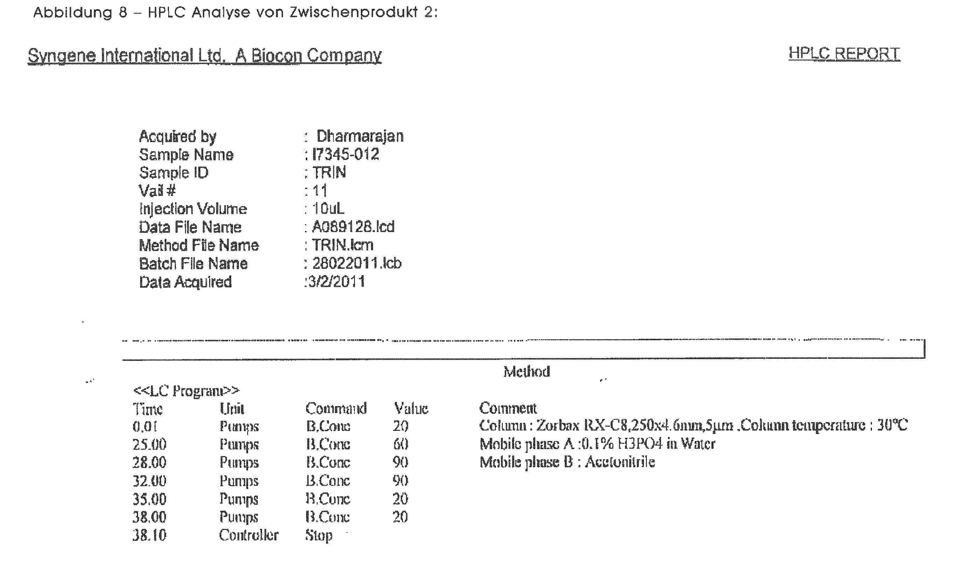

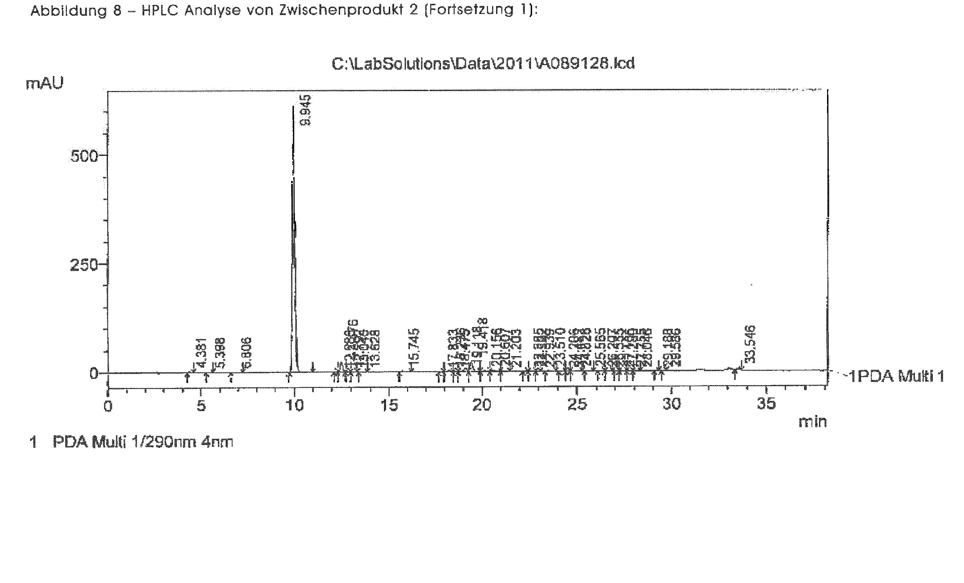

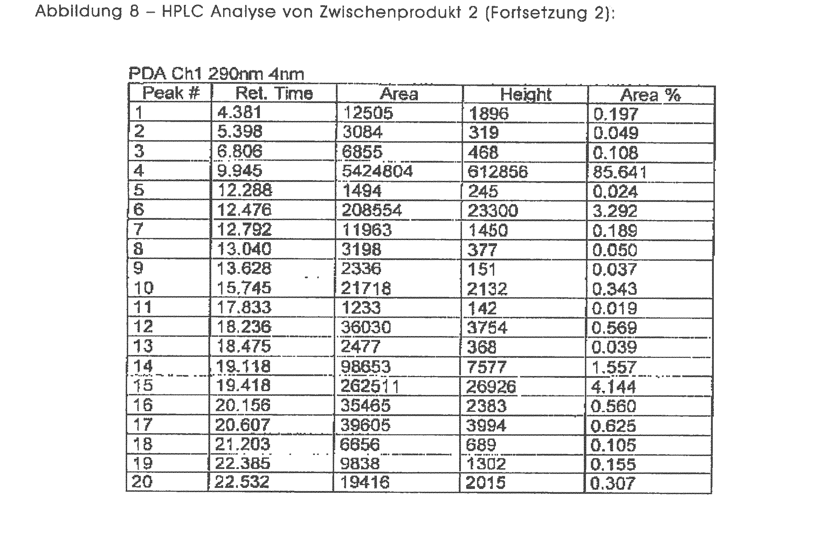

Abbildung 8 zeigt die Ergebnisse der HPLC für das nach diesem Syntheseschema erhaltene Zwischenprodukt 2.

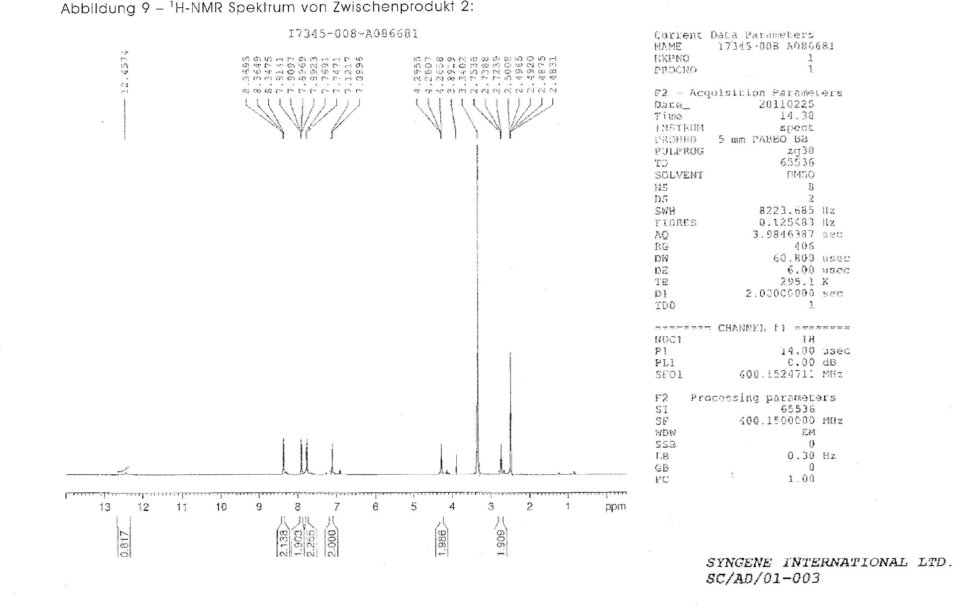

Abbildung 9 zeigt das ¹H-NMR-Spektrum für für das nach diesem Syntheseschema erhaltene Zwischenprodukt 2.

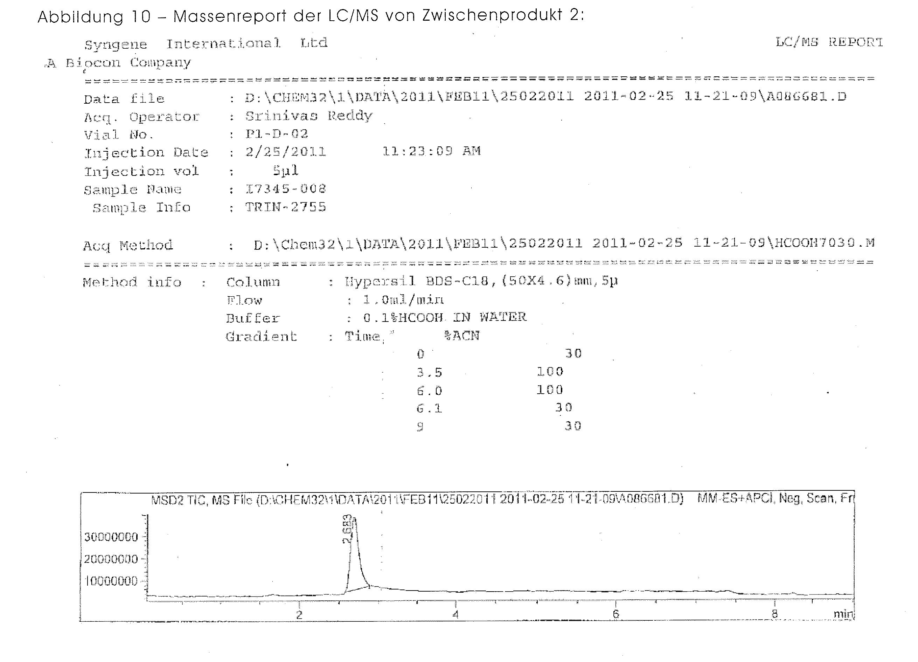

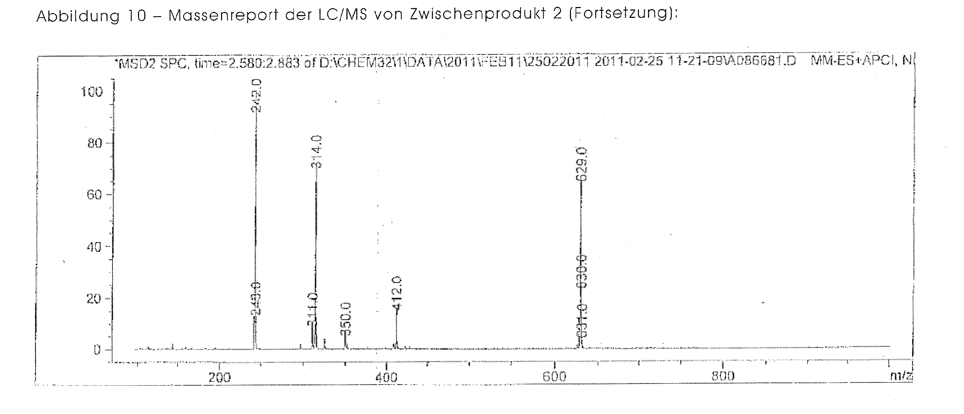

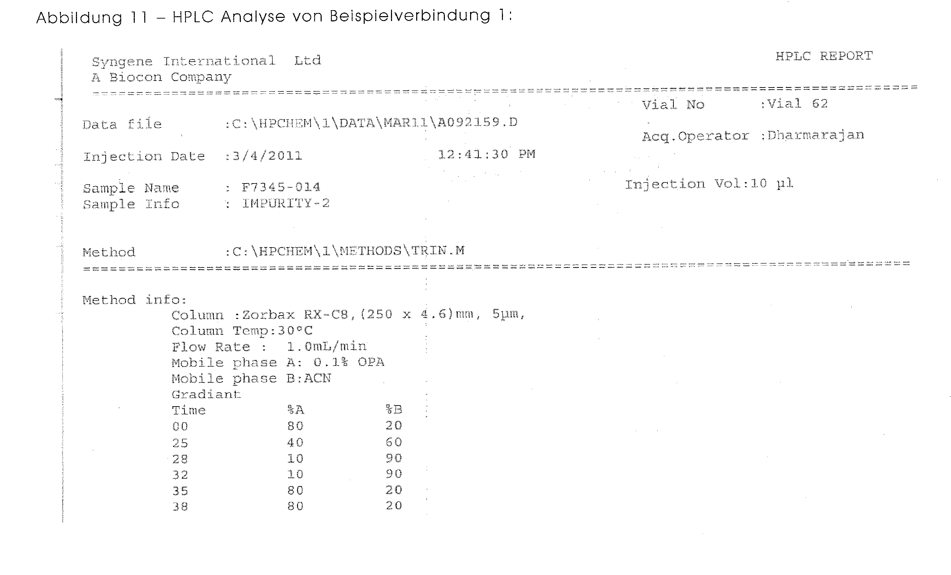

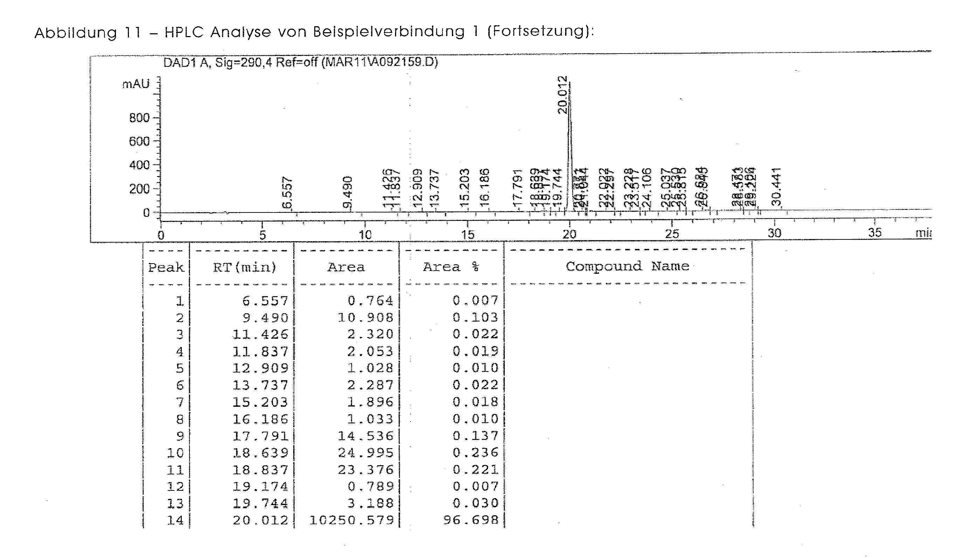

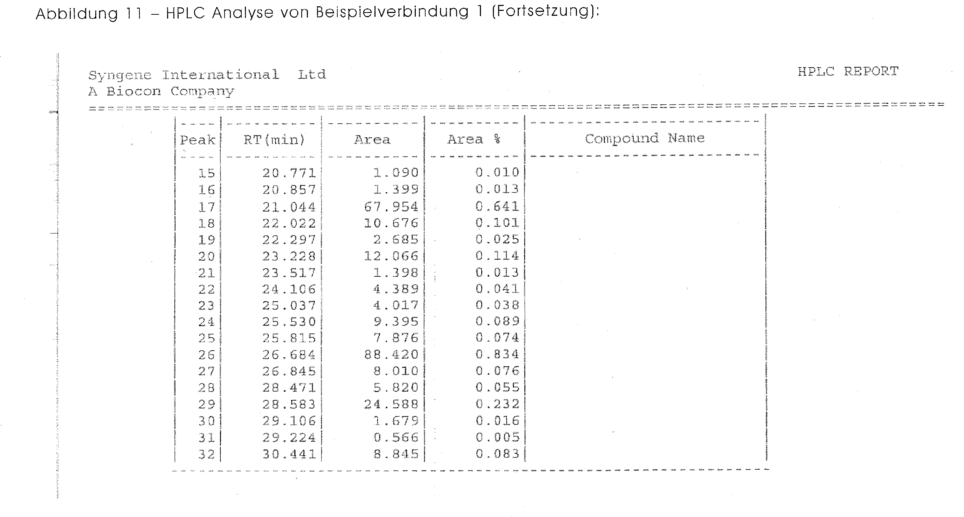

Abbildung 10 zeigt die Ergebnisse der der LC/MS Analyse Spektrum für das nach diesem Syntheseschema erhaltene Zwischenprodukt 2.

### Herstellung von Beispielverbindung 1 :

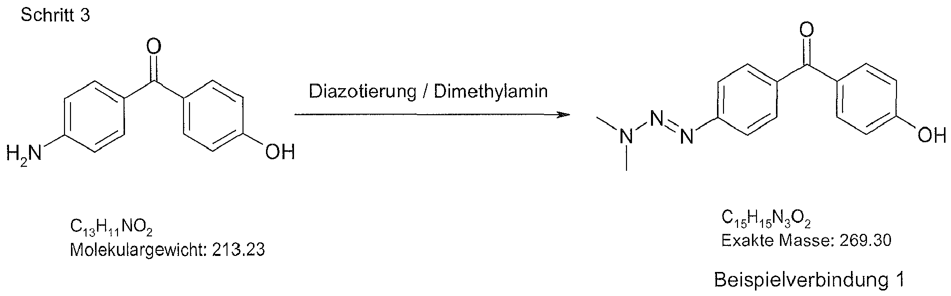

### Schritt 3 Experimentelle Durchführung:

Ein 250-ml-Reaktionsgefäß, ausgestattet mit Thermo-Tasche, Überkopf-Rührer und Kühler wurde in ein Eiswasserbad gestellt. In diesen Kolben wurde 5g (0,023mol) Zwischenprodukt 2 und Wasser (75 ml) zusammengegeben. Die Mischung wurde bei Raumtemperatur (25°C) gerührt und konzentrierte Salzsäure (1,76 g, 0,059 mol) zugegeben. Die Suspension wurde auf 0-5°C gekühlt und eine Lösung von Natriumnitrit (1,76 g, 0,025 mol) in Wasser (8,8 ml) tropfenweise über einen Zeitraum von 1 Stunde zugefügt. Die resultierende Reaktionsmischung wurde bei gleicher Temperatur für eine weitere Stunde geruhrt. In ein zweites Reaktionsgefäß wurde 40% Dimethylamin (5,0 g, 0,044 mol) hineingegeben und auf 0-5°C abgekühlt. Zu der gerührten Lösung von Dimethylamin wurde uber 30 Minuten eine Diazoniumsalz-Lösung bei 0-5°C zugegeben und die Reaktionsmischung für weitere 4 Stunden gerührt. Der Fortschritt der Reaktion wurde durch TLC kontrolliert. (TLC-System: Essigester: Hexan 7:3).
Die Reaktionsmischung wurde mit 1,5 molarer Salzsaurelösung (25 mL) auf pH3 angesäuert, Das Produkt wurde durch 2 maliges Waschen mit Ethylacetat (2x50 ml) extrahiert. Die vereinigten Extrakte wurden mit Wasser gewaschen (2x 25 ml). Die organische Phase wurde über Natriumsulfat (10 g) getrocknet und unter Vakuum destilliert, um den Großteil der Losungsmittel zu entfernen. Die konzentrierte Phase wurde mit Methyl-t-Butyl-Ether (25.0mL) aufgeschlämmt und bei 20-25 ° C für 3 Stunden gerührt. Das Produkt wurde mit Methyl-t-Butyl-Ether (25.0mL) abfiltriert. Das Produkt wurde für 12 Stunden unter Vakuum bei 50°C getrocknet.

| | |
|---|---|
| Ausbeute | 3 g (47.54 % in Theorie) |
| Reinheit HPLC | 96.7 % |
| ¹H NMR | Entspricht der Molekularstruktur |
| LC-MS | Entspricht Molekulargewicht |
| ¹³C NMR | Entspricht Molekularstruktur |

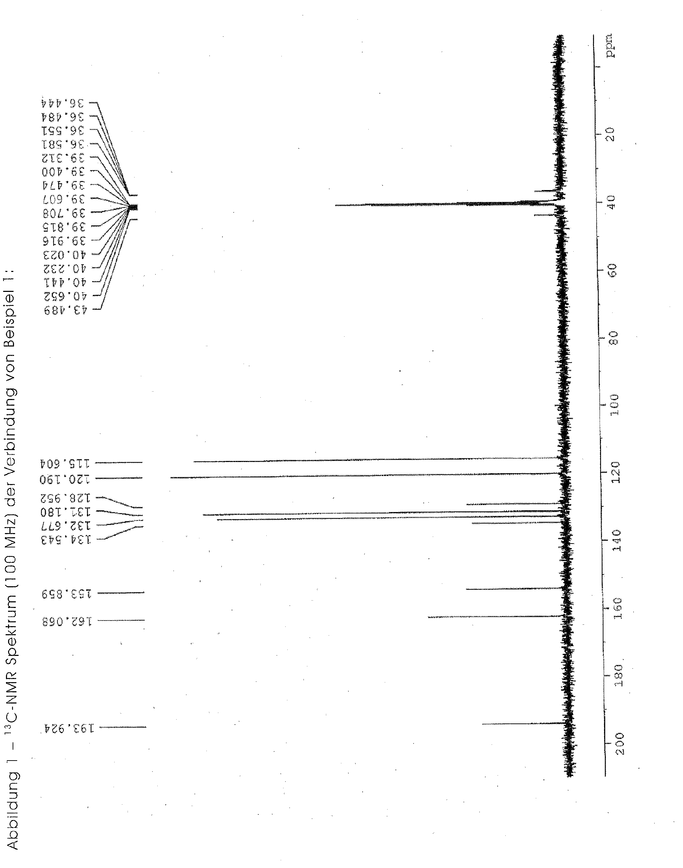

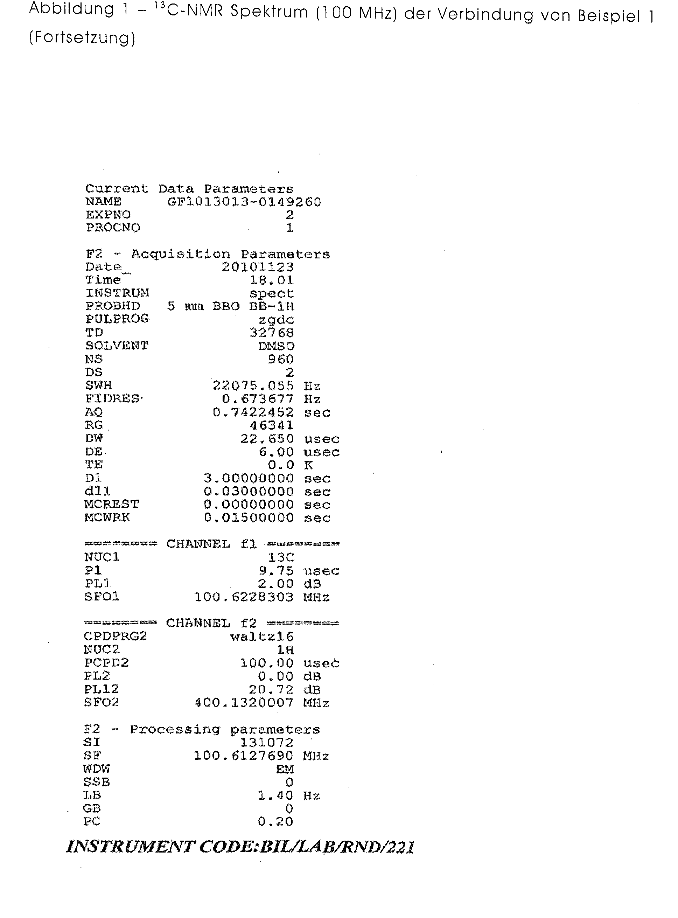

Abbildung 1 1 zeigt die Ergebnisse des HPLC Spektrum für die nach diesem Syntheseschema erhaltene Beispielverbindung 1 .

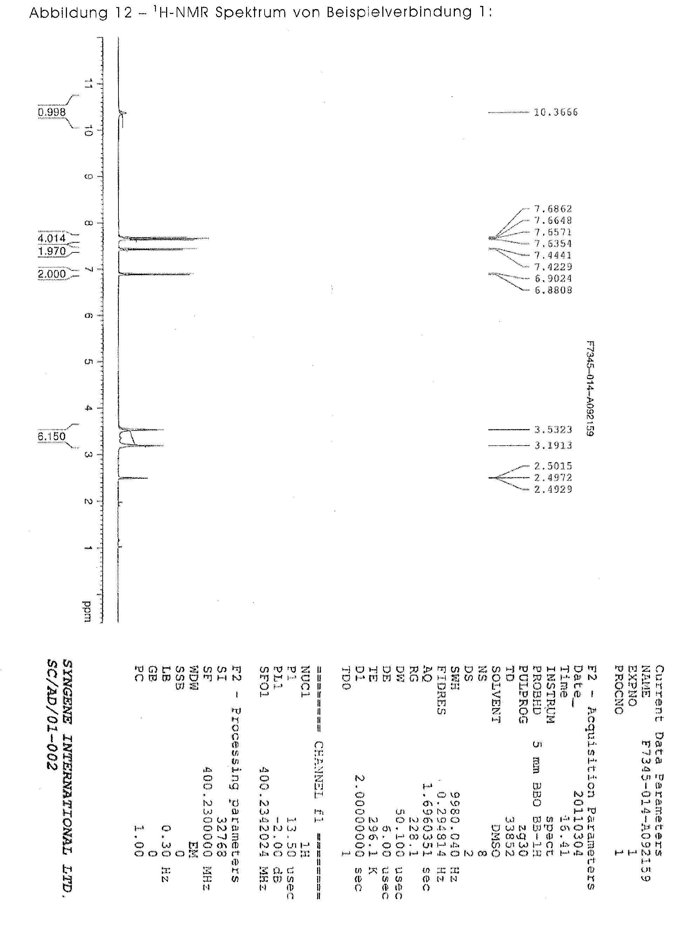

Abbildung 12 zeigt das ¹H-NMR-Spektrum fur die nach diesem Syntheseschema erhaltene Beispielverbindung 1 .

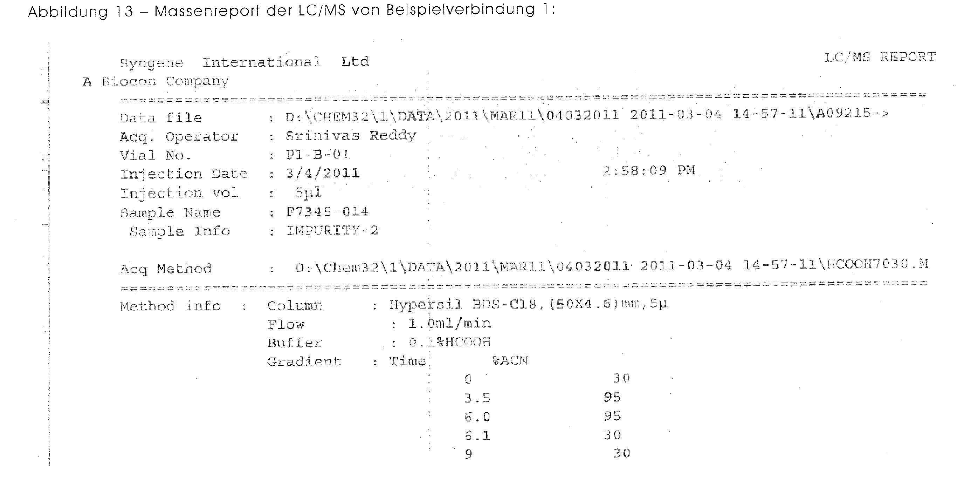

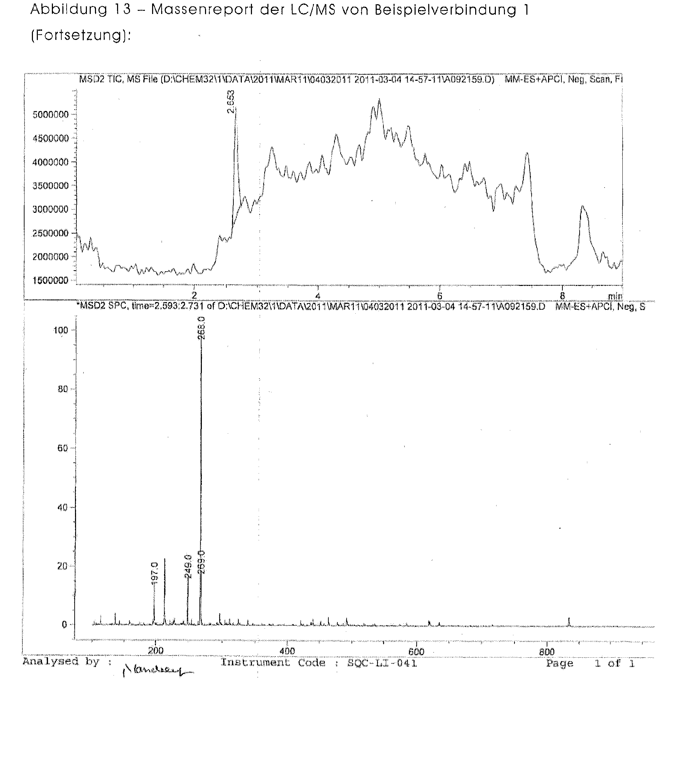

Abbildung 13 zeigt die Ergebnisse aus der LC/MS Analyse für die nach diesem Syntheseschema erhaltene Beispielverbindung 1.

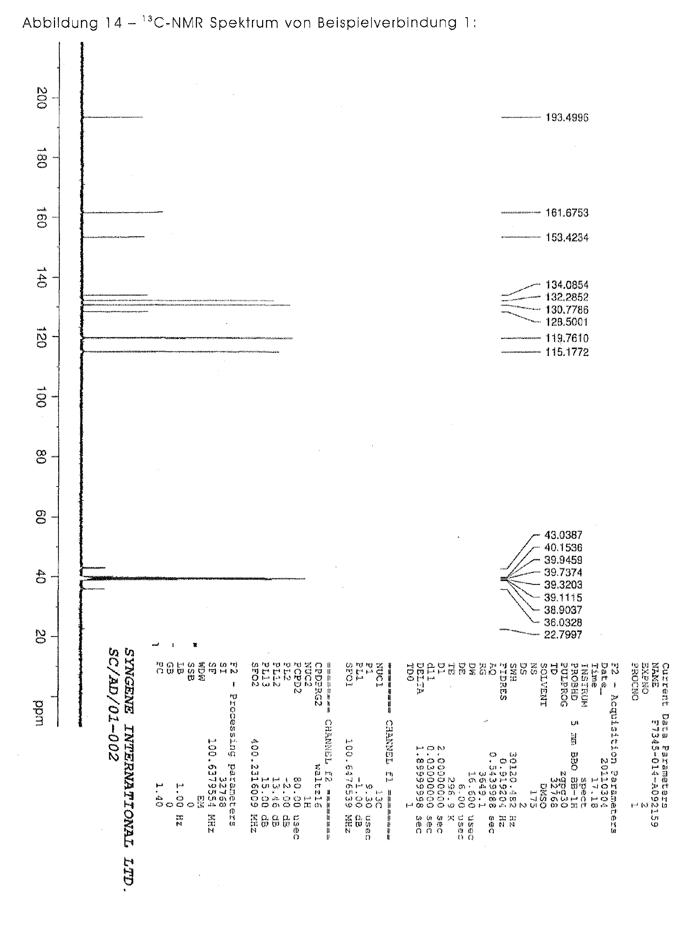

Abbildung 14 zeigt das ¹³C-NMR-Spektrum für die nach diesem Syntheseschema erhaltene Beispielverbindung 1.

### PHARMAKOLOGISCHE WIRKVERSUCHE:

### Daten zur Pharmakologie in-vitro

### Experiment 1: Intrazelluläre Metabolisierung verschiedener Triazene in HepG-2 und MCF-7 Zellkulturen

Es wird gezeigt, dass Verbindungen nach Beispiel 1 schneller und in größerer Menge intrazellulär metabolisiert werden als die als Vergleichssubstanzen eingesetzten Verbindungen nach Präparat 2 und 5 (entsprechend Verbindungen nach Präparat 2 und Beispiel 2a jeweils der WO 2009/004060).

### Experimenteller Aufbau:

Die getesteten Verbindungen wurden wie folgt gelöst:
Erfindungsgemäße Verbindung:
Beispiel 1 : in 20 mg/ml DMSO

Vergleichssubstanzen:
Präparat 2: in 50mg/ml DMSO,
Präparat 5: in 50mg/ml Wasser.

Alle drei Substanzen wurden äquimolar in einer Dosis von 100nmol/ml auf 1 x 10⁷ HepG2 bzw. MCF-7-Zellen gegeben. Als Zellkulturmedium wurde 100% fötales Kälberserum (FCS) verwendet. Für den Versuch wurde der Gehalt aus Ausgangsprodukt und Metaboliten im Zellüberstand nach 1, 3 und 6 (HepG2) bzw. 1,3 und 20 Stunden (MCF-7) Inkubationszeit ermittelt. Alle drei Substanzen zeigten in Kontrollversuchen mit 100% FCS nach 40 Stunden metabolische Stabilität.

### Ergebnis:

Binnen 6 Stunden wurden 28% der der Ausgangssubstanz nach Beispiel 1 metabolisiert, während die beiden Vergleichssubstanzen zu weniger als 1 % metabolisiert wurden.

In MCF-7 Zellen wird die Verbindung nach Beispiel 1 innerhalb von 1 Stunde zu mehr als der Hälfte, und bereits nach 3 Stunden vollständig metabolisiert. Die Vergleichssubstanzen nach WO2009/004060 zeigen hier ein unterschiedliches Bild. Während Präparat 2 keine Metabolisierung zeigt, wird Präparat 5 zu knapp 30% binnen 20 Stunden metabolisiert.

Die Verbindung nach Beispiel 1 wird damit deutlich schneller und in höherer Konzentration von den untersuchten HepG2- und MCF-7 Zellen aufgenommen als die entsprechenden Vergleichssubstanzen nach Präparat 2 und Beispiel 2a der WO2009/004060.

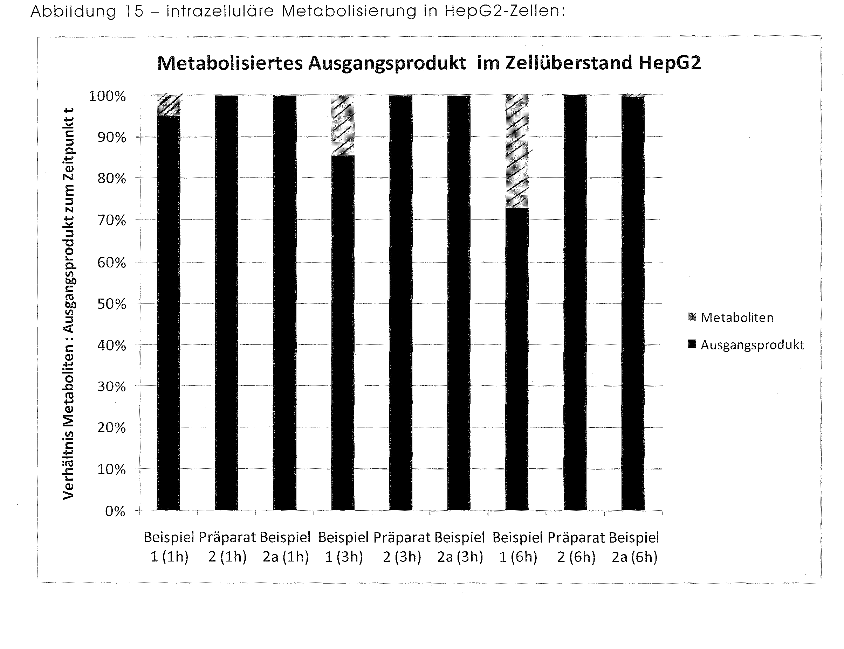

Abbildung 15 zeigt die intrazelluläre Metabolisierung verschiedener Triazene in HepG2-Zellen.

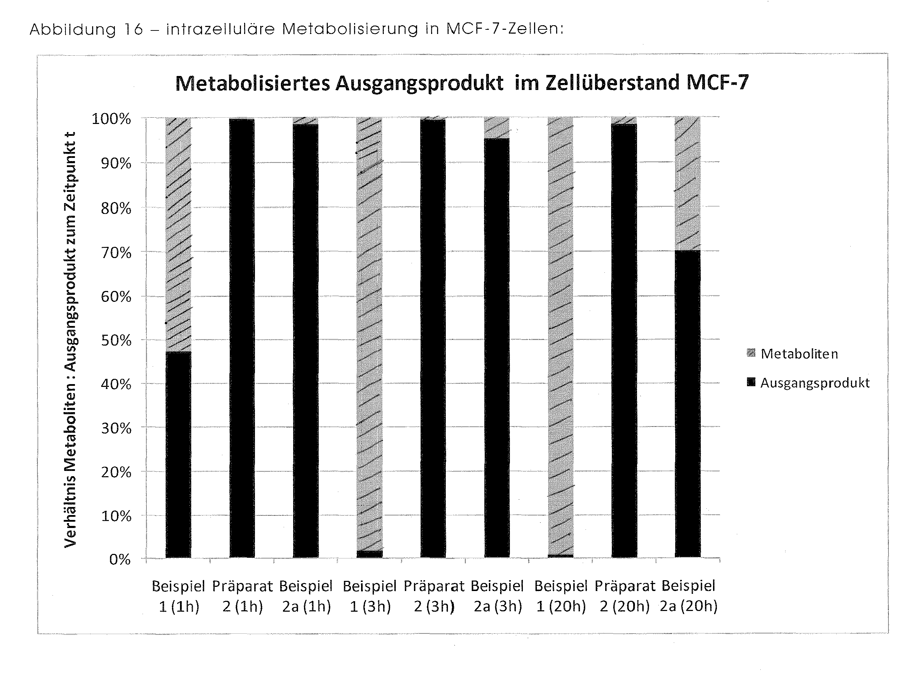

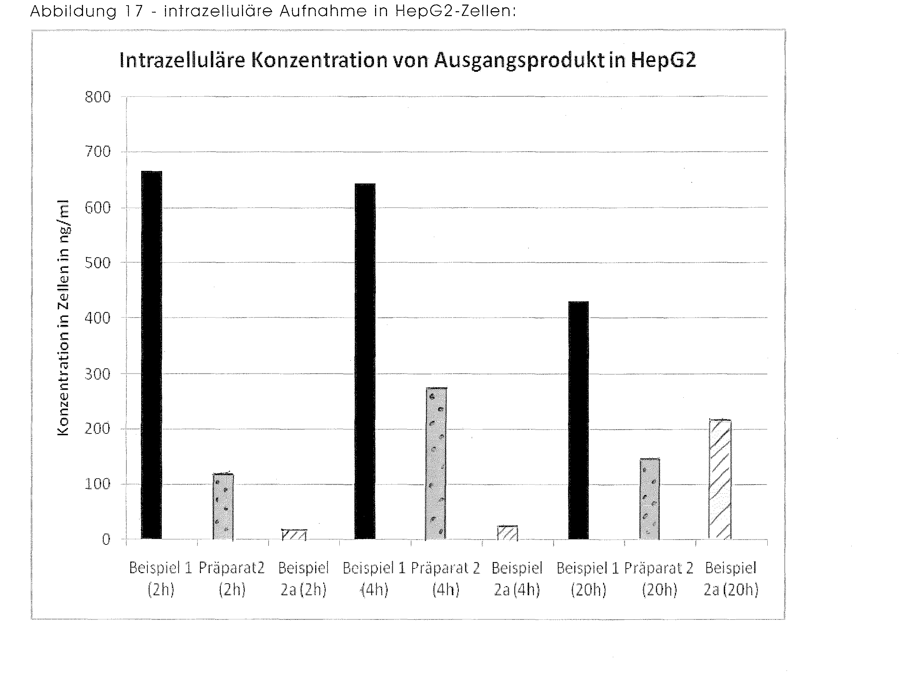

Abbildung 16 zeigt die intrazelluläre Metabolisierung verschiedener Triazene in MCF-7-Zellen.

### Experiment 2: Intrazelluläre Aufnahme verschiedener Triazene in HepG-2, HCT-8 und MD-MBA231 Zellkulturen

Es wird gezeigt, dass Verbindungen nach Beispiel 1 schneller und in größerer Menge intrazellulär aufgenommen werden als die als Vergleichssubstanzen eingesetzten Verbindungen nach Präparat 2 und 5 (entsprechend Verbindungen nach Präparat 2 und Beispiel 2a jeweils der WO 2009/004060).

### Experimenteller Aufbau:

Die getesteten Verbindungen wurden wie folgt gelöst:
Erfindungsgemäße Verbindung:
Beispiel 1: in 20 mg/ml DMSO

Vergleichssubstanzen:
Präparat 2: in 50mg/ml DMSO,
Präparat 5: in 50mg/ml Wasser.

Alle drei Substanzen wurden in einer Dosis von 50.000ng/ml auf 2 x 10⁷ HepG2 und HCT-8 sowie 2 x 10⁶-MDA-MB231 Zellen gegeben. Als Zellkulturmedium wurde 100% (MDA-MB231: 50%) fötales Kälberserum (FCS) verwendet. Zudem wurde bei den Versuchen mit HepG2 und HCT-8 noch 10.000ng/ml O6-Benzylguanin zugesetzt. Für den Versuch wurde die intrazelluläre Konzentration von Ausgangsprodukt und Metaboliten nach 2, 4 und 20 (HepG2), 2 und 3,5 und 20 Stunden (MDA-MB231) und 2,4, 24 und 48 Stunden (HCT-8) Inkubationszeit ermittelt. Alle drei Substanzen zeigten in Kontrollversuchen mit 100% FCS nach 40 Stunden metabolische Stabilität.

### Ergebnis:

Die Verbindung nach Beispiel 1 ist in HepG2- Zellen deutlich schneller und in höheren Konzentrationen intrazellulär nachweisbar als die Vergleichssubstanzen aus WO2009/004060.

Die Verbindung nach Beispiel 1 ist in MDA-MB231- Zellen deutlich schneller und in höheren Konzentrationen intrazellulär nachweisbar als die Vergleichssubstanzen aus WO2009/004060.

Die Verbindung nach Beispiel 1 ist in HCT-8 Zellen deutlich schneller und für die ersten beiden Messpunkte auch in höherer Konzentration intrazellulär nachweisbar als die Vergleichssubstanzen aus WO2009/004060. Zum dritten Messpunkt wurden höhere Konzentrationen für die Vergleichssubstanzen aus WO2009/004060 festgestellt. Zum letzten Messpunkt wurden vergleichbare Konzentrationen für alle Substanzen festgestellt.

Abbildung 1 7 zeigt die intrazelluläre Aufnahme verschiedener Triazene in HepG2-Zellen.

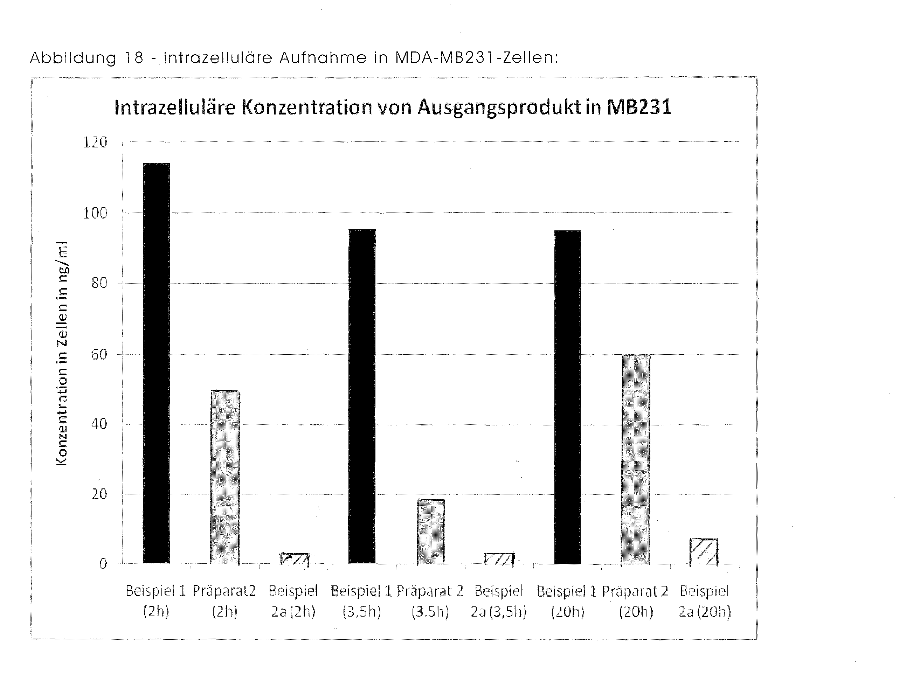

Abbildung 18 zeigt die intrazelluläre Aufnahme verschiedener Triazene in MDA-MB231 -Zellen.

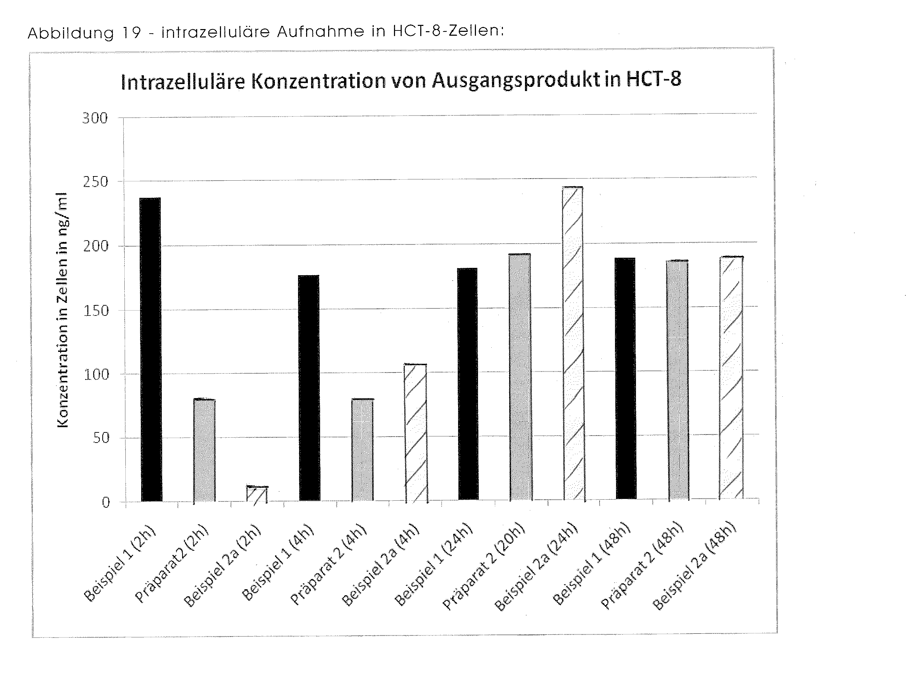

Abbildung 19 zeigt die intrazelluläre Aufnahme verschiedener Triazene in HCT-8-Zellen.

### Experiment 3: IC₅₀ verschiedener Triazene in verschiedenen Zelllinien

Die nachfolgenden Experimente 3 und 4 zeigen, dass die erfindungsgemäßen neuen Triazenverbindungen eine vergleichbare Anti-Tumorwirkung (IC₅₀) in zahlreichen Krebszelllinien wie der HepG2 (Leber), HCT8 (Darm), DU-145 (Prostata), MCF-7 und MDA-MB-231 (Brust) und MiaPaca-2 (Bauchspeicheldrüse) bei bis zu 100fach niedrigeren Konzentrationen zeigen als vergleichbare Triazene wie DTIC, TMZ oder Substanzen wie sie in der WO 2009/004060 und der DE 1 7931 1 5 beschrieben sind.

### Beschreibung des Experiments:

Verschiedene Triazene wie erfindungsgemäße Verbindungen nach Beispiel 1, DTIC, TMZ sowie Verbindungen nach Präparat 2 und 5 (entsprechend Verbindungen nach Präparat 2 und Beispiel 2a jeweils der WO 2009/004060) sowie Verbindungen nach Beispiel 33 der DE1793115 wurden in DMSO bzw. Wasser gelöst.

### Ergebnis:

Die erfindungsgemäße Verbindung nach Beispiel 1 ist nicht nur bei Brustkrebszelllinien (MCF-7, MB-231) wirksam sondern auch in einer Vielzahl anderer Tumorentitäten wie Leber- (HepG2), Darm- (HCT8) und Prostatakrebs (DU-145). Verbindungen nach Beispiel 1 sind deutlich wirksamer als vergleichbare zugelassene Triazene wie Dacarbazine und Temozolomide sowie von den Patenten WO 2009/004060 und DE1793115 erfaßte Verbindungen.

Ferner konnte gezeigt werden, dass die höhere antitumorale Aktivität von Verbindungen nach Beispiel 1 insbesondere auch gegenüber Verbindungen gemäß Beispiel 2 der DE1 793115 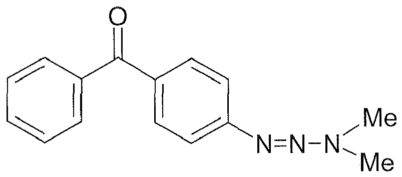 besteht.

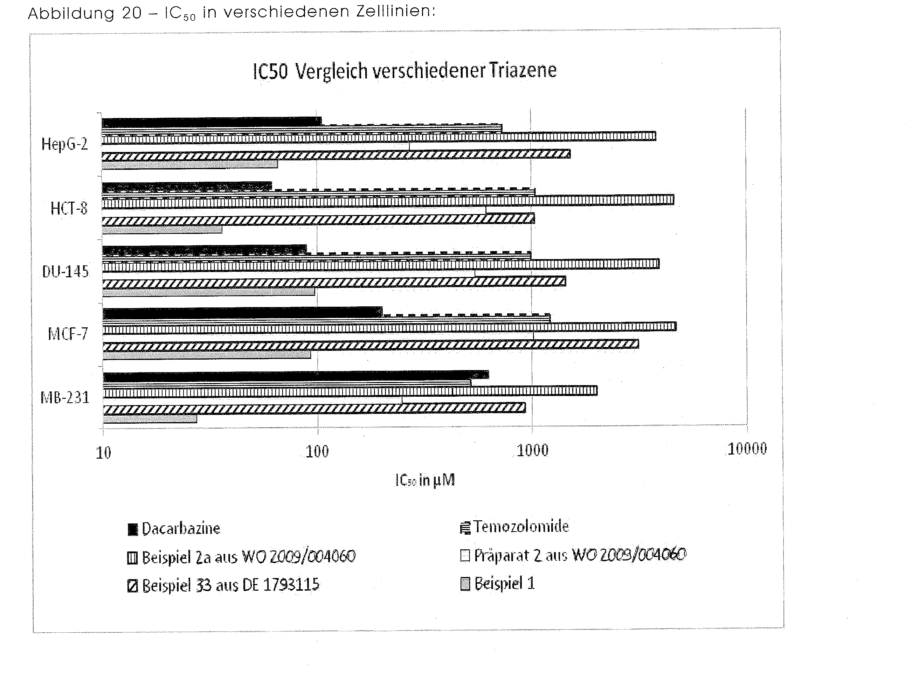

Abbildung 20 zeigt die IC₅₀ verschiedener Triazene in verschiedenen Tumorzelllinien.

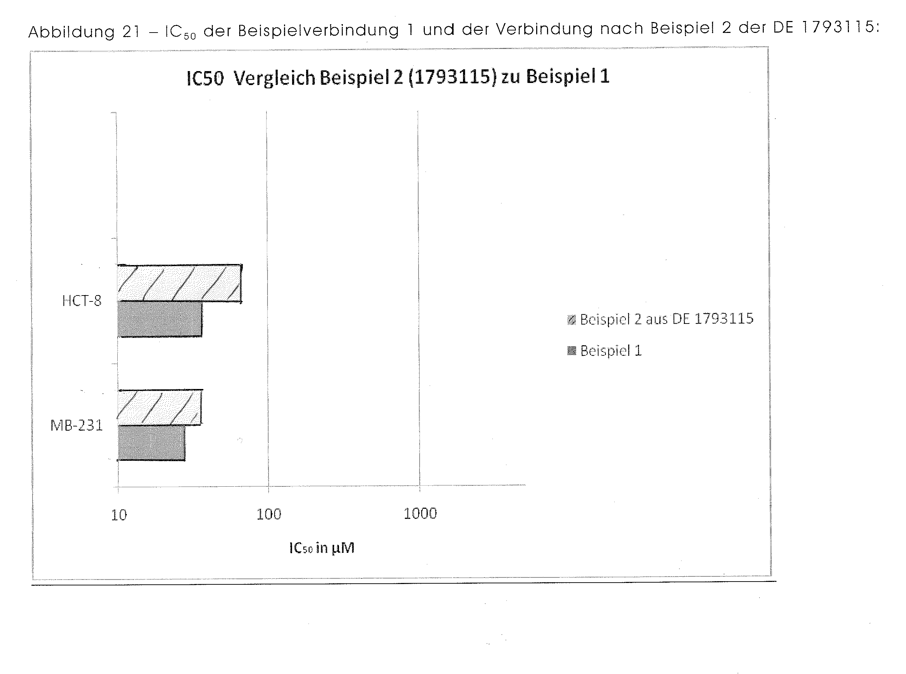

Abbildung 21 zeigt einen Vergleich der IC₅₀ der erfindungsgemäßen Verbindung nach Beispiel 1 und einer Verbindung nach Beispiel 2 der DE 1 7931 1 5 in verschiedenen Tumorzelllinien.

### Experiment 4: IC₅₀ Werte verschiedener Triazene in drei Pankreaskrebszelllinien

### Beschreibung des Experiments:

Verschiedene Triazene wie erfindungsgemäße Verbindungen nach Beispiel 1, DTIC, TMZ sowie Verbindungen nach Präparat 2 und 5 (entsprechend Verbindungen nach Präparat 2 und Beispiel 2a jeweils der WO 2009/004060) sowie Verbindungen nach Beispiel 2 und 33 der DE1793115 wurden in DMSO bzw. Wasser gelöst.

### Ergebnis:

Die erfindungsgemäße Verbindung nach Beispiel 1 zeigte eine deutliche Überlegenheit gegenüber den Vergleichssubstanzen Temozolomide (TMZ), den Präparaten 2 und 5 und Verbindungen nach Beispiel 33 aus DE1793115 in den drei Pankreaskrebszelllinien. Dacarbazine (DTIC) zeigte eine vergleichbare Wirkung wie Beispiel 1 in einer Zelllinie (AsPc-1), in beiden anderen Zelllinien aber deutlich geringere antitumorale Aktivität.

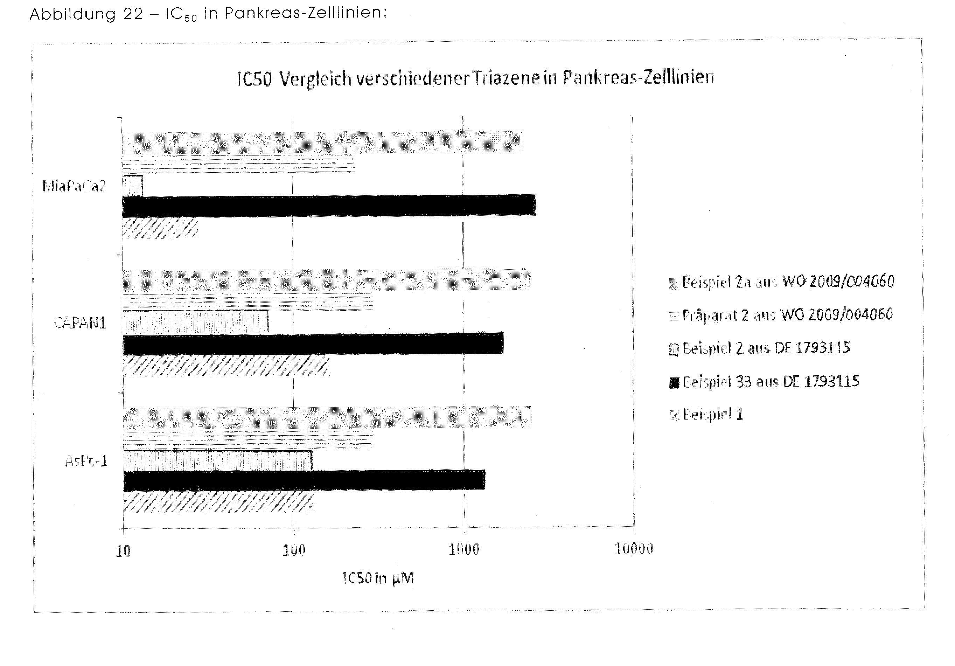

Abbildung 22 zeigt die IC₅₀ verschiedener Triazene in verschiedenen Pankreas-Zelllinien.

### Experiment 5: Zellschädigungen in PBC infolge Inkubation mit verschiedenen Triazenen

Um die therapeutische Breite der neuen Substanzen abschätzen zu können, wurden periphere Blutzellen (PBC) und hämatologische Zellen (Nalm-6; CEM und U-937) für 24 Stunden mit unterschiedlichen Dosen verschiedener Triazenverbindungen bzw. Doxorubicin als positive Kontrolle für die hämatologischen Zelllinien inkubiert. Das Maß der Zellschädigung (Apoptoserate) wurde mittels CaspACE^{™} Assay (am Durchflusszytometer) bestimmt.

### Beschreibung des Experiments:

Verschiedene Triazene wie erfindungsgemäße Verbindungen nach Beispiel 1, TMZ sowie Verbindungen nach Präparat 2 und 5 (entsprechend Verbindungen nach Präparat 2 und Beispiel 2a jeweils der WO 2009/004060) wurden in DMSO bzw. Wasser entsprechend den Angaben in Abbildung 22 gelöst.

Periphäre Blutzellen (PBC) werden jeweils mit der IC₅₀ für die MDA-MB231 gemäß Experiment 3 der entsprechenden Substanzen inkubiert. Es wird gezeigt, dass die erfindungsgemäße Verbindung nach Beispiel 1 nicht mehr DNA-Schädigungen in den PBC erzeugt als eine Negativkontrolle,

### Ergebnis

Periphäre Blutzellen werden durch eine 24 stündige Inkubation mit erfindungsgemäßen Verbindungen nach Beispiel 1 nicht mehr geschädigt als durch eine Negativkontrolle. Eine Inkubation mit der zugelassenen Substanz Temozolomide führt bereits zu einem Anstieg der Apoptoserate.

Die folgende Tabelle zeigt die Apoptoserate für verschiedene Triazenverbindungen:

| | **Versuch 1 Lymphozyten** | |
|---|---|---|
| | µM (=IC50) | % Zellen in Apoptose |
| Beispiel 1 [20mg/ml DMSO] | 27,5 | 8,120% |
| Beispiel 33 aus DE 1793115 [25mg/ml Wasser], deuteriert | 1300,0 | 5,410% |
| Temozolomide [20mg/ml DMSO] | 515,0 | 15,320% |
| Dacarbazine [10mg/ml Wasser] | 625,0 | 8,260% |
| Beispiel 2 aus DE 1793 115 [20mg/ml DMSO] | 25,5 | 6,570% |
| Kontrolle | | 9,960% |

### Experiment 6: Einfluss der Substituierung mit einer Sulfo-Gruppe an den Phenylkernen

Aus der DE 2147781 sind 4-Hydroxytriazen-Verbindungen bekannt, die an den Phenylkernen mit Sulfo- oder Carboxy-Gruppen substituiert sind.

Im Tumormodell wurde der Einfluss einer solchen Sulfo- oder Carboxy-Substitution im Vergleich zu einer unsubstituierten 4-Hydroxytriazen-Verbindung beispielhaft im Vergleich der erfindungsgemäßen bevorzugten Verbindung nach Beispiel 1 : 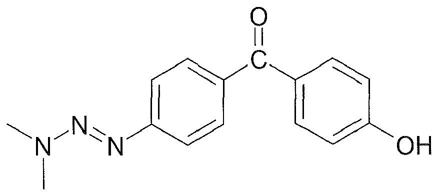 und den Verbindungen gemäß DE 21 47771 :

Natrium-4-Nydroxy-4'-dimethyltriazen-diphenylsulfoxid-sulfonat-(3) 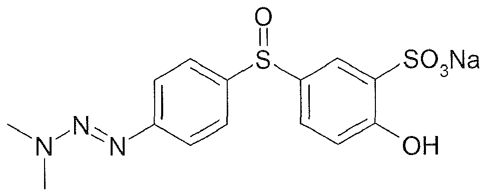 (Beispiel 5 der DE 2147781), sowie

Natrium-4-Hydroxy-4'-(dimethyltriazen)-diphenylsulfid-sulfonat-(3) 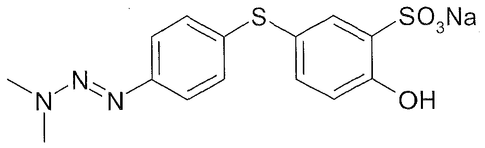 (Figur 6 der DE 2147781) getestet.

### Beschreibung des Experiments:

Die Mammakarzinome werden durch subkutane Injektionen von in physiologischer Kochsalz-Lösung suspendiertem Benzidin an weiblichen Ratten induziert. Je nach Art des Rattenstammes sind dazu unterschiedlich hohe Gesamtdosen und verschieden lange Induktionszeiten erforderlich. Bei zwei untersuchten Wistar-Stämmen (Züchter: Spiegel bzw. Winkelmann) erscheinen die Tumoren nach einer Induktionszeit von 150-330 Tagen und einer Gesamtdosis von 1000 bis 1225mg/kg Benzidin subkutan. Bei zwei verschiedenen Sprague-Dawley-Stämmen (Züchter: Mus Rattus AG bzw. Winkelmann) genügt schon eine einzige Benzidin-Injektion von 150mg/kg s.c., um nach 60-180 Tagen die Mammakarzinome entstehen zu lassen. Die Mammakarzinome entwickeln sich fast immer multipel. Acht oder mehr Tumoren pro Tier sind keine Seltenheit. Die Tumoren wachsen ziemlich schnell und führen etwa 30 bis 1 20 Tage nach Tastbarwerden zum Tode der Ratten. Nach 30 Tagen ist eine Tumorgröße von 6g zu erwarten (Figur 1 aus DE 01 7931 15).

Die Vergleichssubstanzen wurden für 30 Tage täglich s.c, oder peroral in Dosierungen zwischen 50-200mg/kg verabreicht. Die Verbindung nach Beispiel 1 wurde s.c. über die ersten 7 Tage in Dosierungen zwischen 20 und 100mg/kg, so dann in Dosierungen von 200 bis 1000mg/kg (in Stufen von 100mg/kg) für weitere 10 Tage appliziert. Bis zum Tag 30 erfolgte keine weitere Behandlung.

Die Entwicklung der Tumorgewichte wurde gegenüber dem Ausgangswert zu Beginn der Behandlung bestimmt und die prozentualen Medianwerte einander gegenübergestellt.

### Ergebnis:

Trotz einer deutlich niedrigeren Gesamtdosis der applizierten erfindungsgemäßen Verbindung nach Beispiel 1 über die ersten 7 Tage entwickeln sich die Tumore unter dieser Therapie zu jedem Beobachtungszeitpunkt langsamer als unter einer Therapie mit den verwendeten Vergleichssubstanzen. Gegenüber nicht behandelten Tieren scheinen auch die Vergleichssubstanzen eine Wachstumshemmung zu bewirken, die jedoch weniger ausgeprägt erscheint als die durch die Verbindung nach Beispiel 1 erzeugte.

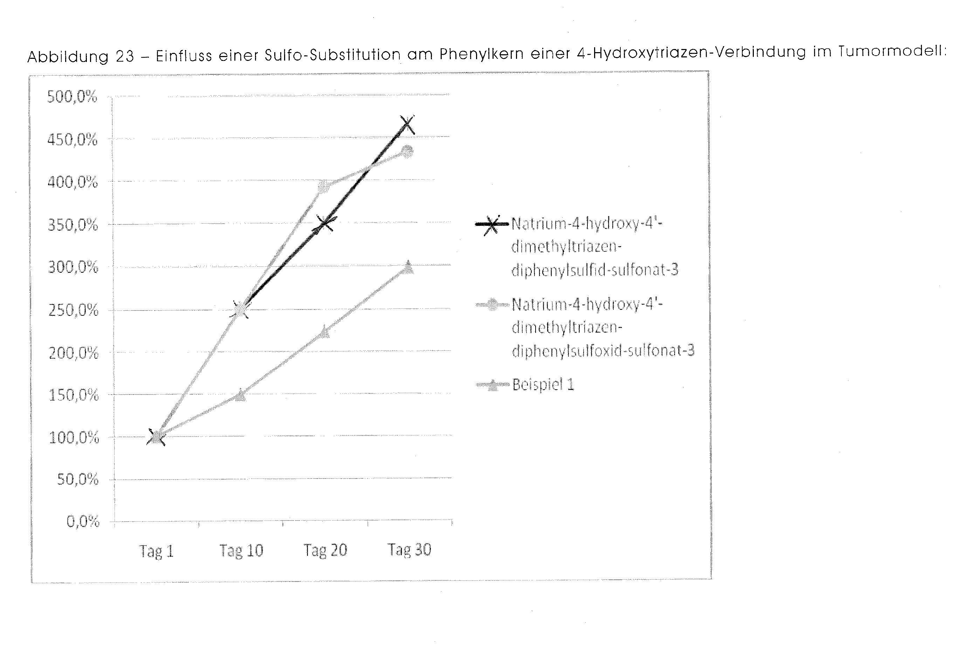

Abbildung 23 zeigt den Einfluss einer Sulfo-Substitution am Phenylkern einer 4-Hydroxytriazen-Verbindung im Tumormodell.

### Experiment 7: Wachstumsinhibition in leukämischen Zellen infolge Inkubation mit verschiedenen Triazenen

Hämatologische Zellen (CEM, HL-60) wurden für 24 Stunden mit unterschiedlichen Dosen verschiedener Triazenverbindungen inkubiert, um die IC₅₀ zu bestimmen.

### Beschreibung des Experiments:

Verschiedene Triazene wie erfindungsgemäße Verbindungen nach Beispiel 1, Beispiel 2a der WO2009/004060 sowie Verbindungen nach Beispiel 2 und 33 (deuteriert) der DE 1 793 1 15 und Doxorubicin als positive Kontrolle wurden in DMSO bzw. Wasser gelöst.

### Ergebnis

Im Unterschied zu den Triazenverbindungen aus WO2009/004060 und Beispiel 33 aus DE 1793115 hemmt die Verbindung nach Beispiel 1 das Wachstum der CEM und HL-60 Zellen in bis zu 100-fach niedrigerer Konzentration, Hinsichtlich der IC₅₀ lässt sich für Beispiel 1 gegenüber der Beispielverbindung 2 aus DE 1 7 93 1 15 kein Vorteil erkennen.
Die folgenden Tabellen zeigen die IC₅₀ Werte für die ausgewählten Triazenverbindungen und Doxorubicin als positive Kontrolle:

### IC50 Werte in µM

| Substanz | Exp. 1 | Exp. 2 | Exp. 3 | Exp. 4 | Exp. 5 | Mittelwert | Std.Abw. |
|---|---|---|---|---|---|---|---|
| Zelllinie HL-60 | | | | | | | |
| Beispiel 2 der DE 1793115 | 8,69 | 11,84 | 11,05 | 9,47 | 9,08 | 10,0 | 1,4 |
| Beispiel 33 der DE 1793115 | 573,77 | 573,77 | 573,77 | 573,77 | 546,45 | 568,3 | 12,2 |
| Beispiel 2a der WO2009/004060 | 829,15 | 879,40 | 1.532,66 | 1.507,54 | | 1.187,2 | 385,1 |
| Beispiel 1 | 12,64 | 15,24 | 24,16 | 15,99 | 13,38 | 16,3 | 4,6 |
| Doxorubicin | 1,20*10⁻³ | 1,16*10⁻³ | 6,62*10⁻⁴ | 1,31*10⁻³ | 1,34*10⁻³ | 1,13*10⁻³ | 2,74*10⁻⁴ |

| Substanz | Exp. 1 | Exp. 2 | Exp. 3 | Exp. 4 | Exp. 5 | Mittelwert | Std.Abw. |
|---|---|---|---|---|---|---|---|
| Zelllinie CEM | | | | | | | |
| Beispiel 2 der DE 1793115 | 10,26 | 10,26 | 8,69 | 10,66 | | 10,0 | 0,9 |
| Beispiel 33 der DE 1793115 | 437,1 6 | 437,16 | 409,84 | 464,48 | | 437,2 | 22,3 |
| Beispiel 2a der WO2009/004060 | 753,77 | 879,40 | 678,39 | 628,14 | | 734,9 | 109,3 |
| Beispiel 1 | 23,42 | 29,74 | 23,79 | 14,13 | | 22,8 | 6,4 |
| Doxorubicin | 6,81*10³ | 5,89*10³ | 9,94*10³ | 1,14*10² | | 8,51*10³ | 2,59*10³ |

### Experiment 8 Formulierung_ einer applizierbaren Formulierung für die intravenöse Applikation ausgewählter Triazenverbindungen aus Experiment 7

Um die Testergebnisse aus Experiment 7 in einem in-vivo Xenograft Modell zu bestätigen, wurden Formulierungsversuche mit Verbindungen nach Beispiel 1 sowie der Verbindung nach Beispiel 2 aus DE 1 793 1 15, als den wirksamsten Verbindungen aus Experiment 7, unternommen. Dabei zeigte sich, dass eine stabile und verträgliche Formulierung der neuen Triazenverbindungen nach Beispiel 1 sowie mit Verbindungen nach Beispiel 2 aus DE 1 793 115 trotz Einsatz verschiedener Lösungsmittel wie Cremophor, PEG400, Tween80, Poloxamer nicht erreicht werden konnte. Schließlich konnte eine Formulierung gefunden werden, in der sich die erfindungsgemäße Substanz nach Beispiel 1 vollständig löst und sich trotz Gehalt eines organischen Lösungsmittels sicher verabreichen lässt.

### Herstellung einer Formulierung mit Verbindungen nach Beispiel 1:

15mg der Verbindung nach Beispiel 1 werden in 30*µ*L DMSO (33*µ*g DMSO) gelöst. Diese Stocklösung wird in 270*µ*L 1 M Na₂CO₃ Lösung gegeben und nur leicht geschwenkt, nicht aber mittels Ultraschallbad behandelt. Die gebrauchsfertige Lösung ist unter Raumtemperatur stabil und kann den Tieren direkt appliziert werden.

### Herstellung einer Formulierung mit Verbindungen nach Beispiel 2 der DE 1793 115:

Gleichermaßen sollte eine Formulierung für Verbindungen nach Beispiel 2 aus DE 1 7 93 1 15 hergestellt werden. Hier wurden 15*µ*L der Verbindung nach Beispiel 2 vorgelegt. Bei einer Dichte von 1,08g/ml entsprach dies 16,2mg der Verbindung nach Beispiel 2. Dazu wurden 32,4*µ*L DMSO gegeben. Die Verbindung nach Beispiel 2 löste sich vollständig. Sodann wurden 90*µ*L einer frisch hergestellten 1 molaren Na₂CO₃ Lösung vorgelegt und 10*µ*L der Lösung der Verbindung nach Beispiel 2 hinzugegeben. Sofort fiel weißer Niederschlag aus und der obere Rand der Flüssigkeit färbte sich gelb. Auch mittels Ultraschallbad konnte keine Auflösung des erzeugten Niederschlags herbeiführen.

### Ergebnis:

Im Unterschied zu Verbindungen nach Beispiel 2 aus DE 17 93 115 ließ sich die erfindungsgemäße Verbindung nach Beispiel 1 in einer Weise formulieren, die eine Anwendung am Tier und Menschen erlaubt.

### Experiment 9: Zellzyklusanalyse von Verbindungen nach Beispiel 1 und Präparat 2 (entsprechend Präparat 2 der WO2009/004060)

Die Progression durch den Zellzyklus ist charakterisiert durch die mitotische Teilung (M-Phase) und der sich anschließenden Interphase, Die Interphase gliedert sich wiederum in die G1 -, S- und G2-Phase, In der G1-Phase beginnt die Zelle zu wachsen und es liegt ein vollständiger Chromosomensatz vor. In der S-Phase erfolgt die Replikation der DNA, wodurch an ihrem Ende der zweifache Chromosomensatz vorliegt. In der G2-Phase bereitet sich die Zelle auf die mitotische Teilung vor, an dessen Ende wieder der einfache Chromosomensatz vorhanden ist. Zusätzlich zu diesen Phasen kann eine sog. sub-G1-Phase vorliegen. Diese ist charakterisiert durch fragmentierte, apoptotische Zellen, die durch degradierte DNA charakterisiert ist. In welchem Stadium sich die Zelle befindet bzw. arretiert sein kann, lässt sich mittels Durchflusszytometrie bestimmen (Brockhoff et al. 2007; Lenz et al. 2007).

Zellkulturen von MDA-MB231 bzw. HL-60 wurden mit der IC₅₀ (MDA-MB231) bzw. IC₉₀ (HL-60) der beiden Test-Substanzen für bis zu 72 Stunden inkubiert und auf die Zellzyklusentwicklung hin untersucht.

### Ergebnis:

Die neue Triazenverbindung nach Beispiel 1 führt in der Zelllinie MDA-MB231 zu einem ausgeprägten G2 Arrest nach 10 bzw. 24 Stunden und massivem Zelltod (SubG1) im Unterschied zu Präparat 2.

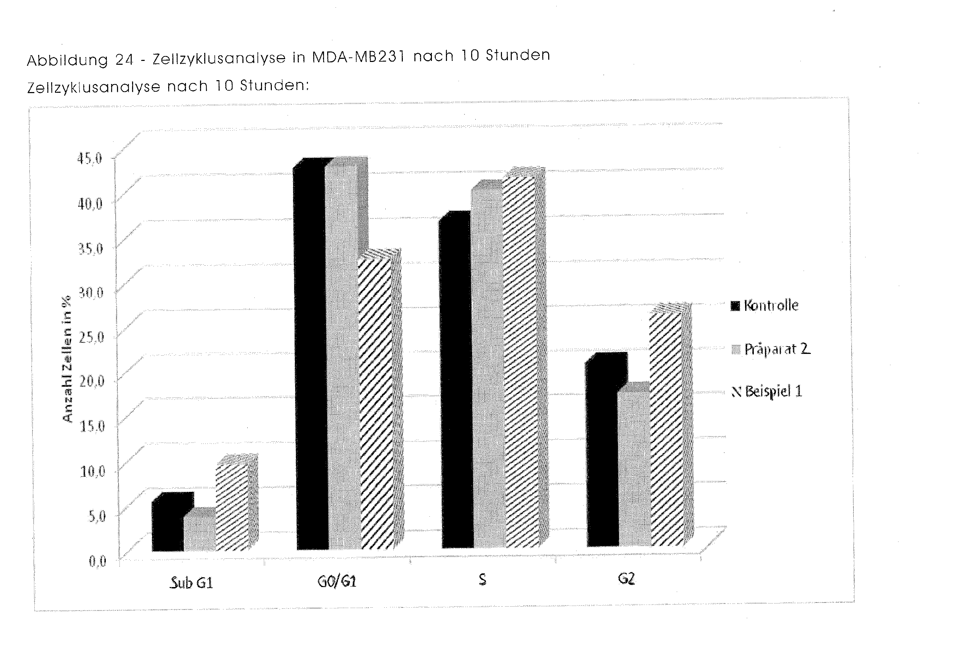

Abbildung 24 zeigt den Einfluss ausgewählter Triazenverbindungen auf den Zellzyklus der MDA-MB231 -Zelllinie nach 1 0 Stunden. Es zeigt sich bereits eine G2-Arretierung und erhöhte Anzahl toter Zellen (SubG1 Fraktion).

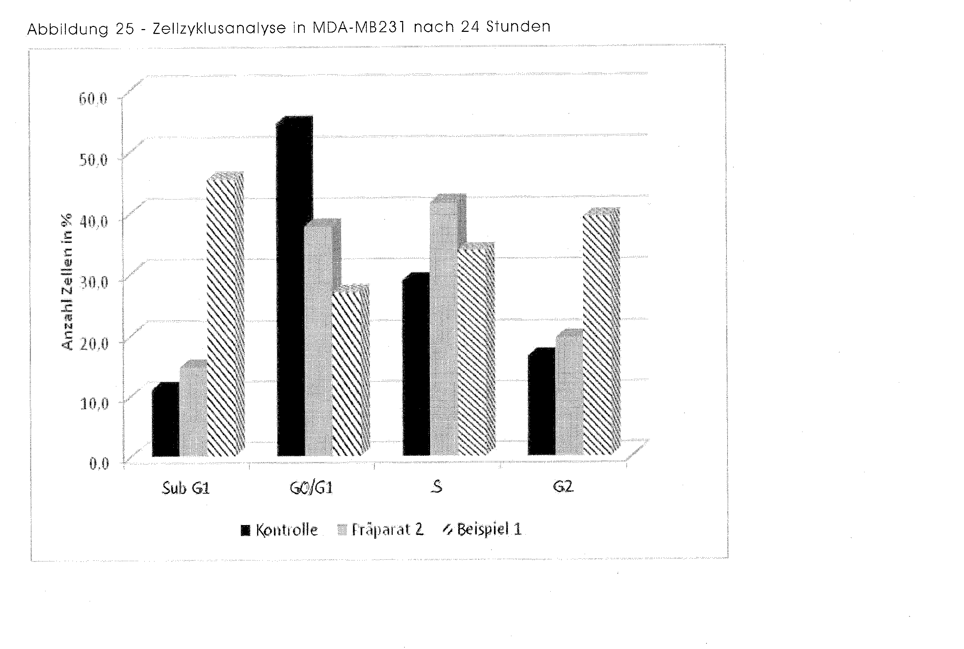

Abbildung 25 zeigt den Einfluss ausgewählter Triazenverbindungen auf den Zellzyklus der MDA-MB231 -Zelllinie nach 24 Stunden. Die G2-Arretierung manifestiert sich; der Anteil toter Zellen ist deutlich stärker ausgeprägt als bei der Kontrolle und der Vergleichssubstanz (Präparat 2).

Markantere Ergebnisse konnten in der hämatologischen Zelllinie HL-60 gewonnen werden. Auch bei dieser kam es nach 12 bzw. 24 Stunden zu einem ausgeprägten G2 Arrest. Nach 24 Stunden befanden sich nur noch weniger als 2% der Zellen in der G1 Phase. Knapp 40% der eingesetzten Zellen waren nach 24 Stunden tot.

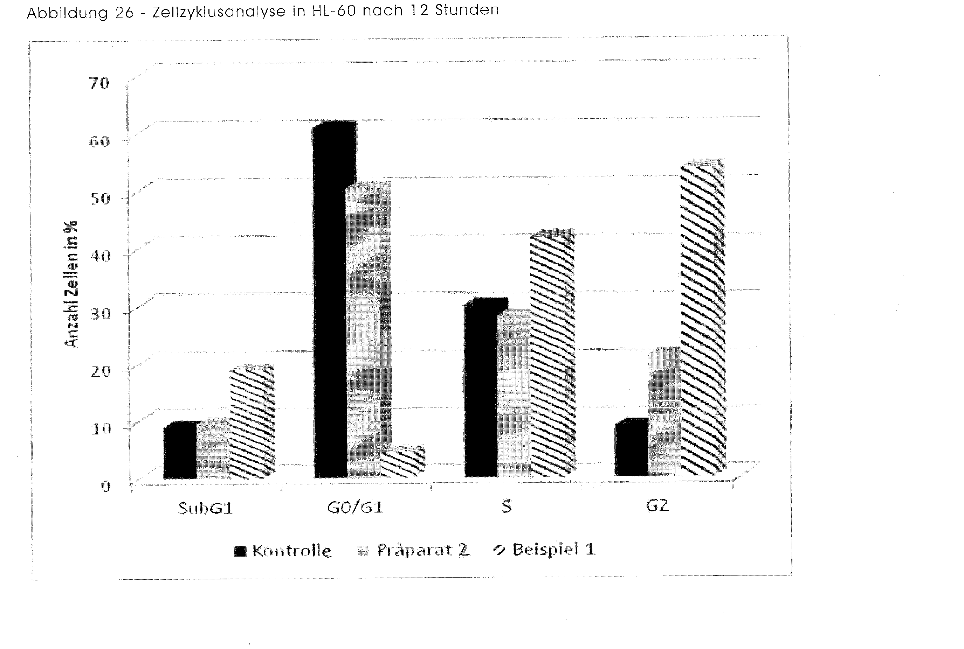

Abbildung 26 zeigt den Einfluss ausgewählter Triazenverbindungen auf den Zellzyklus der HL-60-Zelllinie nach 1 2 Stunden. Es zeigt sich bereits eine G2-Arretierung und erhöhte Anzahl toter Zellen (SubG1 Fraktion)

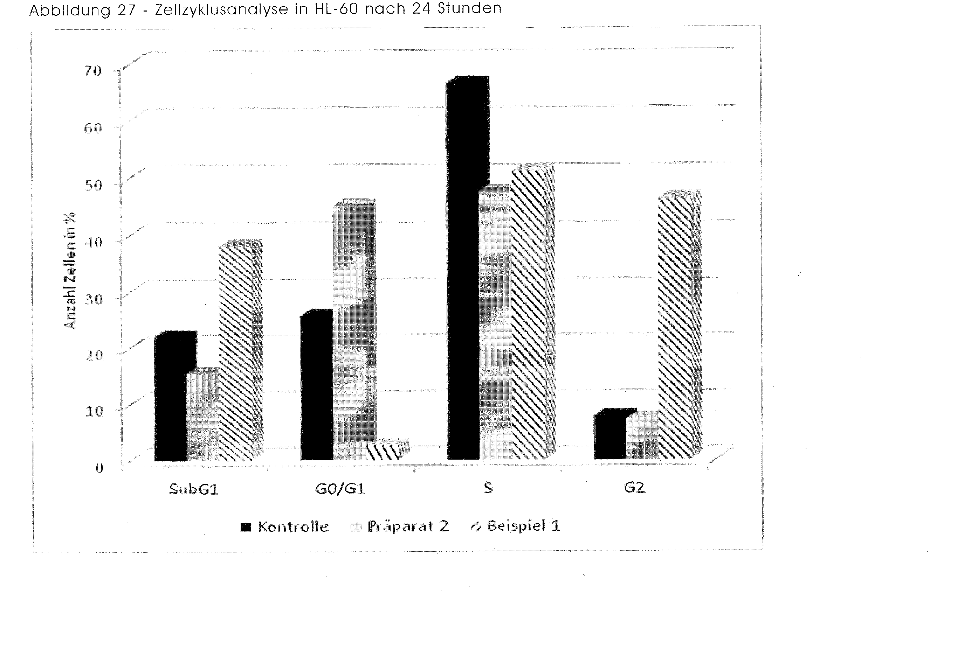

Abbildung 27 zeigt den Einfluss ausgewählter Triazenverbindungen auf den Zellzyklus der HL-60-Zelllinie nach 24 Stunden. Die G2-Arretierung manifestiert sich; der Anteil toter Zellen ist deutlich stärker ausgeprägt als bei der Kontrolle und der Vergleichssubstanz (Präparat 2).

Zusammenfassend zeigen sich die erfindungsgemäßen Verbindungen bzw. die pharmazeutischen Zusammensetzungen davon als potente Antitumorarzneien, die über ihre bessere Zellpermeabilität und vergleichbare Wirksamkeit in deutlich niedrigeren Dosierungen zu einem breiteren therapeutischen Fenster führen als bisherige Triazenverbindungen. Zudem wurde gezeigt, dass sich die erfindungsgemäßen Verbindungen auch pharmazeutisch akzeptabel formulieren lassen, um diese überhaupt am Tier und Mensch einsetzen zu können. Schließlich deuten Zellzyklusanalysen auf einen von anderen Triazenen abweichenden Wirkmechanismus hin.

## Patentansprüche

1. Verbindungen der Formel (1): 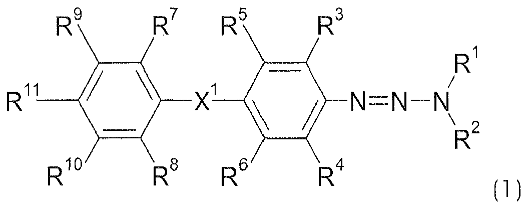 worin
R¹ und R² gleich oder verschieden sind, und jeweils ausgewählt werden aus der Gruppe, die besteht aus:
- gegebenenfalls substituiertem Alkyl,
- gegebenenfalls substituiertem Alkenyl, und
- gegebenenfalls substituiertem Aryl;
X¹ aus der Gruppe ausgewählt wird, die besteht aus:
- einer Einfachbindung
- Carbonyl,
- Schwefel,
- Sauerstoff,
- Sulfoxy,
- Sulfonyl,
- Azo, und
- einem gegebenenfalls substituierten, gesättigten oder ungesättigten aliphatischen Rest mit 1 bis 6 Kohlenstoffatomen;
und worin
i) R¹¹ Hydroxy ist und
R³, R⁴, R⁵, R⁶, R⁷, R⁸, R⁹ und R¹⁰ gleich oder verschieden sind, und jeweils ausgewählt werden aus Wasserstoff und Hydroxy;
oder worin
ii) mindestens einer der Reste R⁷, R⁸, R⁹, und R¹⁰ sowie gegebenenfalls mindestens einer der Reste R³, R⁴, R⁵ und R⁶ Hydroxy ist und
die übrigen Reste R³, R⁴, R⁵, R⁶, R⁷, R⁸, R⁹ und R¹⁰, die nicht Hydroxy bedeuten, sowie der Rest R¹¹ gleich oder verschieden sind und jeweils ausgewählt werden aus der Gruppe, die besteht aus:
- Wasserstoff,
- Halogen,
- Cyano,
- Nitro,
- Carboxyl,
- Alkoxycarbonyl,
- Aminocarbonyl,
- gegebenenfalls substituiertem Acyl,
- gegebenenfalls substituiertem Acyloxy
- gegebenenfalls substituiertem Sulfonyl (-SO₂R),
- Sulfonsäure,
- gegebenenfalls substituiertem Sulfonyloxy (-O-SO₂R),
- einem Sulfatrest,
- gegebenenfalls substituiertem Alkyl,
- gegebenenfalls substituiertem Alkoxy,
- gegebenenfalls substituiertem Aryloxy,
- gegebenenfalls substituiertem Alkenyl,
- gegebenenfalls substituiertem Alkinyl, und
- gegebenenfalls substituiertem Aryl;
oder worin
iii) mindestens einer der Reste R⁷, R⁸, R⁹, R¹⁰ und R¹¹ Methoxy ist und
die übrigen Reste R⁷, R⁸, R⁹, R¹⁰ und R¹¹, die nicht Methoxy bedeuten, sowie die Reste R³, R⁴, R⁵ und R⁶ Wasserstoff sind;
oder worin
iv) R¹¹ ausgewählt ist aus der Gruppe die besteht aus:
- gegebenenfalls substituiertem Acyloxy,
- gegebenenfalls substituiertem Sulfonyloxy,
- einem Sulfatrest,
- gegebenenfalls substituiertem Alkoxy, wobei eine Aminocarbonylsubstituierte Alkoxy-Gruppe der allgemeinen Formel 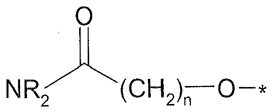 (n = 1 bis 7) ausgenommen ist,
- gegebenenfalls substituiertem Aryloxy; und
R³, R⁴, R⁵, R⁶, R⁷, R⁸, R⁹ und R¹⁰ Wasserstoff sind;
oder worin
v) mindestens einer der Reste R⁷, R⁸, R⁹, R¹⁰ und R¹¹ sowie gegebenenfalls mindestens einer der Reste R³, R⁴, R⁵ und R⁶ gegebenenfalls substituiertes Alkoxy, ausgewählt aus einem über Sauerstoff gebundenen Zucker-Rest, ist
und
die übrigen Reste R³, R⁴, R⁵, R⁶, R⁷, R⁸, R⁹, R¹⁰ und R¹¹ die nicht die vorstehende Bedeutung eines über Sauerstoff gebundenen ZuckerRests aufweisen, gleich oder verschieden sind und jeweils ausgewählt werden aus der Gruppe, die besteht aus:
- Wasserstoff,
- Hydroxy,
- Halogen,
- Cyano,
- Nitro,
- Carboxyl,
- Alkoxycarbonyl,
- Aminocarbonyl,
- gegebenenfalls substituiertem Acyl,
- gegebenenfalls substituiertem Acyloxy
- gegebenenfalls substituiertem Sulfonyl (-SO₂R),
- Sulfonsäure,
- gegebenenfalls substituiertem Sulfonyloxy (-O-SO₂R),
- einem Sulfatrest,
- gegebenenfalls substituiertem Alkyl,
- gegebenenfalls substituiertem Alkoxy,
- gegebenenfalls substituiertem Aryloxy,
- gegebenenfalls substituiertem Alkenyl,
- gegebenenfalls substituiertem Alkinyl, und
- gegebenenfalls substituiertem Aryl;
oder pharmazeutisch verträgliche Salze davon.

2. Verbindungen nach Anspruch 1 , worin
R¹ und R² jeweils Alkyl sind und
X¹ Carbonyl (-CO-) ist,
oder pharmazeutisch verträgliche Salze davon.

3. Verbindungen nach Anspruch 1 oder 2, worin
i) R¹¹ Hydroxy ist und
R³, R⁴, R⁵, R⁶, R⁷, R⁸, R⁹ und R¹⁰ Wasserstoff sind;
oder worin
ii) mindestens einer der Reste R⁷, R⁸, R⁹ und R¹⁰ sowie gegebenenfalls mindestens einer der Reste R³, R⁴, R⁵ und R⁶ Hydroxy ist und
die übrigen Reste R³, R⁴, R⁵, R⁶, R⁷, R⁸, R⁹ und R¹⁰, die nicht Hydroxy bedeuten, sowie der Rest R¹¹ gleich oder verschieden sind und jeweils ausgewählt werden aus der Gruppe, die besteht aus:
- Wasserstoff,
- Halogen,
- Cyano,
- Carboxyl,
- Alkoxycarbonyl,
- gegebenenfalls substituiertem Acyl,
- gegebenenfalls substituiertem Acyloxy
- gegebenenfalls substituiertem Sulfonyl (-SO₂R),
- gegebenenfalls substituiertem Sulfonyloxy (-O-SO₂R),
- gegebenenfalls substituiertem Alkyl,
- gegebenenfalls substituiertem Alkoxy,
- gegebenenfalls substituiertem Aryloxy, und
- gegebenenfalls substituiertem Aryl;
oder worin
iii) der Rest R¹¹ sowie gegebenenfalls mindestens einer der Reste R⁷, R⁸, R⁹ und R¹⁰ Methoxy ist und
die übrigen Reste R⁷, R⁸, R⁹ und R¹⁰, die nicht Methoxy bedeuten, sowie die Reste R³, R⁴, R⁵ und R⁶ Wasserstoff sind;
oder worin
iv) R¹¹ ausgewählt ist aus der Gruppe die besteht aus:
- gegebenenfalls substituiertem Alkoxy, wobei eine Aminocarbonylsubstituierte Alkoxy-Gruppe der allgemeinen Formel 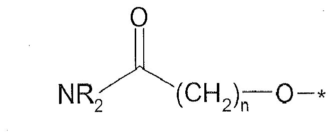 (n = 1 bis 7) ausgenommen ist,
- gegebenenfalls substituiertem Sulfonyloxy, und
- einem Sulfatrest; und
R³, R⁴, R⁵, R⁶, R⁷, R⁸, R⁹ und R¹⁰ Wasserstoff sind;
oder worin
v) mindestens einer der Reste R⁷, R⁸, R⁹, R¹⁰ und R¹¹ sowie gegebenenfalls mindestens einer der Reste R³, R⁴, R⁵ und R⁶ gegebenenfalls substituiertes Alkoxy, ausgewählt aus einem über Sauerstoff gebundenen Zucker-Rest, ist
und
die übrigen Reste R³, R⁴, R⁵, R⁶, R⁷, R⁸, R⁹, R¹⁰ und R¹¹ die nicht die vorstehende Bedeutung eines über Sauerstoff gebundenen ZuckerRests aufweisen, gleich oder verschieden sind und jeweils ausgewählt werden aus der Gruppe, die besteht aus:
- Wasserstoff,
- Hydroxy,
- Halogen,
- Cyano,
- Carboxyl,
- Alkoxycarbonyl,
- gegebenenfalls substituiertem Acyl,
- gegebenenfalls substituiertem Acyloxy
- gegebenenfalls substituiertem Sulfonyl (-SO₂R),
- gegebenenfalls substituiertem Sulfonyloxy (-O-SO₂R),
- gegebenenfalls substituiertem Alkyl,
- gegebenenfalls substituiertem Alkoxy,
- gegebenenfalls substituiertem Aryloxy, und
- gegebenenfalls substituiertem Aryl;
oder pharmazeutisch verträgliche Salze davon.

4. Verbindungen nach einem oder mehreren der Ansprüche 1 bis 3, worin
ii) einer der Reste R⁷, R⁸, R⁹ und R¹⁰ Hydroxy ist und
die übrigen Reste R⁷, R⁸, R⁹ und R¹⁰, die nicht Hydroxy bedeuten, sowie die Reste R³, R⁴, R⁵ und R⁶ Wasserstoff sind;
oder worin
iv) R¹¹ ausgewählt ist aus der Gruppe die besteht aus:
- gegebenenfalls substituiertem Alkoxy, wobei eine Aminocarbonylsubstituierte Alkoxy-Gruppe der allgemeinen Formel 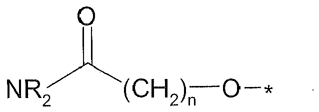 (n = 1 bis 7) ausgenommen ist, insbesondere gegebenenfalls substituiertes Alkoxy ausgewählt aus Methoxy (iii), und
- einem Sulfatrest; und
R³, R⁴, R⁵, R⁶, R⁷, R⁸, R⁹ und R¹⁰ Wasserstoff sind;
v) einer der Reste R⁷, R⁸, R⁹, R¹⁰ und R¹¹ gegebenenfalls substituiertes Alkoxy, ausgewählt aus einem über Sauerstoff gebundenen Zucker-Rest, ist und
die übrigen Reste R⁷, R⁸, R⁹ und R¹⁰, die nicht die vorstehende Bedeutung eines über Sauerstoff gebundenen Zucker-Rests aufweisen, sowie die Reste R³, R⁴, R⁵ und R⁶ Wasserstoff sind;
oder pharmazeutisch verträgliche Salze davon.

5. Verbindungen nach einem oder mehreren der Ansprüche 1 bis 4, worin
v) einer der Reste R⁷, R⁸, R⁹, R¹⁰ und R¹¹, bevorzugt R¹¹, ein über Sauerstoff gebundener Zucker-Rest, ausgewählt aus einem über Sauerstoff gebundenen Glucuronsäure-Rest, ist und
die übrigen Reste R⁷, R⁸, R⁹ und R¹⁰, die nicht die vorstehende Bedeutung eines über Sauerstoff gebundenen Glucuronsäure-Rests aufweisen, sowie die Reste R³, R⁴, R⁵ und R⁶ die Bedeutung von Wasserstoff, aufweisen;
oder pharmazeutisch verträgliche Salze davon.

6. Verbindungen nach einem oder mehreren der Ansprüche 1 bis 5, ausgewählt aus der Gruppe, die besteht aus: 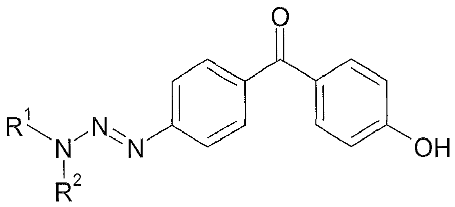 (entsprechend i)), 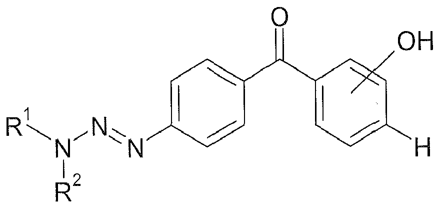 (entsprechend ii)), 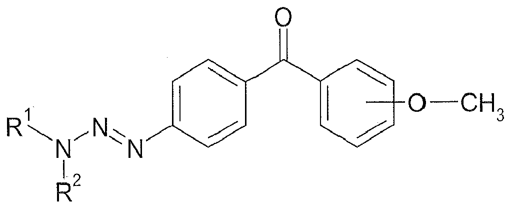 (entsprechend iii)), insbesondere 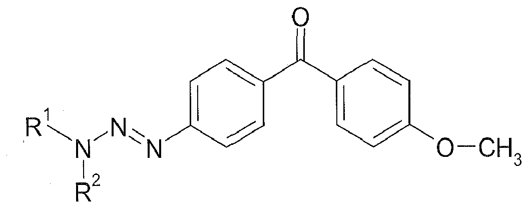 (entsprechend iii) und iv)) und 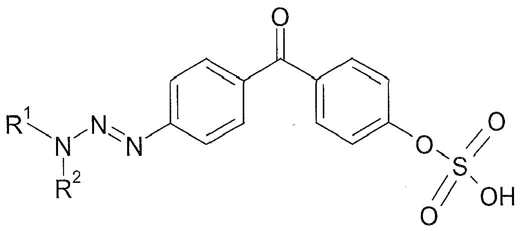 (entsprechend iv)), sowie 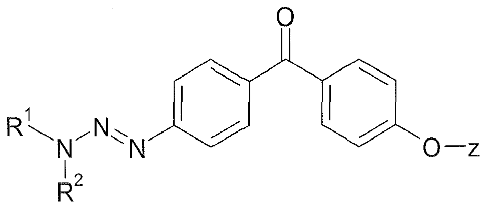 (entsprechend v)); worin jeweils R¹ und R² die Bedeutung gemäß Anspruch 1 oder 2 aufweisen und worin Z für einen Zucker-Rest steht;
oder pharmazeutisch verträgliche Salze davon.

7. Verbindungen nach einem oder mehreren der Ansprüche 1 bis 6, ausgewählt aus der Gruppe, die besteht aus: 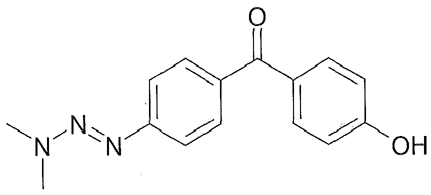 (entsprechend i)), 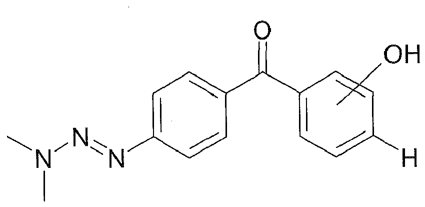 (entsprechend ii)), 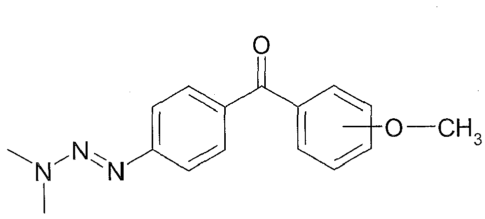 (entsprechend iii)), insbesondere 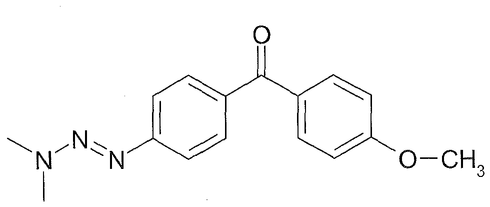 (entsprechend iii) und iv)), und 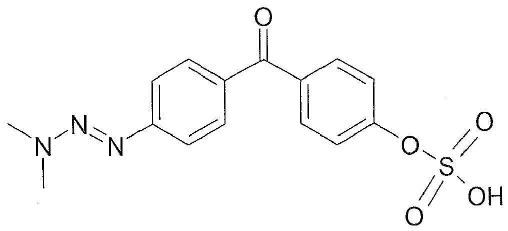 (entsprechend iv)), sowie 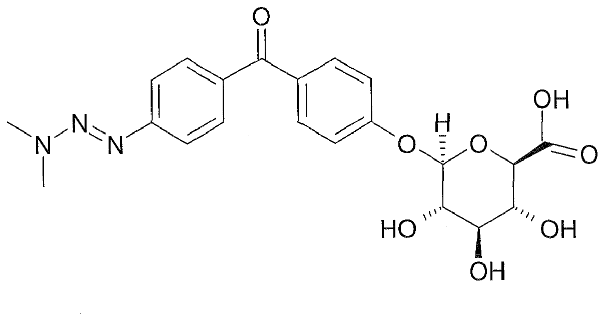 (entsprechend v)); oder pharmazeutisch verträgliche Salze davon.

8. Verfahren zur Herstellung der Verbindungen der Formel (1) nach einem oder mehreren der Ansprüche 1 bis 3, 6 und 7 (i), welches die Schritte umfasst:
Umsetzung einer Verbindung der Formel (3) 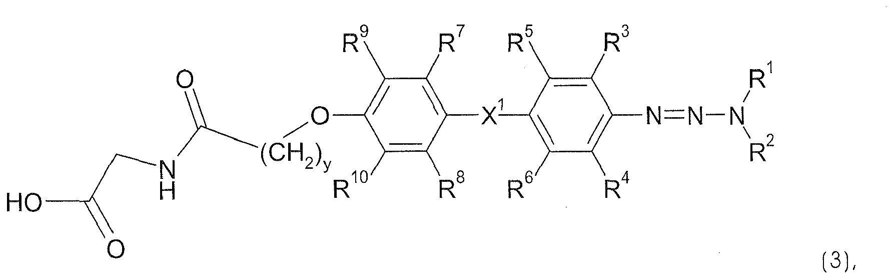
oder einem Salz davon,
durch Hydrolyse der Peptidbindung zu Verbindungen der Formel (3') 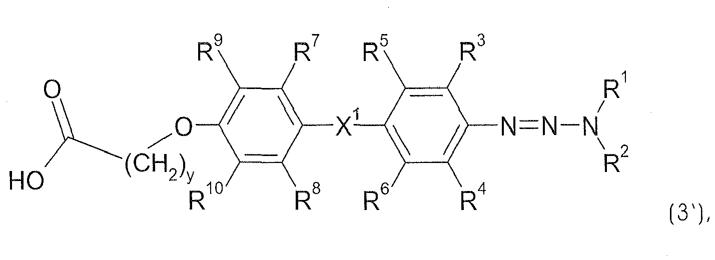
oder einem Salz davon,
worin jeweils y = 1 oder 2 bedeutet,
und/oder
Umsetzung einer Verbindung der Formel (4') oder einem Salz davon mittels beta-Eliminierung zu Verbindungen der Formel (1') 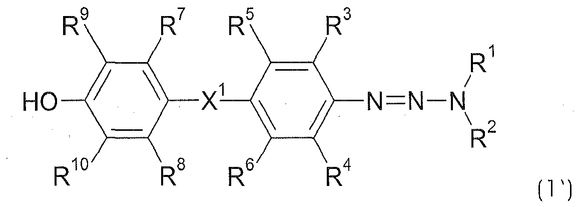
worin jeweils die Substituenten R¹ bis R¹⁰ wie in einem der Ansprüche 1 bis 3 definiert sind.

9. Verfahren nach Anspruch 8, worin eine Verbindung der Formel (1') 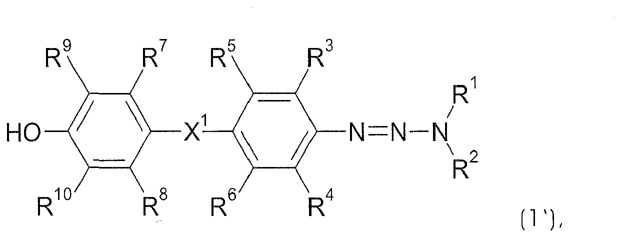 oder ein Salz davon,
unter Glucuronidierung zu Verbindungen der Formel (1"') 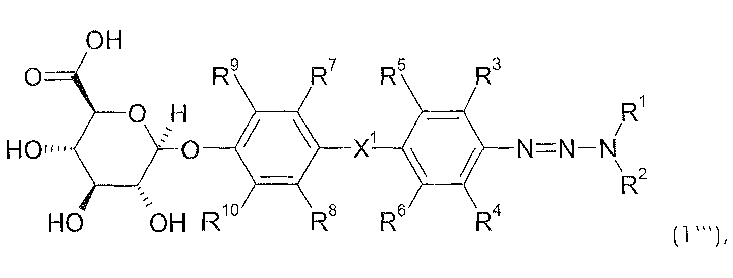 oder einem Salz davon, umgesetzt werden, jeweils mit der Bedeutung der Substituenten R¹ bis R¹⁰ gemäß Anspruch 8.

10. Verbindungen nach einem oder mehreren der Ansprüche 1 bis 7 oder erhältlich nach dem Verfahren nach einem der Ansprüche 8 oder 9 zur Verwendung als Arzneimittel für Mensch und Tier.

11. Verbindungen nach einem oder mehreren der Ansprüche 1 bis 7 oder erhältlich nach dem Verfahren nach einem der Ansprüche 8 oder 9 zur Verwendung in der Behandlung von Krebserkrankungen, insbesondere von Bauchspeicheldrüsenkrebs, Leberkrebs, Prostatakrebs, Brustkrebs, Darmkrebs, Melanomen oder Blutkrebs (hämatologischen Neoplasien) im Menschen oder Tier.

12. Zusammensetzung, enthaltend eine oder mehrere der Verbindungen nach einem oder mehreren der Ansprüche 1 bis 7, 1 0 bis 1 1 , oder erhältlich nach dem Verfahren nach einem der Ansprüche 8 oder 9, sowie einen oder mehrere pharmazeutische Träger und/oder Hilfsstoffe und/oder Lösungsmittel.

13. Kombinationspräparat, enthaltend eine oder mehrere der Verbindungen nach einem oder mehreren der Ansprüche 1 bis 7, 1 0 bis 1 1 , oder erhältlich nach dem Verfahren nach einem der Ansprüche 7, 8 oder 9 oder eine Zusammensetzung nach Anspruch 12, sowie mindestens eine weitere pharmazeutisch wirksame Verbindung, bei der es sich insbesondere um eine Verbindung zur Behandlung von Krebserkrankungen am Mensch oder Tier sowie der jeweils damit einhergehenden Symptome handelt.

14. Verbindungen nach einem oder mehreren der Ansprüche 1 bis 7, 10 bis 1 1 , oder erhältlich nach dem Verfahren nach einem der Ansprüche 8 oder 9, Zusammensetzungen nach Anspruch 12 sowie Kombinationspräparate nach Anspruch 13 zur Verwendung als Arzneimittel zur oralen oder parenteralen Verabreichung.
